# EUROPEAN PATENT APPLICATION

(11) **EP 1 939 194 A1**
(43) Date of publication of application: **02.07.2008**
(21) Application number: 06797767.8
(22) Date of filing: 05.09.2006
(51) Int. Cl.: C07D 401/04, A61K 31/4439, A61K 31/444, A61K 31/4709, A61K 31/4725, A61K 31/498, A61K 31/517, A61K 31/55, A61P 1/16, A61P 3/04, A61P 3/06, A61P 3/10, A61P 5/00, A61P 9/00, A61P 9/10, A61P 9/12, A61P 11/00, A61P 13/12, A61P 15/00

(54) **BICYCLIC AROMATIC SUBSTITUTED PYRIDONE DERIVATIVE**

(30) Priority: 07.09.2005 JP 2005259720
(71) Applicant: BANYU PHARMACEUTICAL CO., LTD., Tokyo 102-8667 (JP)
(72) Inventor: SAKURABA, Shunji, Ibaraki 300-2611 (JP); KAMEDA, Minoru, Ibaraki 300-2611 (JP); KISHINO, Hiroyuki, Ibaraki 300-2611 (JP); HAGA, Yuji, Ibaraki 300-2611 (JP); OTAKE, Norikazu, Ibaraki 300-2611 (JP); MORIYA, Minoru, Ibaraki 300-2611 (JP)
(74) Representative: Buchan, Gavin MacNicol
(86) International application number: PCT/JP2006/317941
(87) International publication number: WO 2007/029847

(57) **Abstract**

Disclosed is a compound represented by the formula (I): 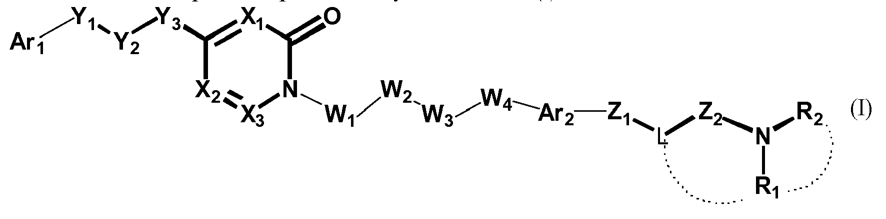 Wherein R₁ and R₂ independently represent a hydrogen atom, a lower alkyl group or the like; X₁ , X₂ and X₃ independently represent a methine group or a nitrogen atom; Y₁ and Y₃ independently represent a single bond, -O- or the like; Y₂ represents a lower alkylene group or the like; W1 to W4 independently represent a single bond, a methylene group or the like; L represents a single bond, a methylene group or the like; Z₁ and Z₂ independently represent a single bond, a C₁₋₄ alkylene group or the like; Ar₁ represents an aromatic carbocyclic ring or the like; and Ar₂ represents a bicyclic aromatic carbocyclic ring or the like. The compound is useful as a pharmaceutical for a central disease, a cardiovascular disease or a metabolic disease.

## Description

### TECHNICAL FIELD

The present invention relates to a bicyclic aromatic substituted pyridone derivative. The compound acts as a melanin concentrating hormone receptor antagonist, and is useful as a preventive, treating or remedial agent for various circular system diseases, nervous system diseases, metabolic diseases, genital diseases, respiratory diseases, digestive diseases, etc.

### BACKGROUND ART

Melanin concentrating hormone (hereafter referred to as "MCH") is a cyclic peptide hormone/neuro-peptide, which was for the first time isolated by Kawauchi, et al., in 1983 from sermon hypophysis. [Nature, Vol. 305, 321 (1983)]. The hormone is known to functionally antagonize for melanin cell stimulating hormone in fishes, to cause concentration of melanin granules in melanophore and participate in body color change [International Review of Cytology, Vol. 126, 1 (1991); Trends in Endocrinology and Metabolism, Vol. 5, 120 (1994)]. Also in mammals, MCH-containing neuron cells are localized in the hypothalamus lateral field and uncertain zone, but their nerve fibers are projecting over a very wide scope in the brain [see The Journal of Comparative Neurology, Vol. 319, 218 (1992)], and MCH is considered to preside over various central functions in living bodies.

Hypothalamus lateral field is known of old as feeding center, and furthermore, recently molecular biological and pharmacological knowledges suggesting participation of MCH in controlling energetic homeostasis are being much accumulated. That is, it has been reported that expression of mRNA, which is an MCH precursor, is accelerated in the brains of ob/ob mice, db/db mice, A^{y}/a mice, Zucker fatty rats which are model animals of hereditary obesity, and in the brains of fasting mice [see Nature, Vol. 380, 243 (1996); Diabetes, Vol. 47, 294 (1998); Biochemical and Biophysical Research Communications, Vol. 268, 88 (2000); Molecular Brain Research, Vol. 92, 43 (2001)].

Acute ventricular administration of MCH to rats was observed to induce accelerated feeding activity [Nature, Vol. 380, 243 (1996)] and chronic administration invites obesity accompanied by polyphagy [see Proceedings of the National Academy of Sciences of the United States of America, Vol. 99, 3240 (2002)]. Moreover, MCH precursor gene-deficient mice show reduced food ingestion or rise in oxygen consumption per body weight compared to wild type mice. Their low body weight due to decrease in body fat was observed [see Nature, Vol. 396, 670 (1998)].

On the contrary, transgenic mice which express excessive MCH precursor develop obesity accompanied by polyphagy and insulin resistance [The Journal of Clinical Investigation, Vol. 107, 379 (2001)]. Consequently, it is suggested that MCH is an important factor for developing obesity and participates in diseases induced by metabolic disorders or respiratory diseases for which obesity is one risk factor. Besides, MCH is known to participate also in anxiety-causing action, epilepsy, memory, learning, diuretic action, sodium/potassium excretory action, oxytocin secreting action, reproduction and reproductive function [see Peptides, Vol. 17, 171 (1996); Peptides, Vol. 18, 1095 (1997); Peptides, Vol. 15, 757 (1994); Journal of Neuroendocrinology, Vol. 8, 57 (1996); Critical Reviews in Neurobiology, Vol. 8,221 (1994)].

MCH causes versatile pharmacological actions through MCH receptors which are present mainly in the central nervous system. As receptors of MCH, at least two types of type 1 receptors (MCH-1R or SLC-1) and type 2 receptors (MCH-2R or SLT) are known [see Nature, Vol. 400, 261 (1999); Nature, Vol. 400, 265 (1999); Biochemical and Biophysical Research Communications, Vol. 261, 622 (1999); Nature Cell Biology, Vol. 1,267 (1999); FEBS Letters, Vol. 457, 522 (1999); Biochemical and Biophysical Research Communications, Vol. 283, 1013 (2001); The Journal of Biological Chemistry, Vol. 276, 20125 (2001); Proceedings of the National Academy of Sciences of the United States of America, Vol. 98, 7564 (2001); Proceedings of the National Academy of Sciences of the United States of America, Vol. 98, 7576 (2001); The Journal of Biological Chemistry, Vol. 276, 34664 (2001); Molecular Pharmacology, Vol. 60, 632 (2001)].

Of those, the pharmacological action observed on rodents is induced mainly via MCH-1R [see Genomics, Vol. 79, 785 (2002)]. Because MCH-1R gene-deficient mice chronically administered with MCH do not develop polyphagy or obesity, it is known that controlling of energy metabolism by MCH is induced via MCH-1R. Furthermore, the deficiency of MCH-1R is known to promote the activity amount of mice [see Proceedings of the National Academy of Sciences of the United States of America, Vol. 99, 3240 (2002)], and its participation in central diseases accompanied by behavioral disorders, for example, attention-deficit hyperactivity disorder, schizophrenia, depression and the like also is strongly suggested [see Molecular Medicine Today, Vol. 6, 43 (2000); Trends in Neuroscience, Vol. 24, 527 (2001)].

It is also reported that an autoantibody to MCH-1R is present in serum of vitiligo vulgaris patients [see The Journal of Clinical Investigation, Vol. 109, 923 (2002)]. Furthermore, expression of MCH-1R in certain species of cancer cells was reported, and in vivo expression sites of MCH and MCH-1R also suggest MCH's participation in cancer, sleep, vigil, drug dependence and digestive disorders [see Biochemical and Biophysical Research Communications, Vol. 289, 44 (2001); Neuroendocrinology, Vol. 61, 348 (1995); Endocrinology, Vol. 137, 561 (1996); The Journal of Comparative Neurology, Vol. 435, 26 (2001)].

Functions of MCH are expressed upon it binding to MCH receptors. Therefore, when its binding to MCH receptor is inhibited, then expression of MCH action can be inhibited. In consequence, substances which are antagonists for binding of MCH with its receptor are useful as preventive, treating or remedial agents for those various diseases in which MCH participates, for example, metabolic disorders such as obesity, diabetes, hormone disorder, hyperlipidemia, gout, fatty liver; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation et al.

As compounds having an MCH receptor antagonistic effect, for example, various compounds are disclosed in International Patent Publication Nos. WO01/21577, WO01/82925, WO02/06245, WO02/02744. However, they do not have a pyridone ring.

WO02/81454 (Patent Reference 1) discloses a pyridone derivative having an anti-hyperglycemia activity. However, concrete compounds in the reference have a single bond in the site of -Y₁-Y₂-Y₃- in the formula of the present invention; but in the present invention, Y₁, Y₂ and Y₃ are not all single bonds at the same time, and therefore the present invention differs from Patent Reference 1. Further, the reference does not describe an MCH receptor antagonistic effect.

WO03/68230 (Patent Reference 2) discloses a pyridone derivative having a P38MAP kinase activity. However, concrete compounds disclosed in the reference have a monocyclic substituent as the -Ar₂- moiety in the formula of the present invention, therefore differing from the present invention in which the moiety is a bicyclic substituent; and further, the reference does not describe an MCH receptor antagonistic effect.
Patent Reference 1: International Patent Publication WO02/81454
Patent Reference 2: International Patent Publication WO03/68230

### DISCLOSURE OF THE INVENTION

The present inventors have assiduously studied compounds having an MCH receptor antagonistic effect, and as a result, have found that a pyridone derivative, in which a bicyclic aromatic group bonds to the N atom of the pyridone ring via or not via a linker and a specific amino group bonds to the bicyclic aromatic group via a linker, has an MCH receptor antagonistic effect and is effective for prevention, treatment or remedy of various MCH receptor-associated diseases, and have completed the present invention.

Specifically, the invention provides:
(1) a pyridone derivative of a formula (I) or a pharmaceutically-acceptable salt thereof: 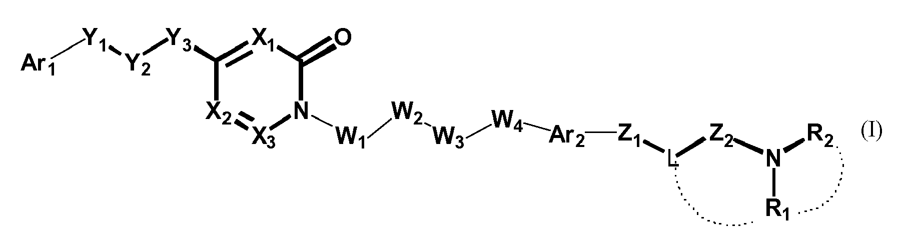 [wherein R₁ and R₂ are the same or different, each representing a hydrogen atom, a lower alkyl group optionally having substituent(s), or a lower cycloalkyl group optionally having substituent(s); or R₁ and R₂, taken together with the nitrogen atom to which they bond, may form an aliphatic nitrogen-containing hetero ring optionally having substituent(s);
   X₁, X₂ and X₃ are the same or different, each representing a methine group optionally having substituent(s), or a nitrogen atom; provided that X₁, X₂ and X₃ are not all nitrogen atoms at the same time;
   Y₁ represents a single bond, -O-, -NR-, -S-, -SO- or -SO₂-;
   Y₂ represents a lower alkylene group optionally having substituent(s), a lower alkenylene group optionally having substituent(s), or a lower cycloalkylene group optionally having substituent(s);
   Y₃ represents a single bond, -O-, -NR-, -S-, -SO- or -SO₂-;
   R each independently represents a hydrogen atom, or a lower alkyl group optionally having substituent(s);
   W₁, W₂, W₃ and W₄ are the same or different, each representing a single bond, a methylene group optionally having substituent(s), or -O-; provided that continuing two or more of W₁, W₂, W₃ and W₄ are not -O- at the same time;
   L represents a single bond, a methylene group optionally having substituent(s), or an ethylene group optionally having substituent(s); and L may form, taken together with Z₂, R₁ and the nitrogen atom to which R₁ bonds, an aliphatic nitrogen-containing hetero ring optionally having substituent(s);
   Z₁ and Z₂ are the same or different, each representing a single bond, or a C₁₋₄ alkylene group optionally having substituent(s), or -O-;
   Ar₁ represents an aromatic carbocyclic group optionally having substituent(s), or an aromatic heterocyclic group optionally having substituent(s);
   Ar₂ is a divalent group, representing a bicyclic aromatic carbocyclic group optionally having substituent(s), or a bicyclic aromatic heterocyclic group optionally having substituent(s)].
   The invention further provides:
(2) a melanin concentrating hormone receptor antagonist comprising a compound of (1) or a pharmaceutically-acceptable salt thereof as the active ingredient;
(3) a pharmaceutical composition comprising a pharmaceutically-acceptable additive and a compound of (1) or a pharmaceutically-acceptable salt thereof,
(4) a preventive, treating or remedial agent comprising a compound of (1) or a pharmaceutically-acceptable salt thereof as the active ingredient, for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; digestive disorders; respiratory disorders; cancer or pigmentation.
   The invention is described in more detail hereinunder.
   In this description, the term "lower" means that the number of the carbon atoms constituting the group or the compound with the term is at most 6, preferably at most 4.
   "Lower alkyl group" includes a linear alkyl group having from 1 to 6 carbon atoms or a branched alkyl group having from 3 to 6 carbon atoms, concretely, for example, a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-amyl group, a 1-methylbutyl group, a 2-methylbutyl group, a 1,2-dimethylpropyl group, a 1-ethylpropyl group, an n-hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1-ethylbutyl group, a 1,1,2-trimethylpropyl group, a 1,2,2-trimethylpropyl group, a 1-ethyl-2-methylpropyl group, 1-ethyl-1-methylpropyl group et al.
   "Lower cycloalkyl group" includes a cycloalkyl group having from 3 to 6 carbon atoms, concretely, for example, a cyclopropyl group, a cyclobutyl group, a cyclopentyl group, and a cyclohexyl group et al.
   "Lower alkylene group" includes a linear alkylene group having from 1 to 6 carbon atoms or a branched alkylene group having from 3 to 6 carbon atoms, concretely, for example, a methylene group, an ethylene group, a propylene group, a butylene group, a pentylene group, a hexylene group et al.
   "Lower alkenylene group" includes a linear alkenylene group having from 2 to 6 carbon atoms or a branched alkenylene group having from 3 to 6 carbon atoms, having one carbon-carbon double bond in the chain, concretely, for example, a vinylene group, a 1-propenylene group, a 2-propenylene group, a 1-butenylene group, a 2-butenylene group, a 3-butenylene group, a 2-pentenylene group, a 3-pentenylene group, a 4-pentenylene group, a 1-hexenylene group, a 2-hexenylene group, a 3-hexenylene group, a 4-hexenylene group, a 5-hexenylene group et al.
   "Lower cycloalkylene group" includes a cycloalkylene group having from 3 to 6 carbon atoms, concretely, for example, a 1,1-cyclopropylene group, a 1,2-cyclopropylene group, a 1,1-cyclobutanylene group, a 1,2-cyclobutanylene group, a 1,3-cyclobutanylene group, a 1,1-cyclopentenylene group, a 1,2-cyclohexenylene group, a 1,3-cyclohexenylene group, a 1,4-cyclohexenylene group et al.
   Examples of the substituent in "lower alkyl group optionally having substituent(s)", "lower cycloalkyl group optionally having substituent(s)", "lower alkylene group optionally having substituent(s)", "methylene group optionally having substituent(s)", "ethylene group optionally having substituent(s)", "C₁₋₄ alkylene group optionally having substituent(s)", "vinylene group optionally having substituent(s)", "lower alkenylene group optionally having substituent(s)" and "lower cycloalkylene group optionally having substituent(s)" may be those selected from a group consisting of group α; and the group may be substituted with one or more such substituents.
   Substituent selected from group consisting of group α:
   A halogen atom, a cyano group, a hydroxyl group, an amino group, a lower alkyl group optionally substituted with a fluorine atom or a hydroxyl group, a mono-lower alkylamino group, a di-lower alkylamino group, a lower alkyloxy group optionally substituted with a fluorine atom, a lower alkyloxy-lower alkyl group, a lower alkyloxycarbonyl group, a lower alkyloxycarbonylamino group, a lower alkyloxycarbonyl(lower alkyl)amino group, a lower alkylcarbonyl group, a lower alkylcarbonyloxy group, a lower alkylcarbonylamino group, a lower alkylcarbonyl(lower alkyl)amino group, a carbamoyl group, a mono-lower alkylcarbamoyl group, a di-lower alkylcarbamoyl group, a carbamoylamino group, a mono-lower alkylcarbamoylamino group, a di-lower alkylcarbamoylamino group, a mono-lower alkylcarbamoyl(lower alkyl)amino group, a di-lower alkylcarbamoyl(lower alkyl)amino group, a carbamoyloxy group, a mono-lower alkylcarbamoyloxy group, a di-lower alkylcarbamoyloxy group, a lower alkylsulfonyl group, a lower alkylsulfonylamino group, a lower alkylsulfonyl(lower alkyl)amino group, a sulfamoyl group, a mono-lower alkylsulfamoyl group, a di-lower alkylsulfamoyl group, a sulfamoylamino group, a mono-lower alkylsulfamoylamino group, a di-lower alkylsulfamoylamino group, a mono-lower alkylsulfamoyl(lower alkyl)amino group, and a di-lower alkylsulfamoyl(lower alkyl)amino group.

   "Aliphatic nitrogen-containing heterocyclic group" includes a 3- to 7-membered monocyclic, or 5 to 12-membered polycyclic, saturated or partially-unsaturated heterocyclic group, containing at least one, preferably from 1 to 3 nitrogen atoms as a part of the ring-constitutive members, and optionally containing from 0 to 2 oxygen atoms or from 0 to 2 sulfur atoms; and concretely, for example, it includes an aziridinyl group, an azetidinyl group, a pyrrolidinyl group, a piperidinyl group, a homopiperazinyl group, a homopiperidinyl group, a morpholinyl group, a thiomorpholinyl group, an octahydocyclopenta[b]pyrrolyl group, a hexahydropyrrolidinyl group, an octahydroindolidinyl group, an octahydroquinolidinyl group, an octahydropyrido[2.1-c]oxazinyl group, a 2,5,6,7-tetrahydro-5H-pyrrolo[1.2-a]imidazolyl group et al.
   "Aromatic carbocyclic group" includes a monocyclic or polycyclic aromatic carbocyclic group having from 6 to 14 carbon atoms, preferably from 6 to 10 carbon atoms, concretely, for example, a phenyl group, a naphthyl group, a phenanthryl group et al.
   "Aromatic heterocyclic group" includes a 5- or 6-membered monocyclic or 8- to 14-membered polycyclic heteroaromatic cyclic group containing at least one, preferably from 1 to 5 hetero atoms selected from a nitrogen atom, an oxygen atom and a sulfur atom as a part of the ring-constitutive members; and concretely, for example, it includes a pyridinyl group, a pyrimidinyl group, a pyridazinyl group, a pyrazyl group, a pyrazolyl group, a pyrrolyl group, an imidazolyl group, a triazolyl group, an oxazolyl group, an isoxazolyl group, an oxadiazolyl group, a thiazolyl group, an isothiazolyl group, a thiadiazolyl group, a tetrazolyl group, a pyridazinyl group, a pyrazinyl group, a furyl group, a thienyl group, an indolyl group, a benzofuranyl group, a benzothienyl group, a benzimidazolyl group, a benzoxazolyl group, a benzisoxazolyl group, a benzothiazolyl group, a benzisothiazolyl group, an indazolyl group, a purinyl group, a quinolyl group, an isoquinolyl group, a phthalazinyl group, a naphthyridinyl group, a quinoxalinyl group, a quinazolinyl group, a cinnolinyl group, a pteridinyl group, a pyrido[3,2-b]pyridyl group et al.

The substituent in "aliphatic nitrogen-containing heterocyclic group optionally having substituent(s)" includes, in addition to the substituents selected from the group consisting of the group α, an oxo group and a lower cycloalkyl group; and the above-mentioned cyclic group may be substituted with one or more such substituents.

The substituent in "aromatic carbocyclic group optionally having substituent(s)" or "aromatic heterocyclic group optionally having substituent(s)" includes, in addition to the substituents selected from the group consisting of the group α, a lower cycloalkyl group; and the above-mentioned cyclic groups may be substituted with one or more such substituents.

The substituent in "methine group optionally having substituent(s)" includes a halogen atom, a lower alkyl group optionally substituted with a halogen atom, a lower alkyloxy group optionally substituted with a halogen atom et al.

The substituent in "lower alkyl group optionally having substituent(s)" defined for R is, for example, preferably a halogen atom, a lower alkoxy group, a lower haloalkoxy group.

"Halogen atom" includes a fluorine atom, a chlorine atom, a bromine atom and an iodine atom.

"Oxo group" means a group (=O) that forms a carbonyl group (C=O) along with the carbon atom in an organic compound.

"Lower alkyl group optionally substituted with a fluorine atom or a hydroxyl group" includes a lower alkyl group, or a lower alkyl group in which a part or all of the hydrogen atoms are substituted with a fluorine atom or a hydroxyl group; The latter lower alkyl group substituted with a fluorine atom or a hydroxyl group includes, for example, a fluoromethyl group, a difluoromethyl group, a trifluoromethyl group, a 2-fluoroethyl group, a 1,2-difluoroethyl group, a 2-hydroxyethyl group, a 1,2-dihydroxyethyl group et al.

"Lower alkyloxy group optionally substituted with a fluorine atom" includes a group including a lower alkyl group and a lower alkyl group substituted with a fluorine atom, bonding to an oxygen atom. Concretely, the lower alkyloxy group includes a methoxy group, an ethoxy group, an n-propyloxy group, an isopropyloxy group, an n-butoxy group, an isobutoxy group, a tert-butoxy group, an n-pentyloxy group; and the lower alkyloxy group substituted with a fluorine atom includes, for example, a fluoromethoxy group, a difluoromethoxy group, a trifluoromethoxy group, a 1,2-difluoroethoxy group et al.

"Mono-lower alkylamino group" is an amino group (-NH₂) in which one hydrogen atom is substituted with a lower alkyl group, concretely, for example, including a methylamino group, an ethylamino group, an n-propylamino group, an isopropylamino group, an n-butylamino group, a sec-butylamino group, a tert-butylamino group et al.

"Di-lower alkylamino group" is an amino group (-NH₂) in which two hydrogen atoms are substituted with lower alkyl groups, concretely, for example, including a dimethylamino group, a diethylamino group, an ethylmethylamino group, a di(n-propyl)amino group, a methyl(n-propyl)amino group, a diisopropylamino group et al.

"Lower alkyloxy-lower alkyl group" is a lower alkyl group substituted with a lower alkyloxy group, and concretely includes, for example, a methoxymethyl group, an ethoxymethyl group, an n-propyloxymethyl group, an isopropyloxymethyl group, a 1-methoxyethyl group, a 2-methoxyethyl group et al.

"Lower alkyloxycarbonyl group" is a lower alkyloxy group bonding to a carbonyl group (-CO-) and includes an alkyloxycarbonyl group having from 1 to 6 carbon atoms, concretely, for example, a methoxycarbonyl group, an ethoxycarbonyl group, an n-propyloxycarbonyl group, an isopropyloxycarbonyl group, an n-butoxycarbonyl group, an isobutoxycarbonyl group, a tert-butoxycarbonyl group, an n-pentyloxycarbonyl group et al.

"Lower alkyloxycarbonylamino group" is a group of an amino group (-NH₂) to which a lower alkyloxycarbonyl group bonds, and includes an alkyloxycarbonylamino group having from 1 to 6 carbon atoms, concretely, for example, a methoxycarbonylamino group, an ethoxycarbonylamino group, an n-propyloxycarbonylamino group, an isopropyloxycarbonylamino group, an n-butoxycarbonylamino group, an isobutoxycarbonylamino group, a tert-butoxycarbonylamino group, an n-pentyloxycarbonylamino group et al.

"Lower alkyloxycarbonyl(lower alkyl)amino group" is a group of a mono-lower alkylamino group in which the hydrogen atom on the nitrogen atom is substituted with a lower alkyloxycarbonyl group bonds and concretely includes, for example, a methoxycarbonyl(methyl)amino group, an ethoxycarbonyl(methyl)amino group, an n-propyloxycarbonyl(methyl)amino group et al.

"Lower alkylcarbonyl group" is a group of a carbonyl group (-CO-) bonding to a lower alkyl group, and includes an alkylcarbonyl group having from 1 to 6 carbon atoms, concretely, for example, an acetyl group, a propionyl group, a butyryl group, an isobutyryl group, a valeryl group, an isovaleryl group, a pivaloyl group et al.

"Lower alkylcarbonyloxy group" is a lower alkylcarbonyl group bonding to an oxygen atom, and concretely includes, for example, an acetoxy group, a propionyloxy group, a valeryloxy group, an isovaleryloxy group, a pivaloyloxy group et al.

"Lower alkylcarbonylamino group" is a group of an amino group (-NH₂) in which one hydrogen atom is substituted with a lower alkylcarbonyl group, and concretely includes, for example, an acetamido group, a propionylamino group, an isobutyrylamino group, a valerylamino group, an isovalerylamino group, a pivaloylamino group et al.

"Lower alkylcarbonyl(lower alkyl)amino group" is a mono-lower alkylamino group in which the hydrogen atom on the nitrogen atom is substituted with a lower alkylcarbonyl group, and includes, for example, a methylcarbonyl(methyl)amino group, an ethylcarbonyl(methyl)amino group, an n-propylcarbonyl(methyl)amino group et al.

"Mono-lower alkylcarbamoyl group" is a carbamoyl group (-CONH₂) in which one hydrogen atom is substituted with a lower alkyl group, and concretely includes, for example, a methylcarbamoyl group, an ethylcarbamoyl group, an n-propylcarbamoyl group, an isopropylcarbamoyl group, an n-butylcarbamoyl group a sec-butylcarbamoyl group, a tert-butylcarbamoyl group et al.

"Di-lower alkylcarbamoyl group" is a carbamoyl group (-CONH₂) in which two hydrogen atoms are substituted with lower alkyl groups, and concretely includes, for example, a dimethylcarbamoyl group, a diethylcarbamoyl group, an ethylmethylcarbamoyl group, a di(n-propyl)carbamoyl group, a methyl(n-propyl)carbamoyl group, a diisopropylcarbamoyl group et al.

"Mono-lower alkylcarbamoylamino group" is an amino group (-NH₂) in which one hydrogen atom is substituted with a mono-lower alkylcarbamoyl group, and concretely includes, for example, a methylcarbamoylamino group, an ethylcarbamoylamino group, an n-propylcarbamoylamino group, an isopropylcarbamoylamino group, an n-butylcarbamoylamino group, a sec-butylcarbamoylamino group, a tert-butylcarbamoylamino group et al.

"Di-lower alkylcarbamoylamino group" is an amino group (-NH₂) in which one hydrogen atom is substituted with a di-lower alkylcarbamoyl group, and concretely includes, for example, a dimethylcarbamoylamino group, a diethylcarbamoylamino group, a di(n-propyl)carbamoylamino group, a diisopropylcarbamoylamino group, a di(n-butyl)carbamoylamino group, a di(sec-butyl)carbamoylamino group, a di(tert-butyl)carbamoylamino group et al.

"Mono-lower alkylcarbamoyl(lower alkyl)amino group" is a mono-lower alkylamino group in which the hydrogen atom on the nitrogen atom is substituted with a mono-lower alkylcarbamoyl group, and concretely includes, for example, a monomethylcarbamoyl(methyl)amino group, a monoethylcarbamoyl(methyl)amino group, a [mono(n-propyl)carbamoyl](methyl)amino group et al.

"Di-lower alkylcarbamoyl(lower alkyl)amino group" is a mono-lower alkylamino group in which the hydrogen atom on the nitrogen atom is substituted with a di-lower alkylcarbamoyl group, and concretely includes, for example, a dimethylcarbamoyl(methyl)amino group, a diethylcarbamoyl(methyl)amino group, a [di(n-propyl)carbamoyl](methyl)amino group et al.

"Mono-lower alkylcarbamoyloxy group" is a mono-lower alkylcarbamoyl group bonding to an oxygen atom, and concretely includes, for example, a methylcarbamoyloxy group, an ethylcarbamoyloxy group, an n-propylcarbamoyloxy group, an isopropylcarbamoyloxy group, an n-butylcarbamoyloxy group, a sec-butylcarbamoyloxy group, a tert-butylcarbamoyloxy group et al.

"Di-lower alkylcarbamoyloxy group" is a di-lower alkylcarbamoyl group bonding to an oxygen atom, and concretely includes, for example, a dimethylcarbamoyloxy group, a diethylcarbamoyloxy group, an ethylmethylcarbamoyloxy group, a di(n-propyl)carbamoyloxy group, a methyl(n-propyl)carbamoyloxy group, a diisopropylcarbamoyloxy group et al.

"Lower alkylsulfonyl group" is a lower alkyl group bonding to a sulfonyl group (-SO₂-), and concretely includes, for example, a methylsulfonyl group, an ethylsulfonyl group, an n-propylsulfonyl group, an isopropylsulfonyl group, an n-butylsulfonyl group, a sec-butylsulfonyl group, a tert-butylsulfonyl group et al.

"Lower alkylsulfonylamino group" is an amino group (-NH₂) in which one hydrogen atom is substituted with a lower alkylsulfonyl group, and concretely includes, for example, a methylsulfonylamino group, an ethylsulfonylamino group, an n-propylsulfonylamino group, an isopropylsulfonylamino group, an n-butylsulfonylamino group, a sec-butylsulfonylamino group, a tert-butylsulfonylamino group et al.

"Lower alkylsulfonyl(lower alkyl)amino group" is a group of a mono-lower alkylamino group in which the hydrogen atom on the nitrogen atom is substituted with a lower alkylsulfonyl group, and concretely includes, for example, a methanesulfonyl group, an ethanesulfonyl group, an n-propanesulfonyl group, an isopropanesulfonyl group et al.

"Mono-lower alkylsulfamoyl group" is a group of a sulfamoyl group (-SO₂NH₂) in which one hydrogen atom is substituted with a lower alkyl group, and concretely includes, for example, a monomethylsulfamoyl group, a monoethylsulfamoyl group, a mono(n-propyl)sulfamoyl group, a monoisopropylsulfamoyl group, a mono(n-butyl)sulfamoyl group, a mono(sec-butyl)sulfamoyl group, a mono(tert-butyl)sulfamoyl group et al.

"Di-lower alkylsulfamoyl group" is a group of a sulfamoyl group (-SO₂NH₂) in which two hydrogen atoms are substituted with lower alkyl groups, and concretely includes, for example, a dimethylsulfamoyl group, a diethylsulfamoyl group, a di(n-propyl)sulfamoyl group, a diisopropylsulfamoyl group, a di(n-butyl)sulfamoyl group, a di(sec-butyl)sulfamoyl group, a di(tert-butyl)sulfamoyl group et al.

"Mono-lower alkylsulfamoylamino group" is a group of an amino group (-NH₂) in which one hydrogen atom is substituted with a mono-lower alkylsulfamoyl group, and concretely includes, for example, a (monomethylsulfamoyl)amino group, a (monoethylsulfamoyl)amino group, a [mono(n-propyl)sulfamoyl]amino group, a (monoisopropylsulfamoyl)amino group, a [mono(n-butyl)sulfamoyl]amino group, a [(mono-sec-butyl)sulfamoyl]amino group, a [(mono-tert-butyl)sulfamoyl] amino group et al.

"(Di-lower alkylsulfamoyl)amino group" is a group of an amino group (-NH₂) in which one hydrogen atom is substituted with a di-lower alkylsulfamoyl group, and concretely includes, for example, a (dimethylsulfamoyl)amino group, a (diethylsulfamoyl)amino group, an (ethylmethylsulfamoyl)amino group, a [di(n-propyl)sulfamoyl]amino group, a [methyl(n-propyl)sulfamoyl]amino group, a (diisopropylsulfamoyl)amino group et al.

"Mono-lower alkylsulfamoyl(lower alkyl)amino group" is a group of a mono-lower alkylamino group in which the hydrogen atom on the nitrogen atom is substituted with a mono-lower alkylsulfamoyl group, and concretely includes, for example, a monomethylsulfamoyl(methyl)amino group, a monoethylsulfamoyl(methyl)amino group, a [mono(n-propyl)sulfamoyl](methyl)amino group et al.

"Di-lower alkylsulfamoyl(lower alkyl)amino group" is a group of a mono-lower alkylamino group in which the hydrogen atom on the nitrogen atom is substituted with a di-lower alkylsulfamoyl group, and concretely includes, for example, a dimethylsulfamoyl(methyl)amino group, a diethylsulfamoyl(methyl)amino group, a [di(n-propyl)sulfamoyl](methyl)amino group et al.

The bicyclic aromatic carbon ring includes a naphthalene ring.

The bicyclic aromatic hetero ring includes a quinoline ring, an isoquinoline ring, a quinazoline ring, a cinnoline ring, a quinoxaline ring, a 1,5-naphthyridine ring, a 1,8-naphthyridine ring, a 2,6-naphthyridine ring, a 1,7-naphthyridine ring, an imidazo[1,2-a]pyridine ring, a benzimidazole ring, an indazole ring, an indole ring, a benzofuran ring, a benzothiophene ring, a benzoxazole ring, a benzothiazole ring et al.

The substituent in "bicyclic aromatic carbocyclic group optionally having substituent(s)" or "bicyclic aromatic heterocyclic group optionally having substituent(s)" includes, for example, a halogen atom, a lower alkyl group optionally substituted with a halogen atom, a lower alkyloxy group optionally substituted with a halogen atom.

"Pharmaceutically-acceptable salts" of a pyridone derivative of formula [I] mean ordinary salts that are acceptable as medicines. Their examples are acid-addition salts to the amine group of the compound of formula (I) or acid-addition salts to the nitrogen-containing hetero ring thereof.

The acid-addition salts include inorganic acid salts such as hydrochlorides, sulfates, nitrates, phosphates, perchlorates et al; organic acid salts such as maleates, fumarates, tartrates, citrates, ascorbates, trifluoroacetates et al; and sulfonates such as methanesulfonates, isethionates, benzenesulfonates, p-toluenesulfonates et al.

For the purpose of more concretely disclosing the pyridone derivatives of the invention hereinunder, various symbols used in formula (I) are described in detail with reference to their examples.

In formula (I), X₁, X₂ and X₃ are the same or different, each representing a methine group optionally having substituent(s), or a nitrogen atom, and all of X₁, X₂ and X₃ are not nitrogen atoms at the same time.

X₁, X₂ and X₃ are concretely as follows:
X₁, X₂ and X₃ are all methine groups optionally having substituent(s);
X₁ and X₂ are methine groups optionally having substituent(s), and X₃ is a nitrogen atom;
X₁ and X₃ are methine groups optionally having substituent(s), and X₂ is a nitrogen atom;
X₂ and X₃ are methine groups optionally having substituent(s), and X₁ is a nitrogen atom.

Preferably, they are all methine groups optionally having substituent(s).

The substituent in "methine group optionally having substituent(s)" is preferably a halogen atom such as a fluorine atom, a chlorine atom, a bromine atom et al; a lower alkyl group such as a methyl group, an ethyl group, an n-propyl group, an isopropyl group et al.
Y₁ represents a single bond, -O-, -NR-, -S-, -SO- or -SO₂-,
Y₂ represents a lower alkylene group optionally having substituent(s), a lower alkenylene group optionally having substituent(s), or a lower cycloalkylene group optionally having substituent(s);
Y₃ represents a single bond, -O-, -NR-, -S-, -SO- or -SO₂-;
R each independently represents a hydrogen atom, or a lower alkyl group optionally having substituent(s).

Examples of R include a methyl group, an ethyl group, an n-propyl group, an isopropyl group, n-butyl group and t-butyl group, in addition to a hydrogen atom.
Y₁ is preferably a single bond or -O-.
Y₂ is preferably a methylene group optionally having substituent(s), a vinylene group optionally having substituent(s), or an ethylene group optionally having substituent(s), and more preferably, an unsubstituted methylene, ethylene or vinylene group.
Y₃ is preferably a single bond or -O-.

Preferred combinations of -Y₁-Y₂-Y₃ are:
-lower alkylene-O-,
-lower alkylene-,
-lower alkenylene-,
-lower cycloalkylene-,
-O-lower alkylene-O-, et al; more preferably,
-CH₂-O-,
-CH=CH-,
-CH₂-CH₂-
-CH₂-CH₂-O-,
-O-CH₂-CH₂-O-, et al are recommended.

W₁, W₂, W₃ and W₄ are the same or different, each representing a single bond, a methylene group optionally having substituent(s), or -O-; provided that continuing two or more of W₁, W₂, W₃ and W₄ are not -O- at the same time.

In this, "continuing two or more are not -O- at the same time" means, for example, that continuing two or more such as W₁ and W₂ are not -O- at the same time, or that is -W₁-W₂- is not -O-O-.

W₁ is preferably a single bond, -O-, or a methylene group optionally having substituent(s).

W₂ is preferably a single bond, or a methylene group optionally having substituent(s).

W₃ is preferably a single bond.

W₄ is preferably a single bond.

Preferred combinations of -W₁-W₂-W₃-W₄- are:
-single bond-,
-O-
-O-CH₂-
-CH₂-
-CH₂-CH₂-
-CH₂-CHF-
-CH₂-CF₂- et al,
more preferably, a single bond or -O-CH₂-.

L represents a single bond, a methylene group optionally having substituent(s), or an ethylene group optionally having substituent(s); and L may form, taken together with Z₂, R₁ and the nitrogen atom to which R₁ bonds, an aliphatic nitrogen-containing hetero ring optionally having substituent(s).

Z₁ and Z₂ are the same or different, each representing a single bond, -O-, or a C₁₋₄ alkylene group optionally having substituent(s).

L is preferably a single bond, a methylene group optionally having substituent(s), or an ethylene group optionally having substituent(s). The substituent of the optionally-substituted methylene or ethylene group is preferably a methyl group.

Z₁ is preferably a single bond, a methylene group optionally having substituent(s), or -O-. The substituent of the optionally-substituted methylene group is preferably a methyl group.

Z₂ is preferably a single bond, or a methylene group optionally having substituent(s). The substituent of the optionally-substituted methylene group is preferably a methyl group.

Preferred combinations of -Z₁-L-Z₂- are:
-single bond-
-CH₂-
-CH(CH₃)-
-CH₂-CH₂-
-CH₂-CH(CH₃)-
-CH₂-CH₂-CH₂-
-O-CH₂-CH₂-
-O-CH₂-CH(CH₃)-
-O-CH₂-CH₂-CH₂-, et al,
more preferably,
-CH₂-
-CH(CH₃)-
-CH₂-CH₂-
-CH₂-CH(CH₃)-
-CH₂-CH₂-CH₂-
-O-CH₂-CH₂-
-O-CH₂-CH(CH₃)-
-O-CH₂-CH₂-CH₂- et al.

On the other hand, when L forms, taken together with Z₂, R₁ and the nitrogen atom to which R₁ bonds, an aliphatic nitrogen-containing hetero ring optionally having substituent(s), its preferred examples are an azetidine ring, a pyrrolidine ring and a piperidine ring. The group with which the ring may be substituted includes a methyl group, an ethyl group, an isopropyl group, a cyclobutyl group, a cyclopentyl group et al.

R₁ and R₂ are the same or different, each representing a hydrogen atom, a lower alkyl group optionally having substituent(s), or a lower cycloalkyl group optionally having substituent(s); or R₁ and R₂ form, taken together with the nitrogen atom to which they bond, an aliphatic nitrogen-containing hetero ring optionally having substituent(s).

Preferably, R₁ and R₂ are the same or different, each representing a hydrogen atom, a methyl group, an ethyl group, an n-propyl group, an isopropyl group, an isobutyl group, a cyclopropyl group et al.

Preferred examples of the aliphatic nitrogen-containing hetero ring optionally having substituent(s), which is formed by R₁ and R₂ taken together with the nitrogen atom to which they bond, are an azetidine ring optionally having substituent(s), a pyrrolidine ring optionally having substituent(s), a piperidine ring optionally having substituent(s), a hexamethyleneimine ring optionally having substituent(s), a morpholine ring optionally having substituent(s) et al, more preferably an azetidine ring optionally having substituent(s) and a pyrrolidine ring optionally having substituent(s).

Preferred examples of the substituent of "aliphatic nitrogen-containing hetero ring optionally having substituent(s)" are a methyl group, a fluorine atom, an oxo group, a hydroxyl group, a methoxy group, a methoxymethyl group, a fluoromethyl group, a 2-fluoroethoxy group, a dimethylamino group et al, more preferably a methyl group, a hydroxyl group, a fluorine atom et al.

Ar₁ represents an aromatic carbocyclic group optionally having substituent(s), or an aromatic heterocyclic group optionally having substituent(s).

Preferred examples of the substituent of "aromatic carbocyclic group optionally having substituent(s)" or "aromatic heterocyclic group optionally having substituent(s)" are a fluorine atom, a chlorine atom, a methyl group, an ethyl group, a methoxy group, a trifluoromethyl group, a difluoromethoxy group, a trifluoromethoxy group et al.

Preferred examples of Ar₁ are phenyl, naphthyl, pyridinyl, pyrimidinyl, pyridazinyl, pyrazyl, pyrazole, pyrrolyl, imidazolyl, triazolyl, oxadiazolyl, isoxazolyl, oxadiazolyl, thiazolyl, isothiazolyl, thiadiazolyl, tetrazolyl et al.

More preferably, phenyl optionally having substituent(s), naphthyl optionally having substituent(s), or pyridinyl optionally having substituent(s); even more preferably, phenyl, 4-chlorophenyl, 4-fluorophenyl, 4-trifluoromethoxyphenyl, 2-pyridinyl, 5-fluoro-2-pyridinyl, 5-chloro-2-pyridinyl are recommended.

Ar₂ is a divalent group, representing a bicyclic aromatic carbocyclic group optionally having substituent(s), or a bicyclic aromatic heterocyclic group optionally having substituent(s).

The substituent of "bicyclic aromatic carbocyclic group optionally having substituent(s)" or "bicyclic aromatic heterocyclic group optionally having substituent(s)" is preferably a methyl group.

The aromatic carbon cycle of the bicyclic aromatic carbocyclic group for Ar₂ is concretely a naphthalene ring; and the aromatic hetero ring of the bicyclic aromatic heterocyclic group includes quinoline, isoquinoline, quinazoline, cinnoline, quinoxaline, 1,5-naphthyridine, 1,8-naphthyridine, 2,6-naphthyridine, 1,7-naphthyridine, imidazo[1,2-a]pyridine, benzimidazole, indazole, indole, benzofuran, benzothiophene, benzoxazole, benzothiazole et al.

Above all, preferred are naphthalene, quinoline, isoquinoline, quinoxaline, quinazoline, 1,5-naphthyridine, imidazo[1,2-a]pyridine, indazole, benzimidazole, indole, benzofuran et al; more preferred are naphthalene, quinoline, isoquinoline, quinoxaline, benzimidazole, indole, benzofuran et al.

Preferred examples of Ar₂ are the following formulae:

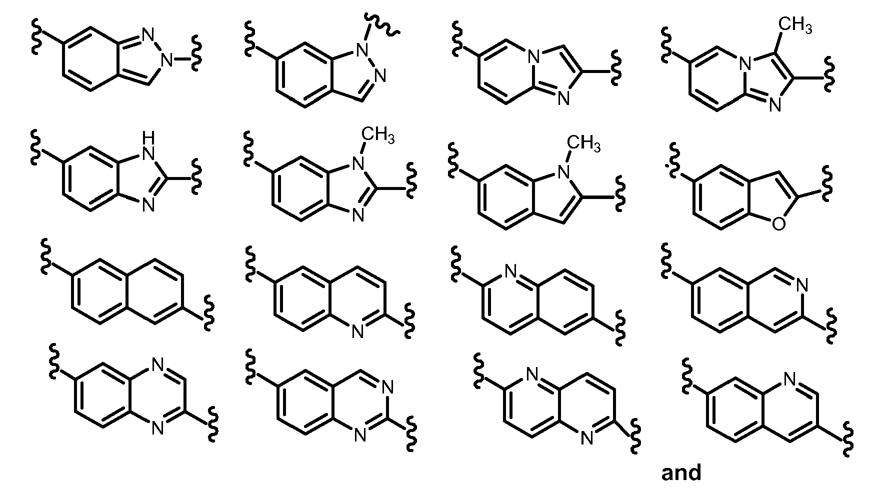

More preferred are the following:

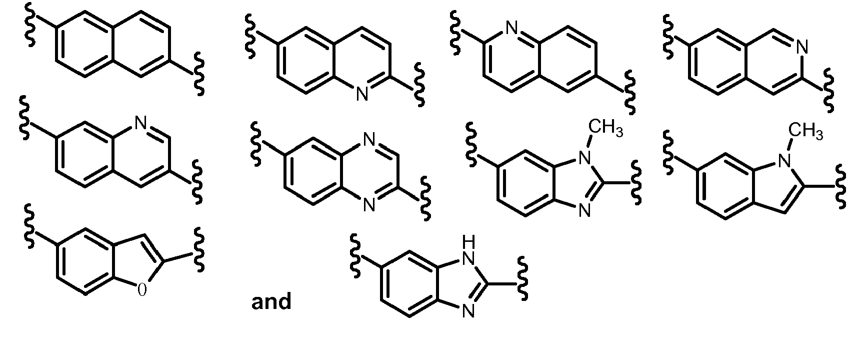

Examples of the compounds of the invention are shown in the following Table 1 to Table 6.

**Table 1**

| Example | Structural Formula | Example | I Structural Formula |
|---|---|---|---|
| 1 | 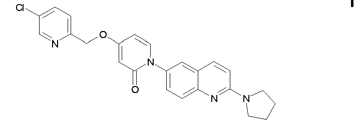 | 10 | 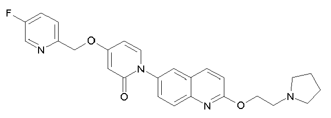 |
| 2 | 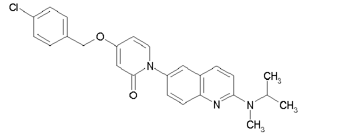 | 11 | 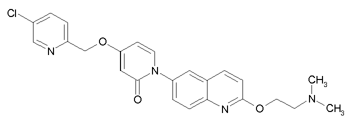 |
| 3 | 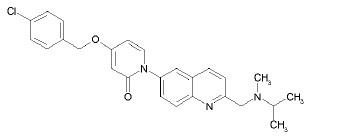 | 12 | 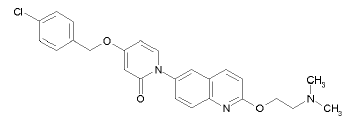 |
| 4 | 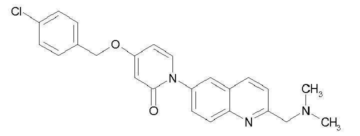 | 13 | 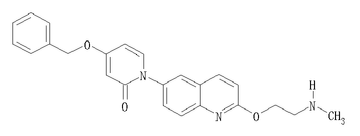 |
| 5 | 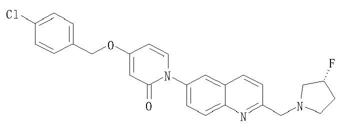 | 14 | 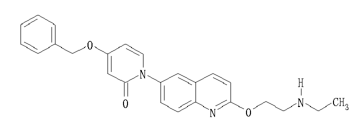 |
| 6 | 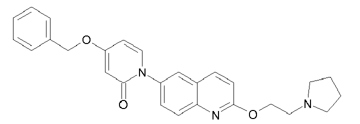 | 15 | 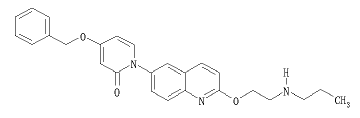 |
| 7 | 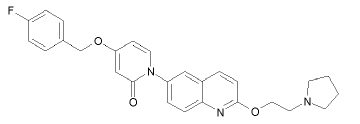 | 16 | 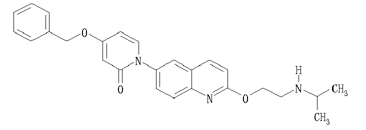 |
| 8 | 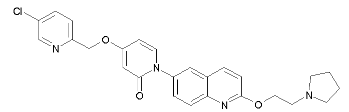 | 17 | 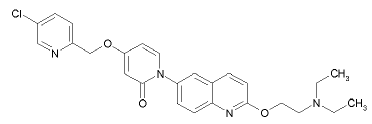 |
| 9 | 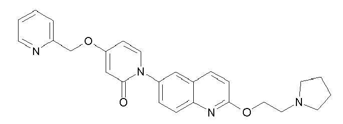 | 18 | 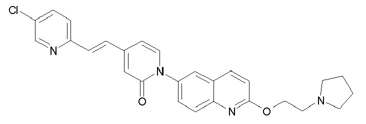 |

**Table 2**

| Example | Structural Formula | Example | Structural Formula |
|---|---|---|---|
| 19 | 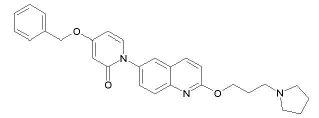 | 28 | 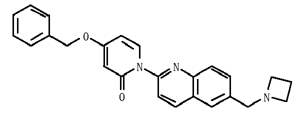 |
| 20 | 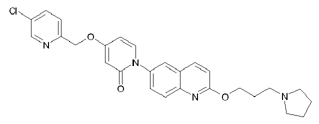 | 29 | 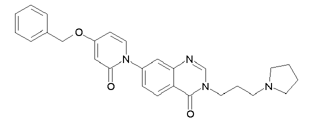 |
| 21 | 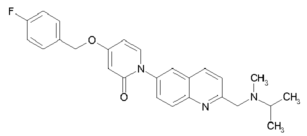 | 30 | 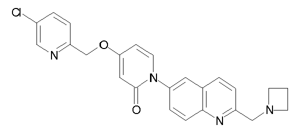 |
| 22 | 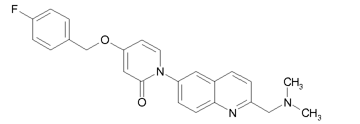 | 31 | 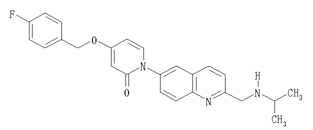 |
| 23 | 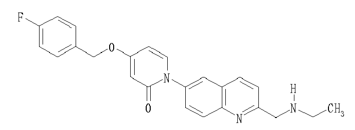 | 32 | 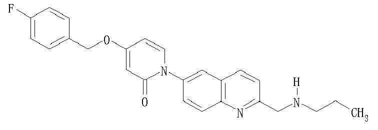 |
| 24 | 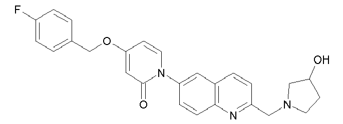 | 33 | 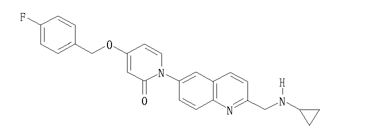 |
| 25 | 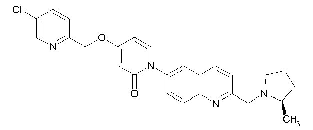 | 34 | 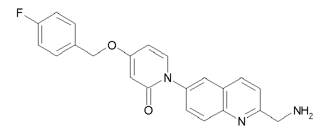 |
| 26 | 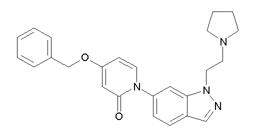 | 35 | 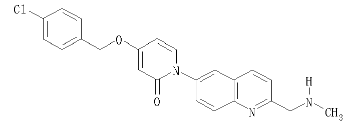 |
| 27 | 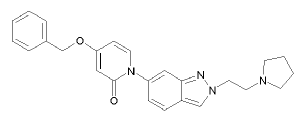 | 36 | 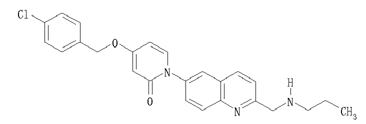 |

**Table 3**

| Example | Structural Formula | Example | Structural Formula |
|---|---|---|---|
| 37 | 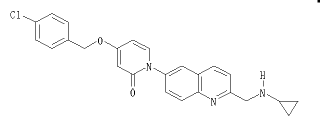 | 46 | 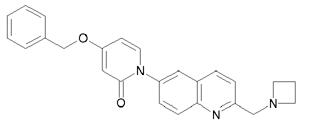 |
| 38 | 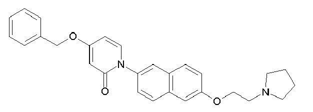 | 47 | 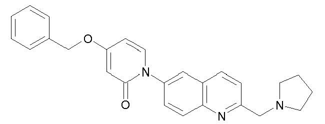 |
| 39 | 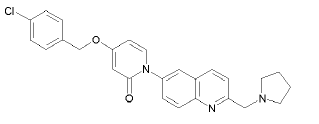 | 48 | 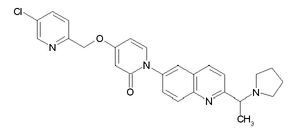 |
| 40 | 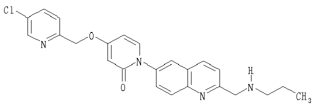 | 49 | 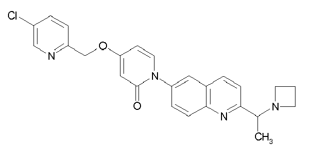 |
| 41 | 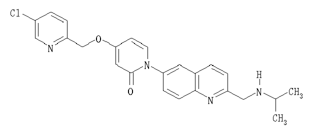 | 50 | 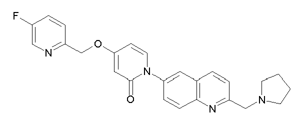 |
| 42 | 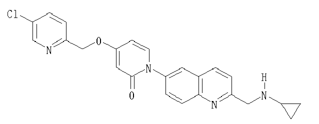 | 51 | 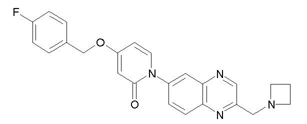 |
| 43 | 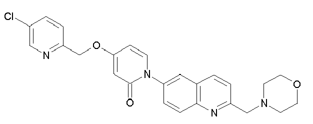 | 52 | 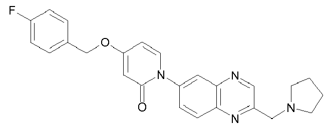 |
| 44 | 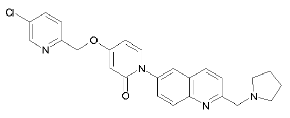 | 53 | 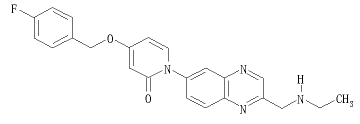 |
| 45 | 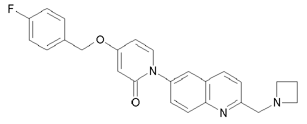 | 54 | 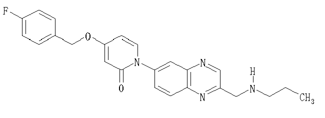 |

**Table 4**

| Example | Structural Formula | Example | Structural Formula |
|---|---|---|---|
| 55 | 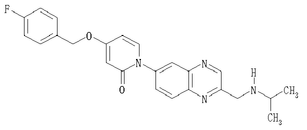 | 64 | 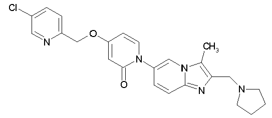 |
| 56 | 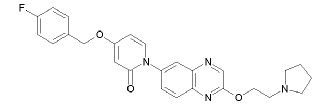 | 65 | 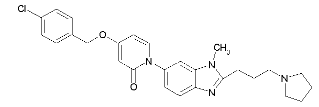 |
| 57 | 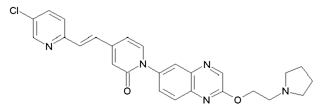 | 66 | 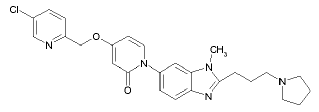 |
| 58 | 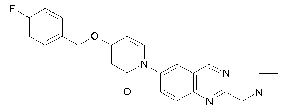 | 67 | 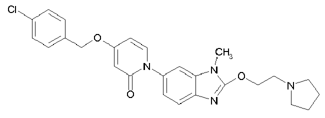 |
| 59 | 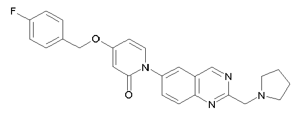 | 68 | 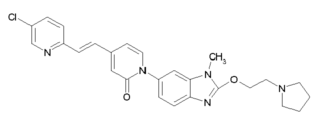 |
| 60 | 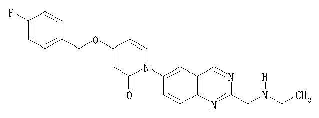 | 69 | 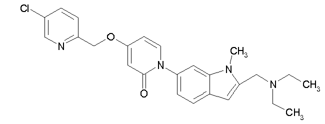 |
| 61 | 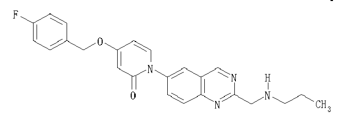 | 70 | 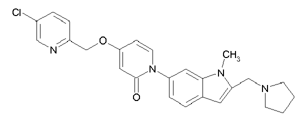 |
| 62 | 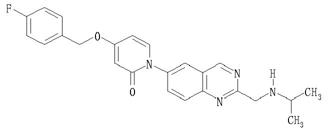 | 71 | 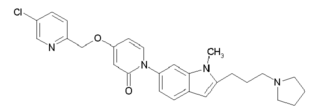 |
| 63 | 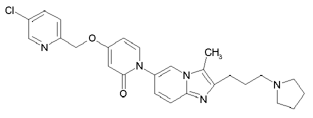 | 72 | 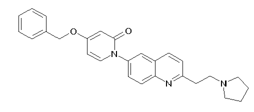 |

**Table 5**

| Example | Structural Formula | Example | Structural Formula |
|---|---|---|---|
| 73 | 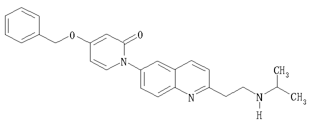 | 82 | 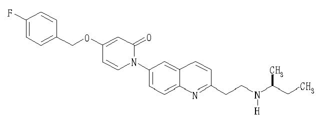 |
| 74 | 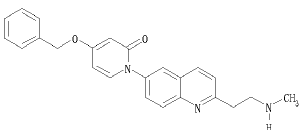 | 83 | 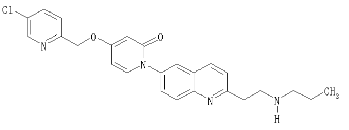 |
| 75 | 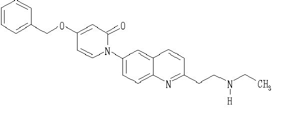 | 84 | 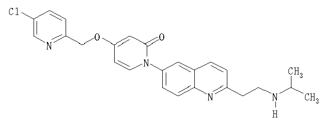 |
| 76 | 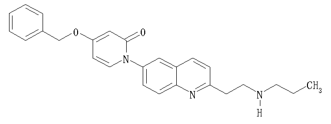 | 85 | 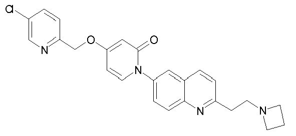 |
| 77 | 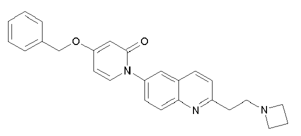 | 86 | 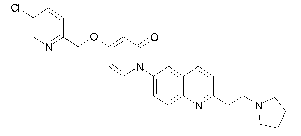 |
| 78 | 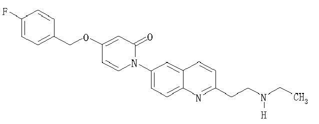 | 87 | 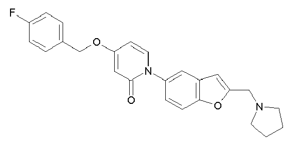 |
| 79 | 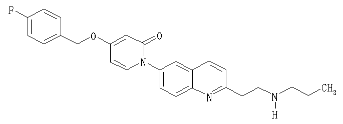 | 88 | 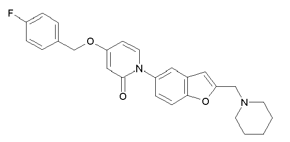 |
| 80 | 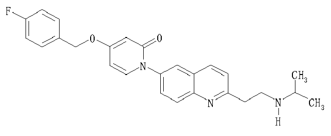 | 89 | 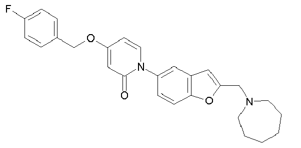 |
| 81 | 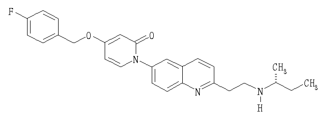 | | 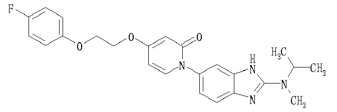 |

**Table 6**

| Example | Structural Formula | Example | Structural Formula |
|---|---|---|---|
| 91 | 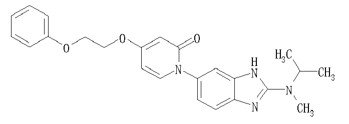 | 97 | 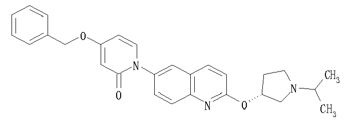 |
| 92 | 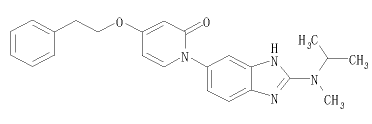 | 98 | 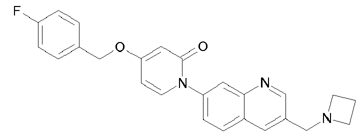 |
| 93 | 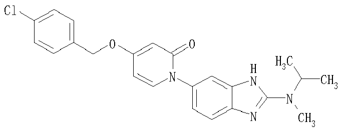 | 99 | 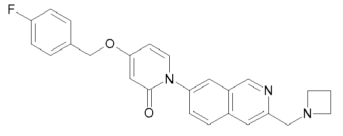 |
| 94 | 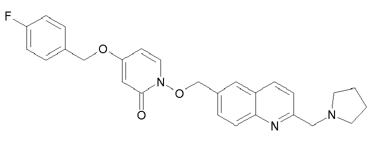 | 100 | 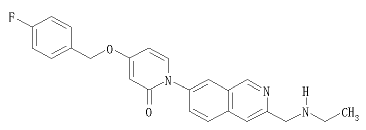 |
| 95 | 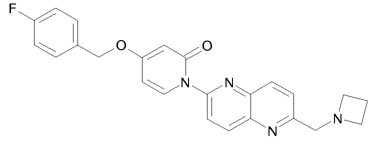 | 101 | 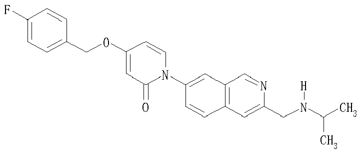 |
| 96 | 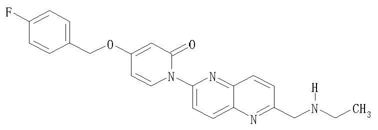 | | |

Preferred examples of the compounds of the invention are:
1-[6-(azetidin-1-ylmethyl)quinolin-2-yl]-4-(benzyloxy)pyridin-2(1H)-one,
4-(benzyloxy)-1-[6-(2-pyrrolidin-1-ylethoxy)-2-naphthyl]pyridin-2(1H)-one,
1-[3-(azetidin-1-ylmethyl)isoquinolin-7-yl]-4-[(4-fluorobenzyl)oxy]pyridin-2(1H)-one,
4-[(4-chlorobenzyl)oxy]-1-{2-[(methylamino)methyl]quinolin-6-yl}pyridin-2(1H)-one,
1-{2-[(ethylamino)methyl]quinolin-6-yl}-4-[(4-fluorobenzyl)oxy]pyridin-2(1H)-one,
4-[(4-fluorobenzyl)oxy]-1-{2-[(propylamino)methyl]quinolin-6-yl}pyridin-2(1H)-one,
4-[(5-chloropyridin-2-yl)methoxy]-1-{2-[(propylamino)methyl]quinolin-6-yl}pyridin-2(1H)-one,
4-[(4-fluorobenzyl)oxy]-1-{2-[(isopropylamino)methyl]quinolin-6-yl}pyridin-2(1H)-one,
1-[2-(azetidin-1-ylmethyl)quinolin-6-yl]-4-[(5-chloropyridin-2-yl)methoxy]pyridin-2(1H)-one,
4-[(5-chloropyridin-2-yl)methoxy]-1-[2-(pyrrolidin-1-ylmethyl)quinolin-6-yl]pyridin-2(1H)-one,
4-(benzyloxy)-1-{2-[2-(propylamino)ethyl]quinolin-6-yl}pyridin-2(1H)-one,
4-[(5-chloropyridin-2-yl)methoxy]-1-{2-[2-(dimethylamino)ethoxy]quinolin-6-yl}pyridin-2(1H)-one,
4- [(5-chloropyridin-2-yl)methoxy]-1-[2-(2-pyrrolidin-1-ylethoxy)quinolin-6-yl]pyridin-2(1H)-one,
4-[(5-chloropyridin-2-yl)methoxy]-1-[2-(3-pyrrolidin-1-ylpropoxy)quinolin-6-yl]pyridin-2(1H)-one,
4-[(E)-2-(5-chloropyridin-2-yl)vinyl]-1-[2-(2-pyrrolidin-1-ylethoxy)quinoxalin-6-yl]pyridin-2(1H)-one,
4-[(4-chlorobenzyl)oxy]-1-[1-methyl-2-(3-pyrrolidin-1-ylpropyl)-1H-benzimidazol-6-yl]pyridin-2(1H)-one,
4-[(5-chloropyridin-2-yl)methoxy]-1-[1-methyl-2-(pyrrolidin-1-ylmethyl)-1H-indol-6-yl]pyridin-2(1H)-one,
4-[2-(4-fluorophenoxy)ethoxy]-1-{2-[isopropyl(methyl)ammo]-1H-benzimidazol-6-yl}pyridin-2(1H)-one,
1-[3-(azetidin-1-ylmethyl)quinolin-7-yl]-4-[(4-fluorobenzyl)oxy]pyridin-2(1H)-one,
4-[(4-fluorobenzyl)oxy]-1-[2-(pyrrolidin-1-ylmethyl)quinazolin-6-yl]pyridin-2(1H)-one,
1-[6-(azetidin-1-ylmethyl)-1,5-naphthyridin-2-yl]-4- [(4-fluorobenzyl)oxy]pyridin-2(1H)-one,
4-(benzyloxy)-1-[2-(2-pyrrolidin-1-ylethyl)-2H-indazol-6-yl]pyridin-2(1H)-one,
4-[(5-chloropyridin-2-yl)methoxy]-1-[3-methyl-2-(3-pyrrolidin-1-ylpropyl)imidazo[1,2-a]pyridin-6-yl]pyridin-2(1H)-one,
1-[2-(azepan-1-ylmethyl)-1-benzofuran-5-yl]-4-[(4-fluorobenzyl)oxy]pyridin-2(1H)-one, and
4-[(4-fluorobenzyl)oxy]-1-{[2-(pyrrolidin-1-ylmethyl)quinolin-6-yl]methoxy}pyridin-2(1H)one, et al.

More preferred are:
1-{2-[(ethylamino)methyl]quinolin-6-yl}4-[(4-fluorobenzyl)oxy]pyridin-2(1H)-one,
1-[2-(azetidin-1-ylmethyl)quinolin-6-yl]-4-[(5-chloropyridin-2-yl)methoxy]pyridin-2(1H)-one,
4-(benzyloxy)-1-{2-[2-(propylamino)ethyl]quinolin-6-yl}pyridin-2(1H)-one,
4-[(5-chloropyridin-2-yl)methoxy]-1-[2-(2-pyrrolidin-1-ylethoxy)quinolin-6-yl]pyridin-2(1H)-one,
4-[(4-fluorobenzyl)oxy]-1-{2-[(propylamino)methyl]quinolin-6-yl}pyridin-2(1H)-one,
4-[(5-chloropyridin-2-yl)methoxy]-1-{2-[(propylamino)methyl]quinolin-6-yl}pyridin-2(1H)-one, and
4-[(4-fluorobenzyl)oxy]-1-{2-[(isopropylamino)methyl]quinolin-6-yl}pyridin-2(1H)-one, et al.

### Production Methods for Compounds of Formula (I)

The compounds of formula (I) may be produced, for example, according to the following production methods, to which, however, the invention should not be limited. Production Method 1:

Production method 1 is for obtaining a compound in which W₁ to W₄ are all single bonds, or that is, a compound of a formula (Ia). 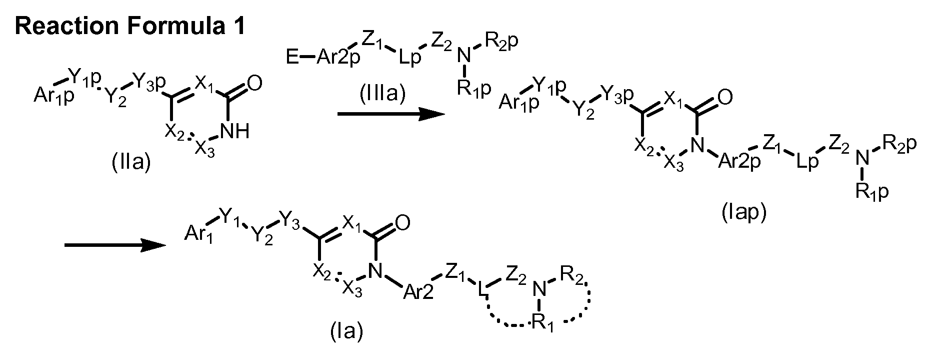 [In the formula, R₁p represents an optionally-protected R₁; R₂p represents an optionally-protected R₂; Y₁p represents an optionally-protected Y₁; Y₃p represents an optionally-protected Y₃; Lp represents an optionally-protected L; Ar₁p represents an optionally-protected Ar₁; Ar₂p represents an optionally-protected Ar₂; E represents a halogen atom such as a chlorine atom, a bromine atom or an iodine atom, or (HO)₂-B- or (R')₃Sn; R' represents an alkyl group having from 1 to 8 carbon atoms; R₁, R₂, Y₁, Y₂, Y₃, Z₁, L, Z₂, Ar₁, Ar₂, X₁, X₂ and X₃ have the same meanings as above.]

A compound of a formula (IIa) is condensed with a compound of a formula (IIIa) in an organic solvent preferably in the presence of a base, and in the presence of a metal catalyst to obtain a compound of a formula (Iap).

The amount of the compound of formula (IIIa) to be used may be from 0.5 to 2.0 mols relative to 1 mol of the compound of formula (IIa), preferably from 0.5 mols to 1.0 mol.

The base includes, for example, sodium acetate, potassium acetate, sodium carbonate, sodium hydrogencarbonate, potassium carbonate, cesium carbonate, tripotassium phosphate, triethylamine, pyridine et al.

The amount of the base to be used may be from an equimolar amount to an excessive molar amount relative to 1 mol of the compound of formula (IIa), preferably from 1.0 mol to 5.0 mols.

The metal catalyst includes copper(0), copper(I) chloride, copper(II) chloride, copper(I) bromide, copper(II) bromide, copper(I) iodide, copper(II) iodide, copper(II) acetate, copper(I) oxide, copper(II) oxide et al, preferably copper(O), copper(I) iodide, copper(II) acetate.

The amount of the metal catalyst to be used may be from 0.01 to an excessive molar amount relative to 1 mol of the compound of formula (IIa), preferably from 0.05 mols to 5 mols.

The organic solvent includes 1,4-dioxane (hereinafter referred to as "dioxane"), benzene, toluene, nitrobenzene, N,N-dimethylformamide (hereinafter referred to as "DMF"), N-methylpyrrolidone (hereinafter referred to as "NMP"), dichloromethane et al.

The reaction temperature may be from 0°C to 300°C, preferably from 20°C to 200°C, and in general, the reaction will be completed in 1 hour to 72 hours, preferably in 2 hours to 48 hours.

In case where the compound of formula (lap) has a protective group, the protective group may be removed to give a compound of formula (Ia).

The removal of the protective group, though differing depending on the type of the protective group and the stability of the compound of formula (Iap), may be attained, for example, according to methods described in literature [see Protective Groups in Organic Synthesis, T. W. Greene, John Wiley & Sons, 1981], concretely, through solvolysis with acid or base, for example, with from 0.01 mol to a large excessive amount of an acid, preferably trifluoroacetic acid (hereinafter referred to as "TFA"), formic acid, hydrochloric acid et al, or with from an equimolar amount to a large excessive amount of a base, preferably potassium hydroxide, calcium hydroxide et al; or through chemical reduction with a metal hydride complex, or through catalytic reduction with a palladium-carbon catalyst or a Raney-nickel catalyst et al.

Thus obtained, the compound of formula (Ia) may be isolated and purified according to a known separation and purification method of, for example, concentration, concentration under reduced pressure, crystallization, solvent extraction, reprecipitation or chromatography et al (the same shall apply to the reactions described below).

The compound of formula (IIa) may be a commercial reagent or may be prepared according to the methods described in Examples.

Examples of the compound of formula (IIIa) are mentioned below, and they may be prepared according to the methods described in Examples.

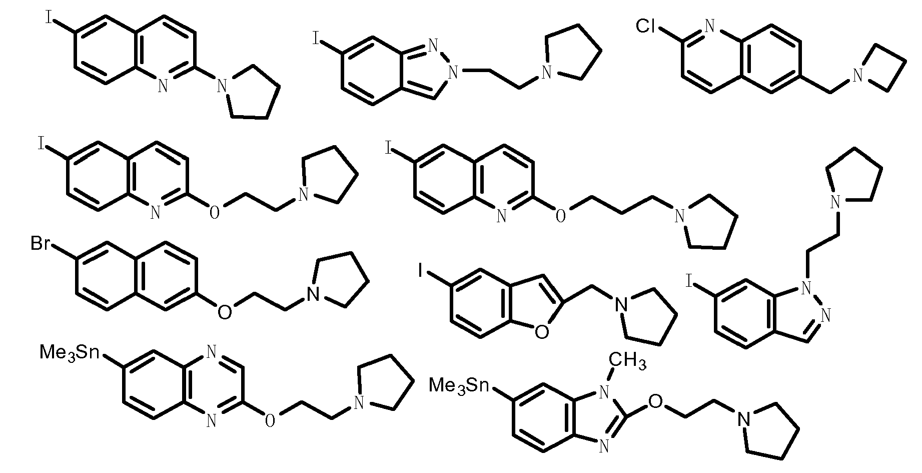

### Production Method 2:

Production Method 2 is for producing a compound of formula (Ia) where Y₃ is an oxygen atom, or that is, a compound of a formula (Ib). 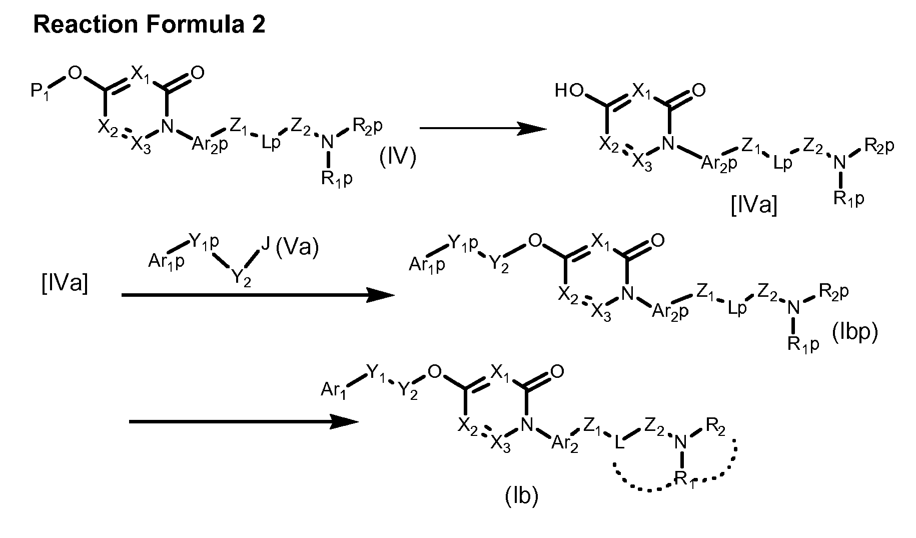 [In the formula, P₁ represents a protective group for hydroxyl group; J represents a leaving group or a hydroxyl group; Ar₁p, Ar₂p, R₁p, R₂p, R₁, R₂, Y₁p, Y₁, Y₂, Z₁, Lp, L, Z₂, Ar₁, Ar₂, X₁, X₂ and X₃ have the same meanings as above.]

The protective group P₁ is removed from the compound of formula (IV) to give a compound of a formula (IVa), then the compound of formula (IVa) is condensed with a compound of a formula (Va) to obtain a compound of a formula (Ibp).

The removal of the protective group P₁ may be attained according to the methods described in Protective Groups in Organic Synthesis.

The protective group P₁ is described below.

The condensation of a compound of formula (IVa) with a compound of formula (Va) may be attained according to the methods described below, though varying depending on the type of the compound of formula (Va).
1) In case where J is a leaving group:
   A compound of formula (IVa) and a compound of formula (Va) are condensed in an organic solvent, preferably in the presence of a base to obtain a compound of formula (Ibp).
   The amount of the compound of formula (Va) to be used may be from 0.5 mols to an excessive molar amount relative to 1 mol of the compound of formula (IVa), preferably from 0.5 mols to 2.0 mols.
   The base includes, for example, potassium carbonate, sodium carbonate, cesium carbonate, sodium hydrogencarbonate, sodium hydrogencarbonate, sodium hydride et al. Preferred is potassium carbonate.
   The amount of the base to be used may be from 0.5 mols to an excessive molar amount relative to 1 mol of the compound of formula (IVa), preferably from 1 mol to 1.5 mols.
   The organic solvent includes, for example, methylene chloride, chloroform, dichloroethane, carbon tetrachloride, n-heptane, n-hexane, benzene, toluene, xylene, diethyl ether, tetrahydrofuran, 1,4-dioxane, ethylene glycol, dimethyl ether, methyl acetate, ethyl acetate, N,N-dimethylformamide, N-methylpyrrolidone, dimethylsulfoxide et al.
   The reaction temperature may be from -20°C to 200°C, preferably from 0°C to 100°C; and in general, the reaction may be completed in 5 minutes to 72 hours, preferably in 5 minutes to 24 hours.
2) In case where J is OH:
   A compound of formula (IVa) and a compound of formula (Va) are condensed through Mitsunobu reaction to obtain a compound of formula (Ibp).
   Specifically, in a reaction solvent, a compound of formula (IVa) and a compound of formula (Va) are condensed in the presence of an azo compound such as dialkyl azodicarboxylate or 1,1'-(azodicarbonyl)diamide, and an organophosphorus compound such as triaryl phosphine or trialkyl phosphine to obtain a compound of formula (Ibp).
   The azo compound includes dimethyl azodicarboxylate, diethyl azodicarboxylate, diisopropyl azodicarboxylate, di-t-butyl azodicarboxylate, 1,1'-(azodicarbonyl)dipiperidide et al; the triaryl phosphine includes triphenyl phosphine, tritolyl phosphine et al; the trialkyl phosphine includes triethyl phosphine, tributyl phosphine, trioctyl phosphine et al. Above all, recommended is a combination of diisopropyl azodicarboxylate and triphenyl phosphine; or a combination of 1,1'-(azodicarbonyl)dipiperidide and tributyl phosphine.
   The amount of the compound of formula (Va) to be used may be from 1 mol to 10 mols relative to 1 mol of the compound of formula (IVa), preferably from 1 mol to 1.5 mols.
   The amount of the azo compound and the organophosphorus compound to be used may be as follows: The amount of the azo compound may be from 1 mol to 3 mols relative to 1 mol of the compound of formula (IVa), preferably from 1 mol to 1.5 mols; and the amount of the organophosphorus compound may be from 1 mol to 3 mols relative to 1 mol of the compound of formula (IVa), preferably from 1 mol to 1.5 mols.
   The reaction solvent includes halogenocarbons such as methylene chloride, chloroform, dichloroethane, carbon tetrachloride et al; aliphatic hydrocarbons such as n-heptane, n-hexane et al; aromatic hydrocarbons such as benzene, toluene, xylene et al; ethers such as diethyl ether, THF, dioxane, ethylene glycol dimethyl ether et al; esters such as methyl acetate, ethyl acetate et al; acetonitrile, NMP, DMF, DMSO et al; and their mixed solvents.
   The reaction temperature may be from 0°C to 100°C, preferably from 0°C to 50°C, and in general, the reaction takes 2 hours to 24 hours.
   In case where the compound of formula (Ibp) has a protective group, the protective group may be removed to obtain a compound of a formula (Ib).
   The removal of the protective group may be attained according to the methods described in the above-mentioned literature.
   Thus obtained, the compound of formula (Ib) may be isolated and purified according to a known separation and purification method of, for example, concentration, concentration under reduced pressure, crystallization, solvent extraction, reprecipitation, chromatography et al.
   The compound of formula (IV) may be prepared according to the following method: 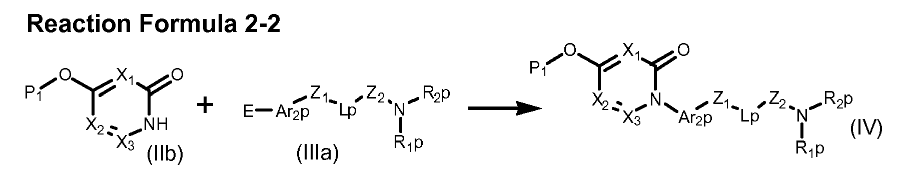 [In the formula, P₁, E, Ar₂p, R₁p, R₂p, R₁, R₂, Z₁, Lp, L, Z₂, Ar₂, X₁, X₂ and X₃ have the same meanings as above.]
   Specifically, a compound of a formula (IIb) and a compound of a formula (IIIa) are reacted according to the production method 1, thereby obtaining a compound of formula (IV).
   The compound of formula (IIb) may be a commercial reagent, or may be prepared according to the methods described in Examples.
Production Method 3:
   Production Method 3 is an alternative production method for compounds of formula (Ia), starting from a compound of a formula (IIa). 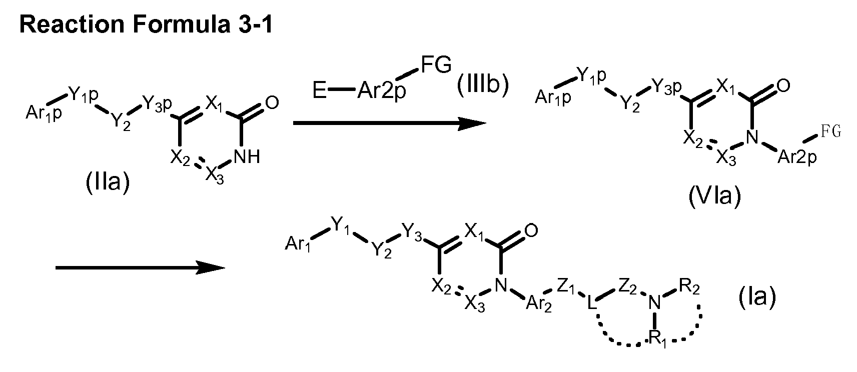 [In the formula, FG represents a side chain containing a functional group convertible into an amino group; Ar₁p, Ar₂p, Y₁p, Y₂, Y₃p, Ar₁, Ar₂, Y₁, Y₂, Y₃, Z₁, L, Z₂, R₁, R₂, X₁, X₂ and X₃ have the same meanings as above.]
   Specifically, a compound of a formula (IIa) and a compound of a formula (IIIb) are condensed to obtain a compound of a formula (VIa). Next, FG in the compound of formula (VIa) is converted into a substituent containing an amino group.
   The reaction of a compound of formula (IIa) and a compound of formula (IIIb) may be attained according to the production method 1. In this, examples of the compound of formula (IIIb) are mentioned below, and they may be prepared according to the methods described in Examples. 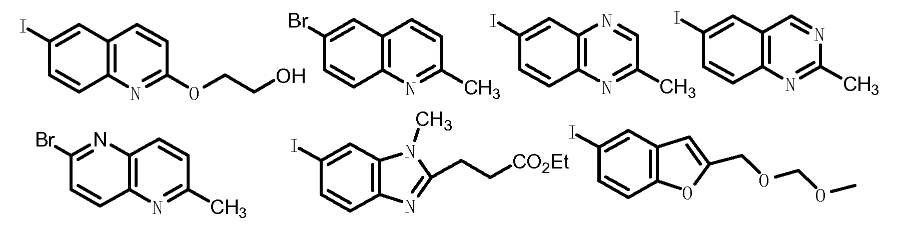
   The reaction from the compound of formula (VIa) to a compound of formula (Iap) includes, for example, the following: 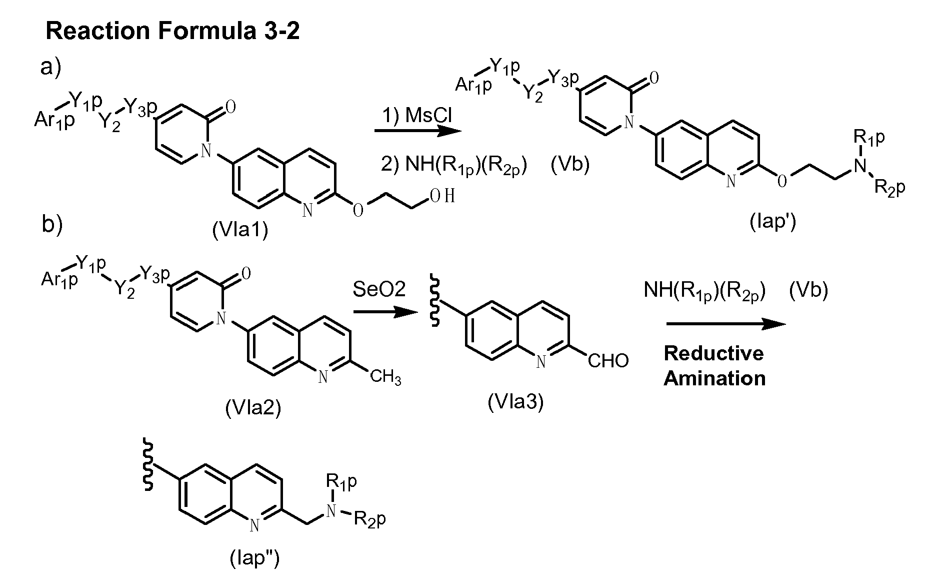 [In the formula, Ar₁p, Y₁p, Y₂, Y₃p, R₁p and R₂p have the same meanings as above.]
   reaction a):
   A compound of a formula (VIa1) is mesylated according to a method known in literature, and then the obtained mesyl compound is alkylaminated according to a method known in literature, thereby obtaining the intended compound of a formula (Iap').
   reaction b):
   A compound of a formula (VIa2) is oxidized with selenium dioxide to give a compound of a formula (VIa3). Next, the compound of formula (VIa3) is processed for reductive amination to give a compound of a formula (Iap").
   The oxidation may be attained in the presence of an organic solvent; and the organic solvent includes dioxane, xylene, ethyl acetate, benzene, nitrobenzene, 1,2-dichlorobenzene, 1,2-dimethoxyethane, acetic acid et al.
   The amount of selenium dioxide to be used may be from an equimolar amount to an excessive molar amount relative to 1 mol of the compound of formula (VIa2), preferably from 1.0 mol to 1.5 mols.
   The reaction temperature may be from 20°C to 300°C, preferably from 50°C to 200°C; and in general, the reaction will be completed in 5 minutes to 72 hours, preferably in 5 minutes to 24 hours.
   Next, the compound of formula (VIa3) is condensed with a compound of a formula (Vb) (reductive amination) in an organic solvent in the presence of a reducing agent, thereby obtaining a compound of a formula (Iap").
   The amount of the compound of formula (VIa3) and the compound of formula (Vb) to be used may be generally such that the two are in an equimolar amount or any one of them is in a small excessive molar amount.
   The reducing agent includes sodium cyanoborohydride, sodium triacetoxyborohydride, zinc biscyanoborohydride, nickel biscyanoborohydride et al.
   The amount of the reducing agent to be used may be from 1 mol to an excessive molar amount relative to 1 mol of the compound of formula (VIa3), preferably from 1 to 5 mols.
   The organic solvent includes alcohols such as methanol, ethanol, propanol et al; ethers such as diethyl ether, THF, dioxane et al; halogenohydrocarbons such as methylene chloride, chloroform, dichloroethane et al; aromatic hydrocarbons such as benzene, toluene, chlorobenzene, xylene et al; DMF, acetonitrile et al; and their mixed solvents.
   The reaction temperature may be generally from -20°C to 100°C, preferably from 0°C to room temperature; the reaction time may be generally from 5 minutes to 7 days, preferably from 1 hour to 6 hours.
   The compound of formula (Iap') or the compound of formula (Iap") may be optionally deprotected to give a compound of formula (Ia).
   Thus obtained, the compound of formula (Ia) may be isolated and purified according to a known separation and purification method of, for example, concentration, concentration under reduced pressure, crystallization, solvent extraction, reprecipitation, chromatography et al.
Production Method 4:
   Production Method 4 is for producing a compound of formula (VIa) wherein Y₃=O, or that is, a compound of a formula (VIb). 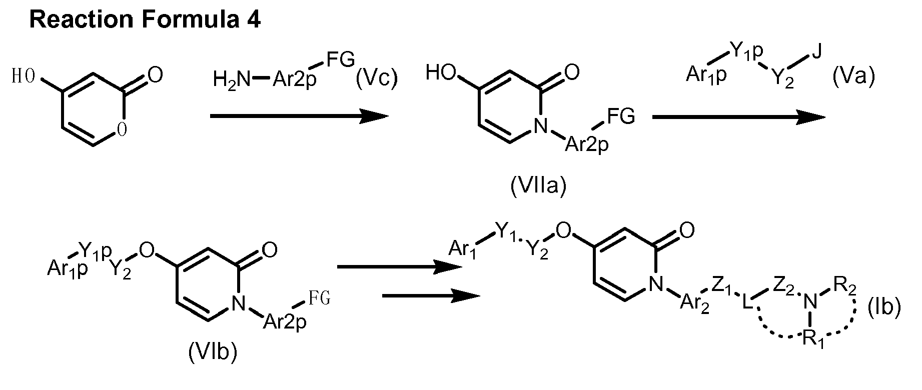 [In the formula, Ar₁p, Ar₂p, FG, J, Ar₁, Ar₂, Y₁p, Y₁, Y₂, Z₁, L, Z₂, R₁ and R₂ have the same meanings as above.]
   4-Hydroxy-2H-pyran-2-one described in JP-A 2-59566 is condensed with a compound of a formula (Vc) in an organic solvent to obtain a compound of a formula (VIIa).
   The amount of 4-hydroxy-2H-pyran-2-one to be used may be from 0.5 mols to an excessive molar amount relative to 1 mol of the compound of formula (Vc), preferably from 1 mol to 2 mols.
   The organic solvent includes 1-butanol, ethanol, THF, dioxane, acetic acid, acetic anhydride, pyridine et al.
   The reaction temperature may be from 0°C to 200°C, preferably from 0°C to 150°C, and in general, the reaction will be completed in 1 hour to 72 hours, preferably in 1 hour to 24 hours.
   Next, the compound of formula (VIIa) is condensed with a compound of a formula (Va) according to the production method 2 to obtain a compound of a formula (VIb). Then, the conversion of the compound of formula (VIb) to a compound of a formula (Ib) may be attained according to the production method 3.
   In the above reaction, the compound of formula (Va), the compound of formula (Vb), and the compound of formula (Vc) may be commercial reagents, or may be prepared according to the methods described in Examples.
   The compounds of formula (I) wherein W₁ to W₄ are all not single bonds may also be readily produced by combining the production methods 1 to 4 and methods known in literature.
   In the above reaction, when the reactants have an amino group, a hydroxyl group, a carboxyl group, an oxo group, a carbonyl group or the like not participating in the reaction, then the amino group, the hydroxyl group, the carboxyl group, the oxo group, the carbonyl group may be suitably protected with a protective group for amino group, a protective group for hydroxyl group, a protective group for carboxyl group, a protective group for oxo group or a protective group for carbonyl group, and after the reaction, the protective group may be removed.
   The method for introduction/removal of the protective group may differ, depending on the type of the protective group and the stability of the compound to be used, but may be attained according to the methods described in Protective Groups in Organic Synthesis.
   In this, the protective group may be applied to an amino group, an imino group, a hydroxyl group, a carboxyl group, an oxo group.
   Having its function, the protective group for amino group and imino group is not specifically limited, and includes, for example, an aralkyl group such as a benzyl group, a p-methoxybenzyl group, a 3,4-dimethoxybenzyl group, an o-nitrobenzyl group, a p-nitrobenzyl group, a benzhydryl group, a trityl group et al; a lower alkanoyl group such as a formyl group, an acetyl group, a propionyl group, a butyryl group, a pivaloyl group et al; an arylalkanoyl group such as a benzoyl group, a phenylacetyl group, a phenoxyacetyl group et al; a lower alkoxycarbonyl group such as a methoxycarbonyl group, an ethoxycarbonyl group, a propyloxycarbonyl group; a tert-butoxycarbonyl group et al; an aralkyloxycarbonyl group such as a benzyloxycarbonyl group, a p-nitrobenzyloxycarbonyl group, a phenethyloxycarbonyl group et al; a lower alkylsilyl group such as a trimethylsilyl group, a tert-butyldimethylsilyl group et al; a tetrahydropyranyl group; a trimethylsilylethoxymethyl group; a lower alkylsulfonyl group such as a methylsulfonyl group, an ethylsulfonyl group et al; an arylsulfonyl group such as a benzenesulfonyl group, a toluenesulfonyl group et al; and especially preferred are an acetyl group, a benzoyl group, a tert-butoxycarbonyl group, a trimethylsilylethoxymethyl group, a methylsulfonyl group et al.
   Having its function, the protective group for hydroxyl group is not specifically limited, and includes, for example, a lower alkyl group such as a methyl group, an ethyl group, a propyl group, an isopropyl group, a tert-butyl group et al; a lower alkylsilyl group such as a trimethylsilyl group, a tert-butyldimethylsilyl group et al; a lower alkoxymethyl group such as a methoxymethyl group, a 2-methoxyethoxymethyl group et al; a tetrahydropyranyl group; a trimethylsilylethoxymethyl group; an aralkyl group such as a benzyl group, a p-fluorobenzyl group, a p-methoxybenzyl group, a 2,3-dimethoxybenzyl group, an o-nitrobenzyl group, a p-nitrobenzyl group, a trityl group et al; an acyl group such as a formyl group, an acetyl group et al. Especially preferred are a methyl group, a methoxymethyl group, a tetrahydropyranyl group, a trityl group, a trimethylsilylethoxymethyl group, a tert-butyldimethylsilyl group, an acetyl group et al.
   Not specifically limited, the carboxyl-protective group may be any one having its function, and includes, for example, a lower alkyl group such as a methyl group, an ethyl group, a propyl group, an isopropyl group, a tert-butyl group et al; a lower haloalkyl group such as a 2,2,2-trichloroethyl group et al; a lower alkenyl group such as a 2-propenyl group et al; an aralkyl group such as a benzyl group, a p-methoxybenzyl group, a p-nitrobenzyl group, a benzhydryl group, a trityl group et al. Especially preferred are a methyl group, an ethyl group, a tert-butyl group, a 2-propenyl group, a benzyl group, a p-methoxybenzyl group, a benzhydryl group et al.
   Not specifically limited, the protective group for oxo group and carbonyl group may be any one having its function, and includes, for example, acetals and ketals such as ethylene ketal, trimethylene ketal, dimethyl ketal et al.
   Thus obtained, the compounds of formula (I) may be readily isolated and purified according to a known separation method of, for example, solvent extraction, recrystallization, column chromatography, preparative thin-layer chromatography et al.
   These compounds may be formed into pharmaceutically-acceptable salts or esters in an ordinary manner, and on the contrary, the salts or the esters may be converted into free compounds in an ordinary manner.
   The effect of the compounds of the invention as an MCH receptor antagonist may be verified, for example, according to the following pharmacological test example.

### Pharmacological Test Example: MCH binding inhibition test

A human MCH-1R encoding cDNA sequence [FEBS Letters, Vol. 398, 253 (1996); Biochimica et Biophisica Acta, Vol. 1401, 216 (1998)] was cloned to a plasmid vector pEF/myc/cyto (Invitrogen Corporation). The obtained expression vector was transfected to host cells CHO-K1 (American Type Culture Collection) using Lipofectamine Plus Reagent (Life Technology Inc.) to provide MCH-1R expression cells.

Membrane samples prepared from the MCH-1R expression cells were incubated with each test compound and 50 pM of [¹²⁵I]MCH (NEN Co.), in an assay buffer (50 mM Tris buffer comprising 10 mM magnesium chloride, 2 mM ethylenediamine tetraacetate, 0.01 % bacitracin and 0.2 % bovine serum albumin; pH 7.4) at 25°C for an hour, followed by filtration through a glass filter GF/C (Wattman Co.). After washing the glass filter with 50 mM Tris buffer (pH 7.4) comprising 10 mM magnesium chloride, 2 mM ethylenediamine tetraacetate and 0.04 % Tween-20, the radioactive activity on the glass filter was measured. The non-specific binding was measured in the presence of 1 µM human MCH and 50 % inhibition concentration (IC₅₀ value) of each test compound to the specific [¹²⁵I]MCH binding was determined. The results are shown in Table 7.

**Table 7**

| **Example** | IC50 (nM) | **Example** | IC50 (nM) |
|---|---|---|---|
| 8 | 6.4 | 63 | 22 |
| 20 | 7.8 | 65 | 5.1 |
| 23 | 6.2 | 70 | 11 |
| 27 | 80 | 76 | 2.9 |
| 28 | 7.6 | 89 | 19 |
| 30 | 5.9 | 90 | 9.8 |
| 35 | 2.2 | 95 | 23 |
| 38 | 3.1 | 99 | 6 |
| 40 | 5.2 | 98 | 9.6 |
| 57 | 10.1 | 94 | 10.5 |

As in the above, the compounds of the invention strongly inhibited the binding of MCH to MCH-1R, therefore exhibiting an excellent effect as an MCH-1R antagonist.

Accordingly, the compounds of the invention are useful as a preventive, treating or remedial agent for various MCH-related disorders, for example, metabolic disorders such as obesity, diabetes, hormone disorder, hyperlipidemia, gout, fatty liver; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation. Pharmaceutical Composition Comprising Compound of Formula (I)

The compound of the invention can be orally or parenterally administered, and can be formulated into preparations suitable to the administration thereof as pharmaceutical compositions for prevention, treatment or remedy for the above-mentioned disorders.

In its clinical use, the compound of the invention may be formulated into various preparations along with a pharmaceutically-acceptable carrier added thereto generally in accordance with the administration route thereof, and the thus-formulated pharmaceutical composition may be administered. As the additives, usable are various conventional additives known in the field of pharmaceutical preparations. For example, concretely, they include gelatin, lactose, white sugar, titanium oxide, starch, crystalline cellulose, hydroxypropylmethyl cellulose, carboxymethyl cellulose, corn starch, microcrystalline wax, white petrolatum, magnesium aluminate metasilicate, anhydrous calcium phosphate, citric acid, trisodium citrate, hydroxypropyl cellulose, sorbitol, sorbitan fatty acid esters, polysorbate, sucrose fatty acid esters, polyoxyethylene, hardened castor oil, polyvinylpyrrolidone, magnesium stearate, light silicic anhydride, talc, vegetable oils, benzyl alcohol, gum arabic, propylene glycol, polyalkylene glycol, cyclodextrin and hydroxypropylcyclodextrin et al.

Preparations to be formed by the use of those additives include, for example, solid preparations such as tablets, capsules, granules, powders and suppositories et al; and liquid preparations such as syrups, elixirs and injections et al. These may be formulated according to conventional methods known in the field of pharmaceutical preparations. The liquid preparations may also be in such a form that may be dissolved or suspended in water or in any other suitable medium in their use. Especially for injections, if desired, the preparations may be dissolved or suspended in physiological saline water or glucose liquid, and a buffer or a preservative may be optionally added thereto.

The pharmaceutical compositions may contain a compound of the invention in an amount of from 1 to 100 % by weight, preferably from 1 to 60 % by weight of the composition. They may further contain any other therapeutically-effective compounds.

In case where the compounds of the invention are used for prevention, treatment or remedy of the above-mentioned diseases or disorders, then the dose and the dosing frequency may be varied, depending on the sex, the age, the body weight and the disease condition of the patient and on the type and the range of the intended remedial effect. In general, in oral administration the dose may be from 0.001 to 50 mg/kg of body weight/day, and it may be administered at a time or in a few times. The dose is preferably from about 0.01 to about 25 mg/kg/day, more preferably from about 0.05 to about 10 mg/kg/day.

The compounds of the invention can be used in combination with drugs effective for hypertension, obesity-associated hypertension, hypertension-associated diseases, hypertrophy, left ventricular hypertrophy, metabolic disorders, obesity, obesity-associated diseases and the like (hereafter referred to as "co-drugs"). Such drugs can be administered simultaneously, separately or in succession, for prevention, treatment or therapy for the above-mentioned diseases. When a compound of the invention is used simultaneously with one, two or more of co-drugs, they may be formulated into a medical preparation suited for single administration form. Whereas, in combination therapy, a composition containing a compound of the invention and a co-drug may be administered to the object of medication in different packages, either simultaneously, separately or successively. They may be administered at time intervals.

The dose of the co-drug may be determined in accordance with the clinically adopted dose thereof, which can be suitably selected according to the individual object of medication, the administration route, the specific disease, the combination of drugs, and the like. The form of the co-drug for administration is not specifically limited, and it may be combined with a compound of the invention when they are administered.

The administration mode includes, for example, the following: (1) A compound of the invention is combined with a co-drug to give a single preparation for single administration; (2) a compound of the invention and a co-drug are separately formulated into different two preparations, and the two preparations are simultaneously administered in one administration route; (3) a compound of the invention and a co-drug are separately formulated into different two preparations, and they are administered at different times in one and the same administration route; (4) a compound of the invention and a co-drug are separately formulated into different two preparations, and they are administered at the same time in two different administration routes; (5) a compound of the invention and a co-drug are separately formulated into different two preparations, and they are administered at different times in different administration routes (for example, a compound of the invention and a co-drug are administered in that order, or in an order contrary to this). The blend ratio of the compound of the invention and the co-drug may be suitably determined depending on the administration object, the administration route, and the disease for the administration.

The co-drugs usable in the invention include, for example, drugs for diabetes, drugs for hyperlipidemia, drugs for hypertension, anti-obesity drugs. Two or more such co-drugs may be combined in an adequate ratio and used.

The drugs for diabetes include, for example, 1) PPAR-γ agonists such as glitazones (e.g., ciglitazone, darglitazone, englitazone, isaglitazone, MCC-555 et al), pioglitazone, rosiglitazone, troglitazone, BRL49653, CLX-0921, 5-BTZD, GW-0207, LG-100641, LY-300512 et al; 2) biguanides such as metformin, buformin, phenformin et al; 3) protein tyrosine phosphatase 1B inhibitors et al; 4) sulfonylureas such as acetohexamide, chloropropamide, diabinese, glibenclamide, glipizide, glyburide, glimepiride, glicilazide, glipentide, gliquidone, glisolamide, trazamide, tolubutamide et al; 5) meglitinides such as repaglinide, nateglinide et al; 6) α-glucoside hydrolase inhibitors such as acarbose, adiposine, camiglibose, emiglitate, miglitol, voglibose, pradimicin-Q, salbostatin, CKD-711, MDL-25, 673, MDL-73, 945, MOR14 et al; 7) α-amylase inhibitors such as tendamistat, trestatin, A13688 et al; 8) insulin secretion promoters such as linogliride, A-4166 et al; 9) fatty acid oxidation inhibitors such as clomoxir, etomoxir et al; 10) A2 antagonists such as midaglizole, isaglidole, deriglidole, idazoxan, earoxan, fluparoxan et al; 11) insulin or insulin mimetics such as biota, LP-100, novalapid, insulin determir, insulin lispro, insulin glargine, insulin zinc, Lys-Pro-insulin, GLP-1 (73-7), GLP1 amide (7-36) et al; 12) non-thiazolidinediones such as JT-501, farglitazar et al; 13) PPARα/γ dual-agonists such as MK-0767, CLX-0940, GW-1536, GW-1929, GW-2433, KRP-297, L-796449, LR-90 and SB219994 et al.

The above drugs for hyperlipidemia include, for example, 1) bile acid absorption promoters such as cholesterylamine, colesevelem, colestipol, crosslinked dextran dialkylaminoalkyl derivatives, Colestid™, LoCholest™, Questran™ et al; 2) HMG-CoA reductase inhibitors such as atorvastatin, itavastatin, fluvastatin, lovastatin, pravastatin, rivastatin, rosuvastatin, simvastatin, ZD-4522 et al; 3) HMG-CoA synthase inhibitors; 4) cholesterol absorption inhibitors such as snatol ester, β-sitosterol, sterol glucoside, ezetimibe et al; 5) acylcoenzyme A·cholesterol acyl transferase inhibitors such as avasimibe, eflucimibe, KY-505, SMP-709 et al; 6) CETP inhibitors such as JTT705, torcetrapib, CP532632, BAY-63-2149, SC-591, SC-795 et al; 7) squalane synthetase inhibitors; 8) antioxidants such as probucol; 9) PPAR-α agonists such as beclofibrate, benzafibrate, syprofibrate, clofibrate, etofibrate, fenofibrate, gemcabene, gemfibrozil, GW-7647, BM-170744, LY-518674, fibric acid derivatives (e.g., Atromid™, Lopid™, Tricor™) et al; 10) FXR receptor antagonists such as GW-4064, SR-103912 et al; 11) LXR receptor agonists such as GW3965, T9013137, XTCO-179628 et al; 12) lipoprotein synthesis inhibitors such as niacin et al; 13) renin-angiotensin system inhibitors; 14) microsome-triglyceride transport inhibitors; 15) bile acid resorption inhibitors such as BARA1453, SC435, PHA384640, S-435, AZD7706 et al; 16) PPAR-δ agonists such as GW501516, GW590735 et al; 17) triglyceride synthesis inhibitors; 18) MTTP inhibitors such as LAB687, CP346086 et al; 19) low-density lipoprotein receptor inducers; 20) squalane epoxidase inhibitors; 21) thrombocyte agglutination inhibitors; 22) 5-lipoxygenase activated protein inhibitors such as MK-591.

The above drugs for hypertension include, for example,
1) thiazide diuretics such as chlorothialidon, chlorothiazide, dichlorofenamide, hydrofluorothiazide, indapamide, hydrochlorothiazide et al; loop diuretics such as bumetanide, ethacrynic acid, flosemide, tolusemide et al; sodium diuretics such as amyloride, triamuteren et al; aldosterone antagonist diuretics such as spironolactone, epilenone et al; 2) β-adrenaline blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, probanolol, sotalol, tartatolol, tilisolol, timolol et al; 3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, verapamil et al; 4) angiotensin converting enzyme inhibitors such as benazepril, captopril, cilazapril, delapril, enalapril, fosinopril, imidapril, rosinopril, moexipril, quinapril, quinaprilat, ramipril, perindopril, perindoropril, quanipril, spirapril, tenocapril, trandolapril, zofenopril et al; 5) neutral endopeptidase inhibitors such as omapatrilat, cadoxatril, ecadotril, fosidotril, sampatrilat, AVE7688, ER4030 et al; 6) endothelin antagonists such as tezosentan, A308165, YM62899 et al; 7) vasodilators such as hydraladine, clonidine, minoxidil, nicotinyl alcohol et al; 8) angiotensin II antagonists such as candesartan, eporsartan, iribesartan, losartan, pratosartan, tasosartan, telmisartan, valsartan, EXP-3137, FI6828K, RNH6270 et al; 9) α/β adrenaline blockers such as nipradilol, arotinolol, amoslalol et a 1; 10) α 1 blockers such as terazosin, urapidil, purazosin, bunazosin, trimazosin, doxazosin, naphthopidil, indolamin, WHIP 164, XEN010 et al; 11) α 2 agonists such as lofexidine, tiamenidine, moxonidine, rilmenidine, guanobenz et al; 12) aldosterone inhibitors.

The above anti-obesity drugs include, for example,
1) 5HT (serotonin) transporter inhibitors such as paroxetine, fluoxetine, fenfluramine, fluvoxamine, sertraline, imipramine et al;
2) norepinephrine transporter inhibitors such as GW320659, desipramine, talsupram, nomifensin et al;
3) cannabinoid-1 receptor 1 (CB-1) antagonists/inverse-agonists such as limonabant (Sanofi Synthelabo), SR-147778 (Sanofi Synthelabo), BAY-65-2520 (Bayer), SLV-319 (Sorbei), as well as compounds disclosed in USP 5,532,237, USP 4,973,587, USP 5,013,837, USP 5,081,122, USP 5,112,820, USP 5,292,736, USP 5,624,941, USP 6,028,084, WO96/33159, WO98/33765, WO98/43636, WO98/43635, WO01/09120, WO01/96330, WO98/31227, WO98/41519, WO98/37061, WO00/10967, WO00/10968, WO97/29079, WO99/02499, WO01/58869, WO02/076949, WO01/64632, WO01/64633, WO01/64634, WO03/006007, WO03/007887, and EP-658546 et al;
4) ghrelin antagonists such as compounds disclosed in WO01/87355, WO02/08250 et al;
5) histamine(H3) antagonists/inverse-agonists such as thioperamide, 3-(1H-imidazol-4-yl)propyl N-(pentenyl)carbonate, clobenpropit, iodofenpropit, imoproxyfen, GT2395, A331440, compounds disclosed in WO02/15905, O-[3-(1H-imidazol-4-yl)propanol] carbamate, piperazine-containing H3-receptor antagonists (Lazewska, D. et al., Pharmazie, 56: 927-32 (2001)), benzophenone derivatives (Sasse, A. et al., Arch. Pharm. (Weinheim) 334: 45-52 (2001)), substituted N-phenylcarbamates (Reidemeister, S. et al., Pharmazie, 55: 83-6 (2000)), proxyfen derivatives (Sasse, A. et al., J. Med. Chem., 43: 3335-43 (2000)) et al;
6) MCH-1R antagonists such as T-226296 (Takeda), SNP-7941 (Synaptic), other compounds disclosed in WO01/82925, WO01/87834, WO02/051809, WO02/06245, WO02/076929, WO02/076947, WO02/04433, WO02/083134, WO02/094799, WO03/004027, and JP-A 2001-226269 et al;
7) MCH-2R agonists/antagonists;
8) NPY1 antagonists such as isopropyl 3-chloro-5-(1-(6-[2-(5-ethyl-4-methyl-thiazol-2-yl)-ethyl]-4-morpholinyl-4-yl-piperidin-2-ylamino)-ethyl)phenyl]carbamate, BIBP3226, BIBO3304, LY-357897, CP-671906, GI-264879, and other compounds disclosed in USP 6,001,836, WO96/14307, WO01/23387, WO99/51600, WO01/85690, WO01/85098, WO01/85173, and WO01/89528 et al;
9) NPY5 antagonists such as 152804, GW-569180A, GW-594884A, GW-587081X, GW-548118X, FR235,208, FR226928, FR240662, FR252384, 1229U91, GI-264879A, CGP71683A, LY-377897, LY366377, PD-160170, SR-120562A, SR-120819A, JCF-104, H409/22, and other compounds disclosed in USP 6,140,354, USP 6,191,160, USP 6,258,837, USP 6,313,298, USP 6,337,332, USP 6,329,395, USP 340,683, USP 6,326,375, USP 6,329,395, USP 6,337,332, USP 6,335,345, EP-01010691, EP-01044970, WO97/19682, WO97/20820, WO97/20821, WO97/20822, WO97/20823, WO98/27063, WO00/107409, WO00/185714, WO00/185730, WO00/64880, WO00/68197, WO00/69849, WO01/09120, WO01/14376, WO01/85714, WO1/85730, WO01/07409, WO01/02379, WO01/23388, WO01/23389, WO01/44201, WO01/62737, WO01/62738, WO02/20488, WO02/22592, WO02/48152, WO02/49648, WO02/094789, and compounds disclosed in Norman et al., J. Med. Chem., 43:4288-4312(2000) et al;
10) leptins such as human recombinant leptin (PEG-OB, Hoffman La Roche), recombinant methionylreptin (Amgen) et al;
11) leptin derivatives such as compounds disclosed in USP 5,552,524, USP 5,552,523, USP 5,552,522, USP 5,521,283, WO96/23513, WO96/23514, WO96/23515, WO96/23516, WO96/23517, 96/23518, WO96/23519, and WO96/23520 et al;
12) opioid antagonists such as nalmefen (Revex™), 3-methoxynaltorexone, naloxone, naltorexone, compounds disclosed in WO00/21509 et al;
13) Orexin antagonists such as SB-334867A, and other compounds disclosed in WO01/96302, WO01/68609, WO02/51232, V02/51838, and WO03/023561 et al;
14) bonbesin receptor subtype-3 agonists;
15) cholecystokinin A (CCK-A) agonists such as AR-R15849, GI-181771, JMV-180, A-71378, A-71623, SR-146131, and other compounds disclosed in USP 5,739,106 et al;
16) CNTF (ciliary neurotrophic factors) such as GI-181771 (Glaxo-Smith Kline), SR146131 (Sanofi Synthelabo), butabindide, PD 170,292, PD 149164 (Pfizer) et al;
17) CNTF derivatives such as axokine (Regeneron), and other compounds disclosed in WO94/09134, WO98/22128, WO99/43813 et al;
18) growth hormone secretion receptor agonists such as NN703, hexarelin, MK-0677, SM-130686, CP-424,391, L-692,429, L-163,255, and compounds disclosed in USP 6,358,951, US Patent Application Nos. 2002/049196, 2002/022637, WO01/56592, WO02/32888 et al;
19) serotonin receptor-2C agonists such as BVT933, DPCA37215, IK264, PNU22394, WAY161503, R-1065, YM348, and other compounds disclosed in USP 3,914,250, WO02/36596, WO02/48124, WO02/10169, WO01/66548, WO02/44152, WO02/51844, WO02/40456, and WO02/40457 et al;
20) melanocortin-3 receptor agonists;
21) melanocortin-4 receptor agonists such as CHIR86036 (Chiron), ME-10142, ME-10145 (Melacure), and other compounds disclosed in WO99/64002, WO00/74679, WO01/991752, WO01/74844, WO01/70708, WO01/70337, WO01/91752, WO02/059095, WO02/059107, WO02/059108, WO02/059117, WO02/12166, WO02/11715, WO02/12178, WO02/15909, WO02/068387, WO02/068388, WO02/067869, WO03/007949, and WO03/009847 et al;
22) monoamine resorption inhibitors such as cibtramin (Meridia™/Reductil™) and its salts, and other derivatives disclosed in USP 4,746,680, USP 4,806,570, USP 5,436,272, US Patent Application No. 2002/0006964, WO01/27068, and WO01/62341 et al;
23) serotonin re-uptake inhibitors such as dexfenfluramine, fluoxetine, and other compounds disclosed in USP 6,365,633, WO01/27060, and WO01/162341 et al;
24) glucagon-like peptide-1 agonists;
25) topiramate (Topimax™);
26) phytopharm compound 57 (e.g., CP644,673);
27) acetyl CoA carboxylase-2 (ACC2) inhibitors;
28) β-adrenalin receptor-3 agonists such as AD9677/TAK677 (Dai-Nippon Pharmaceutical/Takeda Chemical), CL-316,243, SB418790, BRL-37344, L-796568, BMS-196085, BRL-35135A, CGP12177A, BTA-243, W427353, trecadrine, Zeneca D7114, SR59119A, and other compounds disclosed in USP 5,705,515, USP 5,451,677, WO01/74782, and WO02/32897 et al;
29) diacylglycerol acyltransferase-1 inhibitors;
30) diacylglycerol acyltransferase-2 inhibitors,
31) fatty acid synthetase inhibitors such as carulenin, C75;
32) phosphodiesterase inhibitors such as theophylline, pentoxiphylline, zaprinast, sildenafil, amrinone, milrinone, cilostamide, rolipram, and cilomilast et al;
33) thyroid hormone-β agonists such as KB-2611 (KaroBio BMS), and other compounds disclosed in WO02/15845, JP-A 2000-256190 et al; 34) UCP (uncoupling protein)-1, 2, or 3 activators such as phytanic acid, 4-[(E)-2-(5,6,7,8-tetrahydro-5,5,8,8-tetramethyl-2-naphthalenyl-1-propenyl]benzoic acid (TTNPB), retinoic acid, and other compounds disclosed in WO99/00123 et al;
35) acylestrogens such as oleoylestrone, and other compounds disclosed in del Mar-Grasa, M. et al., Obesity Research, 9:202-9 (2001);
36) glucocorticoid antagonists;
37) 11-β-hydroxysteroid dehydrogenase-1 inhibitors such as BVT3498, BVT2733, and other compounds disclosed in WO01/90091, WO01/90090, WO01/90092 et al;
38) stearoyl-CoA desaturase-1 inhibitors;
39) dipeptidyl peptidase-IV inhibitors such as isoleucine thiazolidide, valine pyrrolidide, NVP-DPP728, AF237, P93/01, TSL225, TMC-2A/2B/2C, FE999011, P9310/K364, VIP0177, SDZ274-444, and other compounds disclosed in WO03/004498, WO03/004496, EP1258476, WO02/083128, WO02/062764, WO03/000250, WO03/002530, WO03/002531, WO03/002553, WO03/002593, WO03/000180, and WO03/0001 et al;
40) lipase inhibitors such as tetrahydroliptatin (Orlistat/Xenical™), Triton WR1339, RHC80267, lipstatin, teasaponin, diethylumbelliferyl phosphate, FL-386, WAY-121898, Bay-N-3176, valilactone, esteracin, ebelactone A, ebelactone B, RHC80267, and other compounds disclosed in WO01/77094, USP 4,598,089, USP 4,452,813, USP 5,512,565, USP 5,391,571, USP 5,602,151, USP 4,405,644, USP 4,189,438, and USP 4,242,453 et al;
41) fatty acid transporter inhibitors;
42) dicarboxylate transporter inhibitors;
43) glucose transporter inhibitors;
44) phosphate transporter inhibitors.

Those combination drugs are obtained by concurrent use of a compound of the invention with one, two or more of the above co-drugs. Furthermore, the combination drugs are useful for prevention treatment or therapy of metabolic disorders, when combined with one, two or more drugs selected from the group consisting of diabetes-treating agents and hyperlipidemia-treating agents. Combinations containing, in particular, hypertension-treating agent and anti-obesity agent are useful for prevention, treatment or therapy for metabolic disorders with synergistic effect, when diabetes-treating agent and/or hyperlipidemia-treating agent are added thereto.

### BEST MODE FOR CARRYING OUT THE INVENTION

The invention is described more concretely with reference to the following Examples, to which, however, the invention should not be limited. As silica gel for columns, used were Wakogel™ C-200 (Wako Pure Chemical Industries), Wakogel™ C-300 (Wako Pure Chemical Industries, Chromatorex NH (Fuji Silicia Chemical); as a filled silica gel column, used was a FLASH+™ cartridge, KP-Sil or KP-NH, FLASH12+M, FLASH25+S, FLASH25+M or FLASH40+M (Biotage Japan); as a preparative thin-layer chromatography, used was Kieselgel 60F254 (by Merck). For mass spectrometry, used was QuattroII (Micromass).

### EXAMPLES

### Production Example 1-1:

### 6-Iodo-3,4-dihydroquinolin-2(1H)-one:

An acetic acid solution (355 mL) of 3,4-dihydroquinolin-2(1H)-one (41.1 g) and iodine monochloride (50 g) was stirred overnight at 80°C. After the reaction, the solvent was evaporated off under reduced pressure to obtain a crude product of the entitled compound (47 g).

### Production Example 1-2:

### 2-Chloro-6-iodoquinoline:

2,3-Dichloro-5,6-dicyano-1,4-benzoquinone (46.3 g) and phosphorus oxychloride (79 mL) were added to a toluene solution (1.8 L) of the crude product (47 g) obtained in Production Example 1-1, and stirred overnight at 90°C. The reaction solution was dropwise added to an aqueous 5 N sodium hydroxide solution (2.5 L) cooled with ice. After the addition, the reaction liquid was filtered, the filtrate was partitioned to obtain an organic layer. The aqueous layer was extracted with diethyl ether, the diethyl ether layer was combined with the previous organic layer, and dried with anhydrous magnesium sulfate. The solvent was evaporated off under reduced pressure, and the obtained residue was purified by silica gel column chromatography (hexane:ethyl acetate = 9:1 to 3:2) to obtain the entitled compound (18.7 g).

### Production Example 1-3:

### 6-Iodo-2-pyrrolidin-1-ylquinoline:

Pyrrolidine (490 mg) and potassium carbonate (286 mg) were added to a DMF solution (5 mL) of the compound (200 mg) obtained in Production Example 1-2, and stirred at 80°C for 10 hours. Water was added to the reaction solution, and extracted with a mixed solvent of chloroform/methanol (10:1). The organic layer was dried with anhydrous magnesium sulfate, then the solvent was evaporated off under reduced pressure. The obtained residue was purified by silica gel column chromatography (hexane:ethyl acetate =3:1 to 1:1) to obtain the entitled compound (220 mg) as a pale yellow solid.

### Production Example 1-4:

### 4-(Benzyloxy)-1-(2-pyrrolidin-1-ylquinolin-6-yl)pyridin-2(1H)-one:

4-Benzyloxy-2(1H)-pyridone (136.6 mg), copper(I) iodide (64.7 mg) and potassium carbonate (141 mg) were added to a DMF solution (5 mL) of the compound (110 mg) obtained in Production Example 1-3, and stirred overnight at 150°C. Water was added to the reaction solution, then extracted with a mixed solvent of chloroform/methanol (10:1). The organic layer was dried with anhydrous magnesium sulfate, then the solvent was evaporated off under reduced pressure. The obtained residue was purified by silica gel column chromatography (NH silica gel, hexane:ethyl acetate = 2:3) to obtain the entitled compound (20 mg).

### Production Example 1-5:

### 4-Hydroxy-1-(2-pyrrolidin-1-ylquinolin-6-yl)pyridin-2(1 H)-one:

10 % palladium-carbon (19 mg) and cyclohexene (0.5 mL) were added to a methanol solution (5 mL) of the compound (20 mg) obtained in Production Example 1-4, and heated under reflux for 1 hour. The reaction liquid was filtered, then the filtrate was concentrated under reduced pressure to obtain the entitled compound (20 mg) as a colorless oily substance.

### Production Example 2-1:

### 4-Hydroxy-1-{2-[isopropyl(methyl)amino]quinolin-6-yl}pyridin-2(1H)-one:

A 1-butanol solution (5 mL) of N²-isopropyl-N²-methylquinoline-2,6-diamine (100 mg) produced according to WO2004/103992 and 4-hydroxy-2H-pyran-2-one (69 mg) produced according to JP-A 02-59566 was stirred overnight at 110°C. The solvent was evaporated off from the reaction liquid under reduced pressure, and the obtained residue was purified by silica gel column chromatography (chloroform:methanol = 50:1) to obtain the entitled compound (23 mg) as a brown powder.

### Production Example 3-1:

### 4-Hydroxy-1-(2-methylquinolin-6-yl)pyridin-2(1H)-one:

According to the same process as in Production Example 2-1 but using commercial 2-methylquinoline-6-amine (1 g) and 4-hydroxy-2H-pyran-2-one (1 g), the entitled compound (370 mg) was obtained as a red brown powder.

### Production Example 3-2:

### 4-[(4-Chlorobenzyl)oxy]-1-(2-methylquinolin-6-yl)pyridin-2(1H)-one:

According to the same process as in Example 1 but using the compound (370 mg) obtained in Production Example 3-1 and 1-chloro-4-(chloromethyl)benzene (260 mg), the entitled compound (254 mg) was obtained as an orange powder.

### Production Example 3-3:

### 4-[(4-Chlorobenzyl)oxy]-1-[2-(hydroxymethyl)quinolin-6-yl]pyridin-2(1H)-one:

1) M-chloroperbenzoic acid (210 mg) was added to a chloroform solution (10 mL) of the compound (254 mg) obtained in Production Example 3-2, and stirred at room temperature for 8 hours. Aqueous saturated sodium hydrogencarbonate solution was added to the reaction liquid, and extracted with chloroform. The organic layer was dried with anhydrous magnesium sulfate, then the solvent was evaporated off under reduced pressure. The obtained residue was purified by silica gel column chromatography (chloroform:methanol = 40:1) to obtain 4-[(4-chlorobenzyl)oxy]-1-(2-methyl-1-oxidoquinolin-6-yl)pyridin-2(1H)one (260 mg) as an orange powder.
2) An acetic anhydride solution (50 mL) of the compound (260 mg) obtained in Production Example 3-3-1) was stirred at 85°C for 7 hours. The reaction liquid was concentrated under reduced pressure to obtain {6-[4-[(4-chlorobenzyl)oxy]-2-oxopyridin-1(2H)-yl]quinolin-2-yl}methyl acetate (290 mg) as a brown powder.
3) Aqueous 5 N sodium hydroxide solution (0.5 mL) was added to a methanol/THF mixed solution (10:1, 5 mL) of the compound (290 mg) obtained in Production Example 3-3-2), and stirred at room temperature for 7 hours. Water was added to the reaction liquid, and extracted with a mixed solvent of chloroform/methanol (10:1). The organic layer was dried with anhydrous magnesium sulfate, then the solvent was evaporated off under reduced pressure. The obtained residue was purified by silica gel column chromatography (chloroform:methanol = 20:1) to obtain the entitled compound (212 mg) as a yellow white powder.

### Production Example 4-1:

### 6-Iodo-2-(2-pyrrolidin-1-ylethoxy)quinoline:

1-(2-Hydroxyethyl)pyrrolidine (14.9 g) and sodium hydride (7.8 g) were added to a DMF solution (280 mL) of the compound (18.7 g) obtained in Production Example 1-2, and stirred overnight at 85°C. Water was added to the reaction liquid, then extracted with a mixed solvent of chloroform/methanol (10:1), and the organic layer was dried with anhydrous magnesium sulfate. The solvent was evaporated off under reduced pressure, and the obtained residue was purified by silica gel column chromatography (chloroform:methanol = 40:1) to obtain the entitled compound (31.6 g) as a yellow oily substance.

### Production Example 5-1:

### 4-Hydroxy-1-[2-(2-pyrrolidin-1-ylethoxy)quinolin-6-yl]pyridin-2(1H)-one:

10 % palladium-carbon (140 mg) was added to a methanol solution (30 mL) of the compound (170 mg) obtained in Example 7, and stirred in a hydrogen atmosphere (1 atmospheric pressure) at 50°C for 8 hours. The reaction liquid was filtered, the filtrate was concentrated under reduced pressure to obtain the entitled compound (100 mg) as a yellow oily substance.

### Production Example 6-1:

### {2-[(6-Iodoquinolin-2-yl)oxy]ethyl}dimethylamine:

According to the same process as in Production Example 4-1 but using the compound (1.1 g) obtained in Production Example 1-2 and N,N-dimethylethanolamine (3.5 mL), the entitled compound (1.24 g) was obtained as a colorless oily substance.

### Production Example 6-2:

### 1- {2-[2-(Dimethylamino)ethoxy]quinolin-6-yl}-4-[(4-fluorobenzyl)oxy]pyridin-2(1H)-one:

According to the same process as in Production Example 1-4 but using the compound (1.24 g) obtained in Production Example 6-1 and 4-(fluorobenzyl)oxy-2(1H)-pyridone (1.19 g), the entitled compound (633 mg) was obtained as an orange powder.

### Production Example 6-3:

### 1- {2-[2-(dimethylamino)ethoxy]quinolin-6-yl}-4-hydroxypyridin-2(1H)-one:

According to the same process as in Production Example 1-5 but using the compound (480 mg) obtained in Production Example 6-2, the entitled compound (315 mg) was obtained as a yellow powder.

### Production Example 7-1:

### N,N-dimethyl-2-[(6-nitroquinolin-2-yl)oxy]ethanamine:

N,N-dimethylethanolamine (1.5 g) and potassium carbonate (995 mg) were added to a DMF solution (20 mL) of 2-chloro-6-nitroquinoline (1.2 g) produced according to WO2004/103992, and stirred overnight at 100°C. Water was added to the reaction liquid, extracted with ethyl acetate, and the organic layer was dried with anhydrous magnesium sulfate. The solvent was evaporated off under reduced pressure, and the obtained residue was purified by silica gel column chromatography (chloroform:methanol = 50:3) to obtain the entitled compound (370 mg).

### Production Example 7-2:

### 2-[2-(Dimethylamino)ethoxy]quinoline-6-amine:

10 % palladium-carbon (50 mg) was added to a methanol solution (5 mL) of the compound (78 mg) obtained in Production Example 7-1, and in a hydrogen atmosphere (1 atmospheric pressure), stirred at room temperature for 2 hours. The reaction liquid was filtered, and the solvent was evaporated off under reduced pressure from the filtrate to obtain the entitled compound (70 mg).

### Production Example 7-3:

### 1- {2-[2-(dimethylamino)ethoxy]quinolin-6-yl}-4-hydroxypyridin-2(1H)-one:

According to the same process as in Production Example 2-1 but using the compound (70 mg) obtained in Production Example 7-2 and 4-hydroxy-2H-pyran-2-one (50 mg), the entitled compound (37 mg) was obtained as a brown oily substance.

### Production Example 8-1:

### 2-[(6-Iodoquinolin-2-yl)oxy]ethanol:

According to the same process as in Production Example 4-1 but using the compound (8 g) obtained in Production Example 1-2 and ethylene glycol (40 mL), the entitled compound (2 g) was obtained as a white powder.

### Production Example 8-2:

### 4-(Benzyloxy)-1-[2-(2-hydroxyethoxy)quinolin-6-yl]pyridin-2(1H)-one:

According to the same process as in Production Example 1-4 but using the compound (1 g) obtained in Production Example 8-1 and 4-benzyloxy-2(1H)-pyridone (1.28 g), the entitled compound (667 mg) was obtained as a pale yellow powder.

### Production Example 9-1:

### N,N-diethyl-2-[(6-iodoquinolin-2-yl)oxy]ethanamine:

According to the same process as in Example 3 but using the compound (440 mg) obtained in Production Example 8-1 and diethylamine (20 mL) and purifying the product by silica gel column chromatography (chloroform:methanol = 40:1), the entitled compound (330 mg) was obtained.

### Production Example 9-2:

### 4-(Benzyloxy)-1-{2-[2-(diethylamino)ethoxy]quinolin-6-yl}pyridin-2(1H)-one:

According to the same process as in Production Example 1-4 but using the compound (295 mg) obtained in Production Example 9-1 and 4-benzyloxy-2(1H)-pyridone (320 mg), the entitled compound (53 mg) was obtained as a yellow oily substance.

### Production Example 9-3:

### 1- {2-[2-(Diethylamino)ethoxy]quinolin-6-yl}-4-hydroxypyridin-2(1H)-one:

According to the same process as in Production Example 1-5 but using the compound (53 mg) obtained in Production Example 9-2, the entitled compound (37.9 mg) was obtained as a yellow powder.

### Production Example 10-1:

### 3-[(6-Iodoquinolin-2-yl)oxy]propan-1-ol:

According to the same process as in Production Example 4-1 but using the compound (500 mg) obtained in Production Example 1-2 and 1,3-propanediol (2.5 mL), the entitled compound (408 mg) was obtained as a white powder.

### Production Example 10-2:

### 6-Iodo-2-(3-pyrrolidin-1-ylpropoxy)quinoline:

According to the same process as in Example 3 but using the compound (440 mg) obtained in Production Example 10-1 and pyrrolidine (20 mL) followed by purification by silica gel column chromatography (NH silica gel, hexane:ethyl acetate = 4:1), the entitled compound (406 mg) was obtained.

### Production Example 11-1:

### 4-Hydroxy-1-[2-(3-pyrrolidin-1-ylpropoxy)quinolin-6-yl]pyridin-2(1H)-one:

According to the same process as in Production Example 1-5 but using the compound (220 mg) obtained in Example 19, the entitled compound (165 mg) was obtained.

### Production Example 12-1:

### 4-[(4-Fluorobenzyl)oxy]-1-(2-methylquinolin-6-yl)pyridin-2(1H)-one:

According to the same process as in Production Example 1-4 but using 6-bromo-2-methylquinoline (500 mg) and 4-(fluorobenzyl)oxy-2(1H)-pyridone (986 mg) followed by purification by silica gel column chromatography (chloroform:methanol = 40:1 to 40:2), the entitled compound (720 mg) was obtained.

### Production Example 12-2:

### {6-[4-[(4-Fluorobenzyl)oxy]-2-oxopyridin-1(2H)-yl]quinolin-2-yl}methyl 4-methylbenzenesulfonate:

1) According to the same process as in Production Example 3-3-1) but using the compound (600 mg) obtained in Production Example 12-1 followed by purification by silica gel column chromatography (chloroform:methanol = 40:1), 4-[(4-fluorobenzyl)oxy]-1-(2-methyl-1-oxidoquinolin-6-yl)pyridin-2(1H)-one (273 mg) was obtained as a yellow white powder.
2) Potassium carbonate (300 mg) and p-toluenesulfonyl chloride (200 mg) were added to an acetonitrile/THF mixed solution (1:1, 20 mL) of the compound (218 mg) obtained in Production Example 12-2-1), and stirred overnight at room temperature. The solvent was evaporated off from the reaction liquid under reduced pressure, aqueous saturated sodium hydrogencarbonate solution was added to the residue, and extracted with ethyl acetate. The organic layer was dried with anhydrous magnesium sulfate, then the solvent was evaporated off under reduced pressure. The obtained residue was purified by silica gel column chromatography (chloroform:methanol = 400:5 to 400:10) to obtain the entitled compound (110 mg) as a yellow oily substance.

### Production Example 13-1:

### 6-[4-[(4-Fluorobenzyl)oxy]-2-oxopyridin-1(2H)-yl]quinoline-2-carbaldehyde:

Selenium dioxide (120 mg) was added to a dioxane solution (20 mL) of the compound (300 mg) obtained in Production Example 12-1, and heated under reflux for 1.5 hours. The reaction liquid was filtered through Celite, the solvent was evaporated off under reduced pressure. The obtained residue was purified by silica gel column chromatography (chloroform:methanol = 50:1) to obtain the entitled compound (299 mg) as a gray brown solid.

### Production Example 14-1:

### 4-(Benzyloxy)-1 -(2-methylquinolin-6-yl)pyridin-2(1H)-one:

In the same manner as in Production Example 1-4 but using 6-bromo-2-methylquinoline (6.8 g) and 4-benzyloxy-2(1H)-pyridone (5 g) followed by purification by silica gel column chromatography (chloroform:methanol = 40:1), the entitled compound (5.5 g) was obtained as a yellow powder.

### Production Example 15-1:

### 6-Iodo-1H-indazole:

An aqueous solution (4 mL) of sodium nitrite (1.1 g) was dropwise added to an acetic acid solution (360 mL) of (5-iodo-2-methylphenyl)amine, and after the addition, this was stirred overnight at room temperature. The solvent was evaporated off under reduced pressure, the obtained residue was purified by silica gel column chromatography (hexane:ethyl acetate = 9:1 to 4:1) to obtain the entitled compound (1 g) as an orange powder.

### Production Example 15-2:

### 6-Iodo-1-(2-pyrrolidin-1-ylethyl)-1H-indazole and 6-iodo-2-(2-pyrrolidin-1-ylethyl)-2H-indazole:

Potassium carbonate was added to a DMF solution (10 mL) of the compound (1 g) obtained in Production Example 15-1, and stirred at room temperature for 30 minutes. N-(2-chloroethyl)pyrrolidine hydrochloride (1.1 g) was added to the reaction liquid, and stirred overnight at 60°C. Water was added to the reaction liquid, and extracted with a mixed solvent of chloroform/methanol (10:1). The organic layer was dried with anhydrous magnesium sulfate, then the solvent was evaporated off under reduced pressure. The obtained residue was purified by silica gel column chromatography (NH silica gel, hexane:ethyl acetate = 4:1 to 3:2) to obtain 6-iodo-1-(2-pyrrolidin-2-ylethyl)-1H-indazole (838 mg) and 6-iodo-2-(2-pyrrolidin-1-ylethyl)-2H-indazole (338 mg) successively.

### Production Example 16-1:

### 6-Methylquinoline 1-oxide:

M-chloroperbenzoic acid (45.3 g) was added to a dichloromethane solution (250 mL) of 6-methylquinoline (25 g), and stirred overnight at room temperature. Chloroform was added to the reaction liquid, and washed with aqueous saturated sodium hydrogencarbonate solution. The organic layer was dried with anhydrous magnesium sulfate, the solvent was evaporated off under reduced pressure. The obtained residue was purified by silica gel column chromatography (hexane:ethyl acetate = 3:2 to chloroform:methanol = 9:1) to obtain the entitled compound (13.6 g) as a yellow oily substance.

### Production Example 16-2:

### 2-Chloro-6-methylquinoline:

Phosphorus oxychloride (35 mL) was dropwise added to a toluene solution (39 mL) of the compound (9.4 g) obtained in Production Example 16-1 and DMF (3.9 mL). After the addition, this was stirred at 100°C for 2 hours. The reaction liquid was dropwise added to a mixed solvent of chloroform and aqueous saturated sodium hydrogencarbonate solution. After the addition, the organic layer was separated, dried with anhydrous magnesium sulfate, and the solvent was evaporated off under reduced pressure. The obtained residue was purified by silica gel column chromatography (hexane:ethyl acetate = 4:1) to obtain the entitled compound (3.0 g) as a white solid.

### Production Example 16-3:

### 6-(Bromomethyl)-2-chloroquinoline:

N-bromosuccinimide (997 mg) and benzoyl peroxide (136 mg) were added to a benzene solution (20 mL) of the compound (1 g) obtained in Production Example 16-2, and heated under reflux for 2 hours. The solvent was evaporated off from the reaction liquid under reduced pressure, then aqueous saturated sodium hydrogencarbonate solution was added to the obtained residue, and extracted with chloroform. The chloroform layer was dried with anhydrous magnesium sulfate, and the solvent was evaporated off under reduced pressure. The obtained residue was purified by silica gel column chromatography (hexane:ethyl acetate = 9:1 to 4:1) to obtain the entitled compound (1.3 g) as a white solid. Production Example 16-4:

### 6-(Azetidin-1-ylmethyl)-2-chloroquinoline:

Potassium carbonate (7.4 g) was added to a methanol solution (20 mL) of azetidine hydrochloride (1 g), and stirred at room temperature for 1 hour. The reaction liquid was filtered, a THF solution (2 ml) of the compound (500 mg) obtained in Production Example 16-3 was dropwise added to the filtrate. After the addition, this was stirred overnight at room temperature. The solvent was evaporated off from the reaction liquid under reduced pressure, then aqueous saturated sodium hydrogencarbonate solution was added to the obtained residue, and extracted with a mixed solvent of chloroform/methanol (10:1). The organic layer was dried with anhydrous magnesium sulfate, then the solvent was evaporated off under reduced pressure. The obtained residue was purified by silica gel column chromatography (hexane:ethyl acetate = 4:1 to chloroform:methanol = 10:1) to obtain the entitled compound (190 mg) as a yellow oily substance.

### Production Example 17-1:

### 4-Bromo-2-nitro-N-(3-pyrrolidin-1-ylpropyl)benzamide:

N,N-diisopropylethylamine (1.0 mL) was added to a DMF solution (5 mL) of 4-bromo-2-nitrobenzoic acid (500 mg), (3-pyrrolidin-1-ylpropyl)amine (290.6 mg) and O-(7-azabenzotriazol-1-yl)-1,1,3,3-tetramethyluronium hexafluorophosphate (912 mg), and stirred overnight at room temperature. Water was added to the reaction liquid, and extracted with a mixed solvent of chloroform/methanol (10:1). The organic layer was dried with anhydrous magnesium sulfate, then the solvent was evaporated off under reduced pressure. The obtained residue was purified by silica gel column chromatography (ethyl acetate) to obtain the entitled compound (629 mg) as a yellow oily substance.

### Production Example 17-2:

### 2-Amino-4-bromo-N-(3-pyrrolidin-1-ylpropyl)benzamide:

Iron powder (581.7 mg) and ammonium chloride (1.1 g) were added to a methanol/THF mixed solution (1:1, 70 mL) of the compound (620 mg) obtained in Production Example 17-1, and heated under reflux for 30 minutes. The reaction liquid was filtered through Celite, the solvent was evaporated off from the filtrate under reduced pressure. Water was added to the obtained residue, and extracted with a mixed solvent of chloroform/methanol (10:1). The organic layer was dried with anhydrous magnesium sulfate, then the solvent was evaporated off under reduced pressure to obtain the entitled compound (594 mg).

### Production Example 17-3:

### 7-Bromo-3-(3-pyrrolidin-1-ylpropyl)quinazolin-4(3H)-one:

Triethyl orthoformate (21 mL) was added to an ethanol solution (40 mL) of the compound (470 mg) obtained in Production Example 17-2, and heated overnight under reflux. The solvent was evaporated off from the reaction liquid under reduced pressure, and the obtained residue was purified by silica gel column chromatography (NH silica gel, hexane:ethyl acetate = 1:1) to obtain the entitled compound (238 mg) as a yellow oily substance.

### Production Example 18-1:

### 4-[(5-Chloropyridin-2-yl)methoxy]-1-(2-methylquinolin-6-yl)pyridin-2(1H)-one:

In the same manner as in Production Example 1-5 but using the compound (300 mg) obtained in Production Example 14-1, 4-hydroxy-1-(2-methylquinolin-6-yl)pyridin-2(1H)-one was obtained. Using this and (5-chloropyridin-2-yl)methyl methanesulfonate (205 mg) and in the same manner as in Example 1, the entitled compound (297 mg) was obtained.

### Production Example 18-2:

### 6-[4-[(5-Chloropyridin-2-yl)methoxy]-2-oxopyridin-1(2H)-yl]quinolin-2-carbaldehyde:

In the same manner as in Production Example 13-1 but using the compound (100 mg) obtained in Production Example 18-1, the entitled compound (103 mg) was obtained.

### Production Example 19-1:

### 4-(Benzyloxy)-1-[2-(hydroxymethyl)quinolin-6-yl]pyridin-2(1H)-one:

According to the same process as that of Production Example 3-3-1) to 3-3-3) but using the compound (1.6 g) obtained in Production Example 14-1, the entitled compound (1.2 g) was obtained.

### Production Example 19-2:

### 4-(Benzyloxy)-1-{2-[(tetrahydro-2H-pyran-2-yloxy)methyl]quinolin-6-yl}pyridin-2(1H)-one:

3,4-Dihydro-2H-pyran (3.8 mL) and p-toluenesulfonic acid monohydrate (199 mg) were added at room temperature to a chloroform/THF mixed solution (2:1, 90 mL) of the compound (1.5 g) obtained in Production Example 19-1. The reaction solution was heated at 50°C and stirred for 12 hours, and then poured into aqueous saturated sodium hydrogencarbonate solution, and extracted with chloroform. The organic layer was washed with saturated saline water, then dried with anhydrous sodium sulfate. The solvent was evaporated off under reduced pressure, and the residue was purified by silica gel column chromatography (ethyl acetate) to obtain the entitled compound (1.4 g).

### Production Example 19-3:

### 4-[(5-Chloropyridin-2-yl)methoxy]-1-{2-[(tetrahydro-2H-pyran-2-yloxy)methyl]quinolin-6-yl}pyridin-2(1H)-one:

In the same manner as in Production Example 1-5 but using the compound (1.4 g) obtained in Production Example 19-2, 4-hydroxy-1-{2-[(tetrahydro-2H-pyran-2-yloxy)methyl]quinolin-6-yl}pyridin-2(1H)-one was obtained. Using this and (5-chloropyridin-2-yl)methyl methanesulfonate (732 mg) and in the same manner as in Example 1, the entitled compound (1.4 g) was obtained.

### Production Example 19-4:

### 4-[(5-Chloropyridin-2-yl)methoxy]-1-[2-(hydroxymethyl)quinolin-6-yl]pyridin-2(1H)-one:

1 N hydrochloric acid (8 mL) was added at room temperature to a methanol suspension (40 mL) of the compound (797 mg) obtained in Production Example 19-3, and stirred for 12 hours. The reaction liquid was poured into aqueous saturated sodium hydrogencarbonate solution, and extracted with chloroform. The organic layer was washed with saturated saline water, then dried with anhydrous sodium sulfate. The solvent was evaporated off under reduced pressure, and the residue was washed with ethyl acetate and dried under reduced pressure to obtain the entitled compound (675 mg).

### Production Example 20-1:

### 4-[(4-Fluorobenzyl)oxy]-1-[2-(hydroxymethyl)quinolin-6-yl]pyridin-2(1H)-one:

According to the same process as that of Production Example 3-3-1) to 3-3-3) but using the compound (3.0 g) obtained in Production Example 12-1, the entitled compound (2.3 g) was obtained.

### Production Example 20-2:

### {6-[4-[(4-Fluorobenzyl)oxy]-2-oxopyridin-1(2H)-yl]quinolin-2-yl}methyl methanesulfonate:

In the same manner as in Example 30-1) but using the compound (400 mg) obtained in Production Example 20-1, the entitled compound (480 mg) was obtained.

### Production Example 21-1:

### 6-[4-(Benzyloxy)-2-oxopyridin-1(2H)-yl]quinoline-2-carbaldehyde:

At room temperature, manganese dioxide (974 mg) was added to a chloroform solution (100 mL) of the compound (1.0 g) obtained in Production Example 19-1. The reaction liquid was stirred at 50°C for 16 hours, then the insoluble matter was separated by filtration. The solvent was evaporated off from the filtrate under reduced pressure to obtain the entitled compound (980 mg) as a crude product.

### Production Example 22-1:

### 6-[4-[(4-Fluorobenzyl)oxy]-2-oxopyridin-1(2H)-yl]quinoline-2-carbaldehyde:

In the same manner as in Production Example 21-1 but using the compound (200 mg) obtained in Production Example 20-1, the entitled compound (185 mg) was obtained as a crude product.

### Production Example 23-1:

### 4-[(5-Chloropyridin-2-yl)methoxy]-1-[2-(1-hydroxyethyl)quinolin-6-yl]pyridin-2(1H)-one:

At room temperature, a THF solution (5 mL) of the compound (274 mg) obtained in Production Example 18-2 was dropwise added to a THF solution (3.5 mL) of 1.0 M methylmagnesium bromide. After stirred for 6 hours, a THF solution (3.5 mL) of 1.0 M methylmagnesium bromide was added to the reaction liquid at room temperature, and further stirred for 6 hours. The reaction liquid was poured into water, and extracted with chloroform. The organic layer was washed with saturated saline water, and dried with anhydrous sodium sulfate. The solvent was evaporated off under reduced pressure, and the residue was purified by silica gel column chromatography (chloroform:methanol = 40:1) to obtain the entitled compound (190 mg).

### Production Example 23-2:

### 1-(2-Acetylquinolin-6-yl)-4-[(5-chloropyridin-2-yl)methoxy]pyridin-2(1H)-one:

At -78°C, a dichloromethane solution (2 mL) of dimethyl sulfoxide (100 µL) was dropwise added to a dichloromethane solution (3 mL) of oxalyl chloride (61.5 µL). After stirred for 10 minutes, a dichloromethane solution (5 mL) of the compound (190 mg) obtained in Production Example 23-1 was dropwise added to the reaction liquid at -78°C. After stirred for 30 minutes, triethylamine (327 µL) was added thereto at -78°C, and heated up to room temperature. After further stirred for 30 minutes, the reaction liquid was poured into water, and extracted with chloroform. The organic layer was washed with saturated saline water, and dried with anhydrous sodium sulfate. The solvent was evaporated off under reduced pressure, and the residue was purified by silica gel column chromatography (chloroform:methanol = 70:1 to 60:1) to obtain the entitled compound (146 mg).

### Production Example 24-1:

### 2-Methyl-6-nitroquinoxaline:

At room temperature, 40 % pyruvinaldehyde solution (25 ml) was added to an ethanol suspension (250 mL) of 1,2-diamino-4-nitrobenzene (5.0 g). After this was stirred for 30 minutes, the solvent was evaporated off under reduced pressure. The residue was recrystallized from ethyl acetate/hexane to obtain the entitled compound (2.4 g).

### Production Example 24-2:

### 6-Iodo-2-methylquinoxaline:

At room temperature, ammonium chloride (3.4 g) and iron powder (1.8 g) were added to a methanol/water mixed solution (2:1, 60 mL) of the compound (1.0 g) obtained in Production Example 24-1. The reaction liquid was heated under reflux, then stirred for 3 hours, and the solvent was evaporated off under reduced pressure. Aqueous saturated sodium hydrogencarbonate solution was added to the residue, and extracted with chloroform. The organic layer was dried with anhydrous sodium sulfate, then the solvent was evaporated off under reduced pressure. The residue was suspended in water (10 mL), then at 0°C, concentrated hydrochloric acid (3 mL) was added. After stirred for 30 minutes, an aqueous solution (10 mL) of sodium nitrite (548 mg) was dropwise added to the reaction liquid at 0°C. Further this was stirred for 30 minutes, and an aqueous solution (10 mL) of potassium iodide (2.2 g) was dropwise added at 0°C. This was heated up to room temperature and stirred for 6 hours, and then dichloromethane, aqueous saturated sodium sulfite solution, and aqueous 2 N sodium hydroxide solution (20 mL) were successively added. The insoluble matter was separated by filtration, and the filtrate was extracted with chloroform. The organic layer was washed with saturated saline water, then dried with anhydrous sodium sulfate. The solvent was evaporated off under reduced pressure, and the residue was purified by silica gel column chromatography (ethyl acetate:hexane = 1:4.5 to 1:2.5) to obtain the entitled compound (758 mg).

### Production Example 24-3:

### 4-[(4-Fluorobenzyl)oxy]-1-(2-methylquinoxalin-6-yl)pyridin-2(1H)-one:

In the same manner as in Production Example 1-4 but using the compound (2.5 g) obtained in Production Example 24-2 and 4-(fluorobenzyl)oxy-2(1H)-pyridone (2.0 g), the entitled compound (1.4 g) was obtained.

### Production Example 24-4:

### 6-[4-[(4-Fluorobenzyl)oxy]-2-oxopyridin-1(2H)-yl]quinoxaline-2-carbaldehyde:

In the same manner as in Production Example 13-1 but using the compound (600 mg) obtained in Production Example 24-3, the entitled compound (520 mg) was obtained.

### Production Example 25-1:

### 4-[(4-Fluorobenzyl)oxy]-1-[2-(hydroxymethyl)quinoxalin-6-yl]pyridin-2(1H)-one:

At 0°C, sodium borohydride (30.8 mg) was added to a methanol/THF/dichloromethane mixed solution (2:2:1, 25 mL) of the compound (282 mg) obtained in Production Example 24-4. After heated up to room temperature and stirred for 1 hour, the solvent was evaporated off under reduced pressure. Aqueous saturated sodium hydrogencarbonate solution was added to the residue, and extracted with chloroform. The organic layer was dried with anhydrous sodium sulfate, then the solvent was evaporated off under reduced pressure. The residue was purified by silica gel column chromatography (chloroform:methanol = 30:1 to 15:1) to obtain the entitled compound (219 mg).

### Production Example 26-1:

### Quinoxalin-2(1H)-one:

At room temperature, 1,2-phenylenediamine (1.0 g) was added to a methanol solution (30 mL) of glyoxylic acid monohydrate (3.0 g). After stirred for 30 minutes, the precipitate was collected by filtration. The solid on the filter paper was washed with methanol and diethyl ether, then dried under reduced pressure to obtain the entitled compound (1.1 g).

### Production Example 26-2:

### 6-Nitroquinoxalin-2(1 1H)-one:

As divided in plural portions, potassium nitrate (3.7 g) was intermittently added at 0°C to a concentrated sulfuric acid solution (60 mL) of the compound (5.3 g) obtained in Production Example 26-1. After heated up to room temperature and stirred for 6 hours, the formed precipitate was collected by filtration, and washed with water and diisopropyl ether. This was dried under reduced pressure to obtain the entitled compound (7.1 g).

### Production Example 26-3:

### 2-Chloro-6-nitroquinoxaline:

At room temperature, phosphorus pentoxide (15.2 g) was added to a phosphorus oxychloride solution (70 mL) of the compound (7.1 g) obtained in Production Example 26-2. The reaction liquid was stirred with heating under reflux for 8 hours, then cooled to room temperature, and poured into water with ice. The formed precipitate was collected by filtration and washed with water. This was dried under reduced pressure to obtain the entitled compound (2.4 g).

### Production Example 26-4:

### 2-Chloro-6-iodoquinoxaline:

In the same manner as in Production Example 24-2 but using the compound (700 mg) obtained in Production Example 26-3, the entitled compound (288 mg) was obtained.

### Production Example 26-5:

### 6-Iodo-2-(2-pyrrolidin-1-ylethoxy)quinoxaline:

At room temperature, 1-(2-hydroxyethyl)pyrrolidine (174 µL) and sodium hydride (79.2 mg) were added to a DMF solution (6 mL) of the compound (288 mg) obtained in Production Example 26-4. The reaction liquid was stirred at 60°C for 4.5 hours, then poured into water, and extracted with ethyl acetate. The organic layer was washed with saturated saline water, and dried with anhydrous sodium sulfate. The solvent was evaporated off under reduced pressure, and the residue was purified by silica gel column chromatography (chloroform:methanol = 60:1 to 15:1) to obtain the entitled compound (259 mg).

### Production Example 27-1:

### 2-(2-Pyrrolidin-1-ylethoxy)-6-(trimethylstannyl)quinoxaline:

In a nitrogen atmosphere at room temperature, hexamethylditin (55.0 mg), lithium chloride (17.8 mg), 2,6-di-t-butyl-4-methylphenol (1.5 mg) and tetrakis(triphenylphosphino)palladium (8.0 mg) were successively added to a THF solution (5 mL) of the compound (50.0 mg) obtained in Production Example 26-5. The reaction liquid was stirred at 80°C for 3 hours, then poured into saturated saline water, and extracted with chloroform. The organic layer was dried with anhydrous sodium sulfate, then the solvent was evaporated off under reduced pressure. Without purification, the residue was used directly in the next reaction.

### Production Example 28-1:

### 2-Fluoro-5-nitrobenzaldehyde:

At room temperature, potassium fluoride (15.7 g) was added to a DMF solution (100 mL) of 2-chloro-5-nitrobenzaldehyde (25.0 g). The reaction liquid was stirred at 160°C for 1.5 hours, then poured into water with ice. After extracted with ethyl acetate, the organic layer was washed with saturated saline water, and dried with anhydrous sodium sulfate. The solvent was evaporated off under reduced pressure, and the residue was purified by silica gel column chromatography (hexane:ethyl acetate = 6:1 to 3:1) to obtain the entitled compound (17.1 g).

### Production Example 28-2:

### 2-Methyl-6-nitroquinazoline:

At room temperature, molecular sieve (4A, powder, 20.0 g), potassium carbonate (19.7 g) and acetamidine hydrochloride (13.5 g) were successively added to an acetonitrile solution (600 mL) of the compound (17.1 g) obtained in Production Example 28-1. The reaction liquid was stirred for 11 hours with heating under reflux, then the insoluble matter was separated by filtration. The solvent was evaporated off from the filtrate under reduced pressure, and the residue was purified by silica gel column chromatography (hexane:ethyl acetate = 2:1 1 to 1:1.5). The obtained solid was washed with a mixed solvent of hexane/ethyl acetate (4:1, 100 mL), then dried under reduced pressure to obtain the entitled compound (6.6 g).

### Production Example 28-3:

### 6-Iodo-2-methylquinazoline:

In the same manner as in Production Example 24-2 but using the compound (7.7 g) obtained in Production Example 28-2, the entitled compound (3.9 g) was obtained.

### Production Example 28-4:

### 4-[(4-Fluorobenzyl)oxy]-1-(2-methylquinazolin-6-yl)pyridin-2(1H)-one:

In the same manner as in Production Example 1-4 but using the compound (1.5 g) obtained in Production Example 28-3 and 4-(fluorobenzyl)oxy-2(1H)-pyridone (1.2 g), the entitled compound (728 mg) was obtained.

### Production Example 28-5:

### 6-[4-[(4-Fluorobenzyl)oxy]-2-oxopyridin-1(2H)-yl]quinazoline-2-carbaldehyde:

In the same manner as in Production Example 13-1 but using the compound (727 mg) obtained in Production Example 28-4, the entitled compound (657 mg) was obtained.

### Production Example 28-6:

### 4-[(4-Fluorobenzyl)oxy]-1-[2-(hydroxymethyl)quinazolin-6-yl]pyridin-2(1H)-one:

In the same manner as in Production Example 25-1 but using the compound (300 mg) obtained in Production Example 28-5, the entitled compound (171 mg) was obtained.

### Production Example 29-1:

### Diethyl 2-propionylsuccinate:

At room temperature, benzoyl peroxide (59.6 mg) was added to a mixture of diethyl maleate (10.0 mL) and propionaldehyde (8.9 mL). With heating at 80°C under irradiation with light, the reaction liquid was stirred for 6 hours, then benzoyl peroxide (59.6 mg) was added. Further under irradiation with light, this was stirred for 12 hours, and then the compound was purified by distillation under reduced pressure (116°C, 3.5 Torr) to obtain the entitled compound (6.9 g).

### Production Example 29-2:

### Ethyl 4-oxohexanoate:

Boric acid (1.9 g) was added at room temperature to the compound (6.9 g) obtained in Production Example 29-1, then the reaction mixture was heated up to 150°C, and stirred for 1 hour. This was further stirred at 170°C for 2 hours, then cooled to room temperature. The reaction liquid was poured into water, and extracted with toluene. The organic layer was dried with anhydrous sodium sulfate, the solvent was evaporated off under reduced pressure. The residue was purified by distillation under reduced pressure (84°C, 8 Torr) to obtain the entitled compound (4.6 g).

### Production Example 29-3:

### Ethyl 5-bromo-4-oxohexanoate:

Bromine (323 µL) was dropwise added at 0°C to a diethyl ether solution (2 mL) of the compound (1.0 g) obtained in Production Example 29-2. After stirred for 1 hour, the reaction liquid was poured into aqueous saturated sodium hydrogencarbonate solution. After extracted with diethyl ether, the organic layer was washed with aqueous saturated sodium sulfite solution and saturated saline water. The organic layer was dried with sodium sulfate, the solvent was evaporated off under reduced pressure to obtain the entitled compound (1.5 g) as a crude product.

### Production Example 29-4:

### Ethyl 5-[2-{[(4-methylphenyl)sulfonyl]imino}-5-nitropyridin-1(2H)-yl]-4-oxohexanoate:

At room temperature, diisoproylethylamine (838 µL) was added to a DMF solution (10 mL) of the compound (1.5 g) obtained in Production Example 29-3 and 4-methyl-N-(5-nitropyridin-2-yl)benzenesulfonamide (1.3 g) obtained according to JP-A 2004-139909. After stirred for 12 hours, the reaction liquid was poured into water. The obtained mixture was extracted with ethyl acetate, the organic layer was washed with saturated saline water, and dried with sodium sulfate. The solvent was evaporated off under reduced pressure, and the residue was purified by silica gel column chromatography (ethyl acetate:chloroform:hexane = 1:1:1 to 2:2:1) to obtain the entitled compound 1 (635 mg).

### Production Example 29-5:

### Ethyl 3-(3-methyl-6-nitroimidazo[1,2-a]pyridin-2-yl)propanoate:

At room temperature, trifluoroacetic anhydride (3 mL) was added to a dichloromethane solution (6 mL) of the compound (635 mg) obtained in Production Example 29-4. The reaction liquid was heated at 40°C, then stirred for 6 hours, and the solvent was evaporated off under reduced pressure. Aqueous saturated sodium hydrogencarbonate solution was added to the residue, and extracted with ethyl acetate. The organic layer was dried with sodium sulfate, then the solvent was evaporated off under reduced pressure, and the residue was purified by silica gel column chromatography (ethyl acetate:chloroform:hexane = 1:1:1) to obtain the entitled compound (328 mg).

### Production Example 29-6:

### Ethyl 3-(6-amino-3-methylimidazo[1,2-a]pyridin-2-yl)propanoate:

In a nitrogen atmosphere, 10 % palladium-carbon (20 mg) was added at room temperature to a methanol/THF mixed solution of the compound (50 mg) obtained in Production Example 29-5. The reaction system was purged with hydrogen (1 atmospheric pressure), stirred at room temperature for 1 hour, and then the catalyst was separated by filtration. The solvent was evaporated off from the filtrate under reduced pressure, and the residue was dried under reduced pressure to obtain the entitled compound (44.5 mg).

### Production Example 29-7:

### Ethyl 3-[6-(4-hydroxy-2-oxopyridin-1(2H)-yl)-3-methylimidazo [1,2-a]pyridin-2-yl)propanoate:

In the same manner as in Production Example 2-1 but using the compound (266 mg) obtained in Production Example 29-6, the entitled compound (69.6 mg) was obtained.

### Production Example 29-8:

### Ethyl 3- {6-[4-[(5-chloropyridin-2-yl)methoxy]-2-oxopyridin-1(2H)-yl]-3-methylimidazo[1,2-a]pyridin-2-yl}propanoate:

In the same manner as in Example 1 but using the compound (34.2 mg) obtained in Production Example 29-7 and (5-chloropyridin-2-yl)methyl methanesulfonate, the entitled compound (45.7 mg) was obtained.

### Production Example 29-9:

### 4-[(5-Chloropyridin-2-yl)methoxy]-1-[2-(3-hydroxypropyl)-3-methylimidazo[1,2-a]pyridin-6-yl]pyridin-2(1H)-one:

At 0°C, a toluene solution (250 µL) of 1.0 M diisobutylaluminium hydride was dropwise added to a dichloromethane solution (4 mL) of the compound (46.7 mg) obtained in Production Example 29-8. After stirred for 1 hour, sodium sulfate 10-hydrate was added, and further stirred for 1 hour at room temperature. The insoluble matter was separated by filtration, the solvent was evaporated off from the filtrate under reduced pressure. The residue was purified by silica gel column chromatography (chloroform:methanol = 80:1) to obtain the entitled compound (16.0 mg).

### Production Example 30-1:

### Methyl 6-[4-[(5-chloropyridin-2-yl)methoxy]-2-oxopyridin-1(2H)-yl]-3-methylimidazo[1,2-a]pyridine-2-carboxylate:

1) In the same manner as in Production Example 29-6 but using methyl 3-methyl-6-nitroimidazo[1,2-a]pyridine-2-carboxylate (250 mg) produced according to JP-A 2004-139909, methyl 6-amino-3-methylimdazo[1,2-a]pyridine-2-carboxylate (215 mg) was obtained.
2) In the same manner as in Production Example 2-1 but using the compound (215 mg) obtained in Production Example 30-1-1), methyl 6-(4-hydroxy-2-oxopyridin-1(2H)-yl)-3-methylimdazo[1,2-a]pyridine-2-carboxylate (131 mg) was obtained.
3) In the same manner as in Example 1 but using the compound (131 mg) obtained in Production Example 30-1-2) and (5-chloropyridin-2-yl)methyl methanesulfonate (253 mg), the entitled compound (60.0 mg) was obtained.

### Production Example 31-1:

### Ethyl 3-(1-methyl-6-nitro-1H-benzimidazol-2-yl)propanoate:

At room temperature, a chloroform solution (10 mL) of ethyl 4-chloro-4-oxobutyrate (988 mg) was dropwise added to a chloroform solution (30 mL) of N²-methyl-4-nitrobenzene-1,2-diamine (1.0 g) and triethylamine (835 µL). After stirred for 3 hours, the reaction liquid was poured into aqueous saturated sodium hydrogencarbonate solution, and extracted with chloroform. The organic layer was washed with saturated saline water, then dried with anhydrous sodium sulfate. The solvent was evaporated off under reduced pressure, and the obtained residue was dissolved in ethanol (30 mL). Concentrated hydrochloric acid (4.5 mL) was added to the reaction liquid at room temperature, heated at 90°C and stirred for 14 hours. The solvent was evaporated off under reduced pressure, and aqueous saturated sodium hydrogencarbonate solution was added to the residue, and extracted with ethyl acetate. The organic layer was washed with saturated saline water, then dried with anhydrous sodium sulfate. The solvent was evaporated off under reduced pressure, and the residue was purified by silica gel column chromatography (chloroform:methanol = 30:1) to obtain the entitled compound (1.2 g).

### Production Example 31-2:

### Ethyl 3-(6-iodo-1-methyl-1H-benzimidazol-2-yl)propanoate:

In the same manner as in Production Example 24-2 but using the compound (150 mg) obtained in Production Example 31-1, the entitled compound (69.5 mg) was obtained. Production Example 31-3:

### Ethyl 3- {6-[4-[(4-chlorobenzyl)oxy]-2-oxopyridin-1(2H)-yl]-1-methyl-1H-benzimidazol-2-yl}propanoate:

In the same manner as in Production Example 1-4 but using the compound (69.5 mg) obtained in Production Example 31-2 and 4-[(4-chlorobenzyl)oxy]pyridin-2(1H)-one (67.2 mg), the entitled compound (8.9 mg) was obtained.

### Production Example 32-1:

### Ethyl 3-[6-(4-hydroxy-2-oxopyridin-1(2H)-yl)-1-methyl-1H-benzimidazol-2-yl]propanoate:

1) In the same manner as in Production Example 29-6 but using the compound (250 mg) obtained in Production Example 31-1, ethyl 3-(6-amino-1-methyl-1H-benzimidazol-2-yl)propanoate (220 mg) was obtained.
2) In the same manner as in Production Example 2-1 but using the compound (220 mg) obtained in Production Example 32-1-1), the entitled compound (51.2 mg) was obtained.

### Production Example 32-2:

### Ethyl 3- {6-[4-[(5-chloropyridin-2-yl)methoxy]-2-oxopyridin-1(2H)-yl]-1-methyl-1H-benzimidazol-2-yl}propanoate:

In the same manner as in Example 1 but using the compound (51.2 mg) obtained in Production Example 32-1 and (5-chloropyridin-2-yl)methyl methanesulfonate (43.2 mg), the entitled compound (48.0 mg) was obtained.

### Production Example 33-1:

### 2-Chloro-6-iodo-1-methyl-1H-benzimidazole:

In the same manner as in Production Example 24-2 but using 2-chloro-1-methyl-6-nitro-1H-benzimidazole (100 mg), the compound (114 mg) was obtained.

### Production Example 33-2:

### 6-Iodo-1-methyl-2-(2-pyrrolidin-1-ylethoxy)-1H-benzimidazole:

In the same manner as in Production Example 26-5 but using the compound (114 mg) obtained in Production Example 33-1 and 1-(2-hydroxyethyl)pyrrolidine (68.4 µL), the entitled compound (114 mg) was obtained.

### Production Example 33-3:

### 1-Methyl-2-(2-pyrrolidin-1-ylethoxy)-6-(trimethylstannyl)-1H-benzimidazole:

In the same manner as in Production Example 27-1 but using the compound (55.7 mg) obtained in Production Example 33-2, the entitled compound was obtained as a crude product.

### Production Example 34-1:

### 6-Nitroindoline-2-carboxylic acid:

As divided in a few portions, indoline-2-carboxylic acid (5.0 g) was intermittently added at -10°C to concentrated sulfuric acid (40 mL), and further fuming nitric acid (1.4 mL) was dropwise added at -20 to -10°C. After stirred for 30 minutes, the reaction liquid was poured into water with ice. The aqueous layer was washed with ethyl acetate, and made to have a pH of from 4 to 5 by addition of aqueous 5 N sodium hydroxide solution thereto. The aqueous layer was extracted with ethyl acetate, then the organic layer was washed with saturated saline water, and dried with anhydrous sodium sulfate. The solvent was evaporated off under reduced pressure to obtain the entitled compound (4.0 g) as a crude product.

### Production Example 34-2:

### Methyl 6-nitroindoline-2-carboxylate:

At room temperature, p-toluenesulfonic acid monohydrate (1.8 g) was added to a methanol suspension (20 mL) of the compound (4.0 g) obtained in Production Example 34-1. The reaction liquid was stirred at 80°C for 1 hour, then the solvent was evaporated off under reduced pressure. Aqueous saturated sodium hydrogencarbonate solution was added to the residue, and extracted with ethyl acetate. The organic layer was washed with water and saturated saline water, then dried with anhydrous sodium sulfate. The solvent was evaporated off under reduced pressure to obtain the entitled compound (4.0 g) as a crude product.

### Production Example 34-3:

### Methyl 6-nitro-1H-indole-2-carboxylate:

At room temperature, 2,3-dichloro-5,6-dicyano-1,4-benzoquinone (4.5 g) was added to a mixed solution of benzene/ethyl acetate (2:1, 30 mL) of the compound (4.0 g) obtained in Production Example 34-2. The reaction liquid was heated at 80°C and stirred for 45 minutes, then poured into aqueous saturated sodium hydrogencarbonate solution. The mixture was extracted with ethyl acetate, the organic layer was washed three times with aqueous saturated sodium hydrogencarbonate solution, then once with saturated saline water, and dried with anhydrous sodium sulfate. The solvent was evaporated off under reduced pressure to obtain the entitled compound (3.9 g) as a crude product.

### Production Example 34-4:

### Methyl 1-methyl-6-nitro-1H-indole-2-carboxylate:

At room temperature, dimethyl carbonate (15 mL) and 1,4-diazabicyclo[2,2,2]octane (329 mg) were successively added to a DMF solution (2 mL) of the compound (1.5 g) obtained in Production Example 34-3. The reaction liquid was stirred at 95°C for 17 hours, then poured into water. The mixture was extracted with chloroform, the organic layer was washed with aqueous 10 % citric acid solution and saturated saline water, then dried with anhydrous sodium sulfate. The solvent was evaporated off under reduced pressure to obtain the entitled compound (1.5 g) as a crude product.

### Production Example 34-5:

### Methyl 6-(4-hydroxy-2-oxopyridin-1(2H)-yl)-1-methyl-1H-indole-2-carboxylate:

1) In the same manner as in Production Example 29-6 but using the compound (500 mg) obtained in Production Example 34-4, methyl 6-amino-1-methyl-1H-indole-2-carboxylate (430 mg) was obtained.
2) In the same manner as in Production Example 2-1 but using the compound (430 mg) obtained in Production Example 34-5-1), the entitled compound (159 mg) was obtained. Production Example 34-6:

### 4-[(5-Chloropyridin-2-yl)methoxy]-1-[2-(hydroxymethyl)-1-methyl-1H-indol-6-yl]pyridin-2(1H)-one:

1) In the same manner as in Example 1 but using the compound (159 mg) obtained in Production Example 34-5-2) and (5-chloropyridin-2-yl)methyl methanesulfonate (153 mg), obtained was methyl 6-[4-[(5-chloropyridin-2-yl)methoxy]-2-oxopyridin-1(2H)-yl]-1-methyl-1H-indole-2-carboxylate (52.4 mg).
2) In the same manner as in Example 65-1) but using the compound (52.4 mg) obtained in Production Example 34-6-1), the entitled compound (47.2 mg) was obtained. Production Example 34-7:

### 6-[4-[(5-Chloropyridin-2-yl)methoxy]-2-oxopyridin-1(2H)-yl]-1-methyl-1H-indole-2-carbaldehyde:

In the same manner as in Production Example 21-1 but using the compound (23.4 mg) obtained in Production Example 34-6-2), the entitled compound (22.7 mg) was obtained. Production Example 35-1:

### Ethyl (2E)-3- {6-[4-[(5-chloropyridin-2-yl)methoxy]-2-oxopyridin-1(2H)-yl]-1-methyl-1H-indol-2-yl}acrylate:

At 0°C, ethyl diethylphosphonoacetate (33.9 µL) was dropwise added to a THF suspension (4 mL) of sodium hydride (4.1 mg). After stirred for 30 minutes, a DMF solution (2 mL) of the compound (22.7 mg) obtained in Production Example 34-7 was dropwise added thereto at 0°C. After stirred for 4.5 hours, the reaction liquid was poured into water, and extracted with ethyl acetate. The organic layer was washed with saturated saline water, and dried with anhydrous sodium sulfate. The solvent was evaporated off under reduced pressure, and the residue was purified by silica gel column chromatography (chloroform:methanol = 30:1) to obtain the entitled compound (26.4 mg).

### Production Example 35-2:

### Ethyl 3- {6-[4-[(5-chloropyridin-2-yl)methoxy]-2-oxopyridin-1(2H)-yl]-1-methyl-1H-indol-2-yl}propanoate:

At 0°C, sodium borohydride (4.4 g) and nickel chloride 6-hydrate (2.7 mg) were successively added to a methanol/THF mixed solution (1:1, 10 mL) of the compound (26.4 mg) obtained in Production Example 35-1. After stirred for 4 hours, the reaction liquid was poured into water, and extracted with chloroform. The organic layer was washed with saturated saline water, and dried with anhydrous sodium sulfate. The solvent was evaporated off under reduced pressure to obtain the entitled compound (25.0 mg) as a crude product.

### Production Example 35-3:

### 4-[(5-Chloropyridin-2-yl)methoxy]-1-[2-(3-hydroxypropyl)-1-methyl-1H-indol-6-yl]pyridin-2(1H)-one:

In the same manner as in Example 65-1) but using the compound (25.0 mg) obtained in Production Example 35-2, the entitled compound (11.3 mg) was obtained.

### Production Example 36-1:

### 4-(Benzyloxy)-1-(2-oxiran-2-ylquinolin-6-yl)pyridin-2(1H)-one:

DMSO (5 mL) was added to sodium hydride (73 mg, 50 to 72 % oily), and stirred at 60°C for 30 minutes. Next, anhydrous THF (5 mL) was added, then with cooling with ice, a DMSO solution (2 mL) of trimethylsulfonium iodide (370 mg) and a DMSO solution (10 ml) of the compound (500 mg) obtained in Production Example 21-1 were successively added thereto, and stirred at 0°C for 10 minutes and then at room temperature for 1.5 hours. Water was added to the reaction liquid, and extracted with ethyl acetate. The organic layer was washed with water and saturated saline water, then dried with anhydrous sodium sulfate. The solvent was evaporated off from the organic layer under reduced pressure, then the residue was purified by silica gel column chromatography (chloroform:ethyl acetate = 4:1 to 2:1) to obtain the entitled compound (260 mg) as a colorless solid.

### Production Example 36-2:

### 4-(Benzyloxy)-1-[2-(2-hydroxyethyl)quinolin-6-yl]pyridin-2(1H)-one:

10 % palladium-carbon (150 mg) was added to a methanol solution (100 mL) of the compound (730 mg) obtained in Production Example 36-1 and ammonium formate (300 mg), and in a hydrogen atmosphere (1 atmospheric pressure), this was stirred at room temperature for 1.5 hours. The reaction liquid was filtered through Celite, the solvent was evaporated off under reduced pressure, and the residue was diluted with chloroform. The organic layer was washed with water, then dried with anhydrous sodium sulfate. The solvent was evaporated off from the organic layer under reduced pressure, then the residue was purified by silica gel column chromatography (chloroform:methanol = 80:1 to 20:1) to obtain the entitled compound (604 mg) as a colorless solid.

### Production Example 36-3:

### 2- {6-[4-(Benzyloxy)-2-oxopyridin-1(2H)-yl]quinolin-2-yl}ethyl methanesulfonate:

With cooling with ice, methanesulfonyl chloride (30 µL) was added to a chloroform solution (10 mL) of the compound (105 mg) obtained in Production Example 36-2 and triethylamine (54 µL), and stirred at the same temperature for 1 hour. Water was added to the reaction liquid, extracted with chloroform, and dried with anhydrous sodium sulfate. The organic layer was evaporated under reduced pressure, then the obtained solid was diluted with diethyl ether, collected by filtration, and dried under reduced pressure to obtain the entitled compound (120 mg) as a colorless solid.

### Production Example 37-1:

### 4-(Benzyloxy)-1- {2-[2-(tetrahydro-2H-pyran-2-yloxy)ethyl]quinolin-6-yl}pyridin-2(1H)-one:

P-toluenesulfonic acid monohydrate (50 mg) was added to a chloroform solution (6 mL) of the compound (110 mg) obtained in Production Example 36-2 and 3,4-dihydro-2H-pyran (0.5 mL), and stirred at room temperature for 5 hours. Aqueous saturated sodium hydrogencarbonate solution was added to the reaction liquid, then extracted with chloroform. The organic layer was dried with anhydrous sodium sulfate, the solvent was evaporated off under reduced pressure, and the residue was purified by silica gel column chromatography (chloroform:methanol = 80:1) to obtain the entitled compound (85 mg) as a colorless solid. Production Example 37-2:

### 4-Hydroxy-1-{2-[2-(tetrahydro-2H-pyran-2-yloxy)ethyl]quinolin-6-yl}pyridin-2(1H)-one:

10 % palladium-carbon (150 mg) was added to a methanol solution (30 mL) of the compound (590 mg) obtained in Example 37-1, and in a hydrogen atmosphere (1 atmospheric pressure), this was stirred at room temperature for 4 hours. The reaction liquid was filtered through Celite, and the solvent was evaporated off under reduced pressure to obtain a crude product of the entitled compound as a colorless solid.

### Production Example 37-3:

### 4-[(4-Fluorobenzyl)oxy]-1-{2-[2-(tetrahydro-2H-pyran-2-yloxy)ethyl]quinolin-6-yl}pyridin-2(1H)-one:

Cesium carbonate (893 mg) and 1-(bromomethyl)-4-fluorobenzene (207 mg) were added to a DMF solution (20 mL) of the crude product obtained in Production Example 37-2, and stirred at 80°C for 1.5 hours. Water was added to the reaction liquid, then extracted with ethyl acetate, and the organic layer was washed with water and saturated saline water, and dried with anhydrous sodium sulfate. The solvent was evaporated off from the organic layer under reduced pressure, and the obtained residue was purified by silica gel column chromatography (chloroform:methanol = 80:1 to 40:1) to obtain the entitled compound (160 mg) as a colorless solid.

### Production Example 37-4:

### 4-[(4-Fluorobenzyl)oxy]-1-[2-(2-hydroxyethyl)quinolin-6-yl]pyridin-2(1H)-one:

p-toluenesulfonic acid monohydrate (20 mg) was added to a methanol solution (10 mL) of the compound (160 mg) obtained in Production Example 37-3, and stirred at 60°C for 2 hours. The solvent was evaporated off from the reaction liquid under reduced pressure, the residue was diluted with chloroform, and washed with aqueous saturated sodium hydrogencarbonate solution. The organic layer was dried with anhydrous sodium sulfate, then the solvent was evaporated off under reduced pressure, the obtained solid was diluted with diisopropyl ether, collected by filtration, and dried under reduced pressure to obtain the entitled compound (101 mg) as a colorless solid.

### Production Example 37-5:

### 2- {6-[4-[(4-Fluorobenzyl)oxy]-2-oxopyridin-1(2H-yl]quinolin-2-yl}ethyl methanesulfonate:

In the same manner as in Production Example 36-3 but using the compound (101 mg) obtained in Production Example 37-4, the entitled compound (74 mg) was obtained as a colorless solid.

### Production Example 38-1:

### 4-[(5-Chloropyridin-2-yl)methoxy]-1-{2-[2-(tetrahydro-2H-pyran-2-yloxy)ethyl]quinolin-6-yl}pyridin-2(1H)-one:

In the same manner as in Production Example 37-3 but using the compound (160 mg) obtained in Production Example 37-2 and (5-chloropyridin-2-yl)methyl methanesulfonate (112 mg), the entitled compound (145 mg) was obtained as a colorless solid.

### Production Example 38-2:

### 4-[(5-Chloropyridin-2-yl)methoxy]-1-[2-(2-hydroxyethyl)quinolin-6-yl]pyridin-2(1H)-one:

In the same manner as in Production Example 37-4 but using the compound (145 mg) obtained in Production Example 38-1, the entitled compound (123 mg) was obtained as a colorless solid.

### Production Example 38-3:

### 2- {6-[4-[(5-Chloropyridin-2-yl)methoxy]-2-oxopyridin-1(2H)-yl]quinolin-2-yl}ethyl methanesulfonate:

In the same manner as in Production Example 36-3 but using the compound (123 mg) obtained in Production Example 38-2, the entitled compound (130 mg) was obtained as a colorless solid.

### Production Example 39-1:

### 2-(Iodomethyl)-2,3-dihydro-1-benzofuran:

Iodine (50.8 g) and 1.0 M tin chloride/dichloromethane solution (100 mL) were added to a dichloromethane solution (50 mL) of 2-allylphenol (26.8 g), and in a nitrogen atmosphere, this was stirred at room temperature for 2 days. The reaction liquid was made to have a pH of 9 by addition of aqueous 2 N sodium hydroxide solution thereto at 0°C, then ethyl acetate and aqueous 10 % sodium thiosulfate solution were added for liquid-liquid separation. The organic layer was washed successively with water and saturated saline water. This was dried with anhydrous sodium sulfate, the solvent was evaporated off under reduced pressure, and the obtained residue was evaporated under reduced pressure to obtain the entitled compound (24.0 g) as a red oily substance.

### Production Example 39-2:

### 5-Iodo-2-(iodomethyl)-2,3-dihydro-1-benzofuran:

Iodine (58.6 g) and mercury oxide (50.0 g) were added to a dichloromethane solution (460 mL) of the compound (24.0 g) obtained in Production Example 39-1, and in a nitrogen atmosphere, this was stirred at room temperature for 5 days. The reaction liquid was filtered, then the filtrate was washed successively with water and saturated saline water. After dried with anhydrous sodium sulfate, the solvent was evaporated off under reduced pressure, and the obtained residue was purified by silica gel column chromatography (hexane:ethyl acetate = 20:1) to obtain the entitled compound (29.5 g) as a red oily substance.

### Production Example 39-3:

### 5-Iodo-2-methyl-1-benzofuran:

Sodium hydroxide (6.1 g) was added to a methanol solution (300 mL) of the compound (29.5 g) obtained in Production Example 39-2, and in a nitrogen atmosphere, this was stirred at 70°C for 8 hours, then stirred at room temperature for 10 hours. The reaction liquid was made to have a pH of 2 by addition of 1 N hydrochloric acid thereto at 0°C, and then extracted with ethyl acetate. The organic layer was washed successively with aqueous saturated sodium hydrogencarbonate solution and saturated saline water, then dried with anhydrous sodium sulfate, and the solvent was evaporated off under reduced pressure. The obtained residue was purified by silica gel column chromatography (hexane) to obtain the entitled compound (18.9 g) as a colorless oily substance.

### Production Example 39-4:

### 1-[(5-Iodo-1-benzofuran-2-yl)methyl]pyrrolidine:

N-chlorosuccinimide (543 mg) was added to a chloroform solution (10 mL) of the compound (1.0 g) obtained in Production Example 39-3, and in a nitrogen atmosphere, this was stirred at room temperature for 23 hours. Benzoyl peroxide (80 mg) was added to the reaction liquid, and stirred at room temperature for 3 days. The reaction liquid was cooled to 0°C, then pyrrolidine (5.0 mL) was added, and stirred at room temperature for 2 hours. The solvent was evaporated off from the reaction liquid under reduced pressure, then ethyl acetate was added to the obtained residue, and washed successively with aqueous saturated sodium hydrogencarbonate solution and saturated saline water. The organic layer was dried with anhydrous sodium sulfate, then the solvent was evaporated off under reduced pressure. The obtained residue was purified by silica gel column chromatography (NH silica gel, hexane:ethyl acetate = 1:0 to 9: 1) to obtain the entitled compound (602 mg) as a colorless solid.

### Production Example 40-1:

### Ethyl 5-iodo-1-benzofuran-2-carboxylate:

Diethyl bromomalonate (2.5 mL), potassium carbonate (2.5 g) and tetrabutylammonium bromide (390 mg) were added to a toluene solution (50 mL) of 2-hydroxy-5-iodobenzaldehyde (3.0 g), and in a nitrogen atmosphere, this was stirred at 140°C for 3 days. Diethyl ether was added to the reaction liquid, and washed successively with water and saturated saline water. The organic layer was dried with anhydrous magnesium sulfate, then the solvent was evaporated off under reduced pressure. The obtained residue was purified by silica gel column chromatography (hexane:ethyl acetate = 20:1) to obtain the entitled compound (3.5 g) as a colorless oily substance.

### Production Example 40-2:

### 5-Iodo-2-[(methoxymethoxy)methyl]-1-benzofuran:

At 0°C, lithiumaluminium hydride (300 mg) was added to a THF solution (30 mL) of the compound (2.0 g) obtained in Production Example 40-1, and in a nitrogen atmosphere, this was stirred at the same temperature for 15 minutes. 2 N hydrochloric acid was added to the reaction liquid, and extracted with ethyl acetate. The organic layer was washed with saturated saline water, then dried with anhydrous sodium sulfate, and the solvent was evaporated off under reduced pressure. The obtained residue was dissolved in chloroform (30 mL), then N,N-diisopropylethylamine (2.8 mL) and chloromethyl methyl ether (526 µL) were added, and stirred in a nitrogen atmosphere at room temperature for 20 hours. N,N-diisopropylethylamine (2.8 mL) and chloromethyl methyl ether (1.1 mL) were added to the reaction liquid, and further stirred for 23 hours. Ethyl acetate was added to the reaction liquid, then washed successively with aqueous saturated sodium hydrogencarbonate solution and saturated saline water. The organic layer was dried with anhydrous sodium sulfate, then the solvent was evaporated off under reduced pressure. The obtained residue was purified by silica gel column chromatography (hexane:ethyl acetate = 20:1 to 9:1) to obtain the entitled compound (1.4 g) as a colorless oily substance.

### Production Example 40-3:

### 4-[(4-Fluorobenzyl)oxy]-1-{2-[(methoxymethoxy)methyl]-1-benzofuran-5-yl}pyridin-2(1H)-one:

In the same manner as in Example 87 but using the compound (1.42 g) obtained in Production Example 40-2 and 4-(fluorobenzyl)oxy-2(1H)-pyridone (979 mg) followed by purification by silica gel column chromatography (hexane:ethyl acetate = 1:1 to 1:3), the entitled compound (920 mg) was obtained as a colorless solid.

### Production Example 40-4:

### 4-[(4-Fluorobenzyl)oxy]-1-[2-(hydroxymethyl)-1-benzofuran-5-yl]pyridin-2(1H)-one:

1 N hydrochloric acid (8.0 mL) was added to a THF solution (30 mL) of the compound (820 mg) obtained in Production Example 40-3, and in a nitrogen atmosphere, this was stirred at room temperature for 3 hours, then at 50°C for 18 hours. 2 N hydrochloric acid (2.0 mL) was added to the reaction liquid, and further stirred for 5 hours. Ethyl acetate was added to the reaction liquid, and washed successively with aqueous saturated sodium hydrogencarbonate solution and saturated saline water. The organic layer was dried with anhydrous sodium sulfate, then the solvent was evaporated off under reduced pressure to obtain the entitled compound (594 mg) as a colorless solid.

### Production Example 40-5:

### 1-[2-(Chloromethyl)-1-benzofuran-5-yl]-4-[(4-fluorobenzyl)oxy]pyridin-2(1H)-one:

Thionyl chloride (162 µL) was added to a THF solution (50 mL) of the compound (540 mg) obtained in Production Example 40-4, and stirred in a nitrogen atmosphere at room temperature for 2 hours. At 0°C, aqueous saturated sodium hydrogencarbonate solution was added to the reaction liquid, then extracted with ethyl acetate. The organic layer was washed with saturated saline water, dried with anhydrous sodium sulfate, and the solvent was evaporated off under reduced pressure to obtain the entitled compound (568 mg) as a colorless solid.

### Production Example 41-1:

### Ethyl (4E)-5-({2-[isopropyl(methyl)amino]-1H-benzimidazol-6-yl}amino)-3-oxopent-4-enoate:

60 % sodium hydride (160 mg) and ethyl (4E)-5-ethoxy-3-oxopent-4-enoate (550 µL) produced according to Chem. Ber. 115, 2766-2782 (1982) were added to an ethanol solution (10 mL) ofN²-isopropyl-N²-methyl-1H-benzimidazole-2,6-diamine dihydrochloride (554 mg) produced according to JP-A 2002-220905, and stirred overnight at room temperature. The reaction liquid was concentrated under reduced pressure, then ethyl acetate was added to the obtained residue, and washed with water and saturated saline water. The organic layer was dried with anhydrous magnesium sulfate, and the solvent was evaporated off under reduced pressure. The obtained residue was purified by silica gel column chromatography (chloroform:methanol = 100:1 to 50:1) to obtain the entitled compound (397 mg).

### Production Example 41-2:

### 4-Hydroxy-1-{2-[isopropyl(methyl)amino]-1H-benzimidazol-6-yl}pyridin-2(1H)-one:

A toluene solution (10 mL) of the compound (245 mg) obtained in Production Example 41-1 was heated overnight under reflux, and then cooled to room temperature. The precipitated crystal was collected by filtration to obtain the entitled compound (165 mg).

### Production Example 42-1:

### Methyl 2-methylquinoline-6-carboxylate 1-oxide:

In the same manner as in Production Example 3-3-1) but using methyl 2-methylquinoline-6-carboxylate (500 mg) produced according to J. Heterocyclic. Chem., 26, 929 (1989), the entitled compound (312 mg) was obtained.

### Production Example 42-2:

### Methyl 2-({[(4-methylphenyl)sulfonyl]oxy}methyl)quinoline-6-carboxylate:

In a nitrogen atmosphere, the compound (463.3 mg) obtained in Production Example 42-1 was dissolved in acetonitrile (20 mL), and with cooling with ice, potassium carbonate (443.5 mg) and p-toluenesulfonyl chloride (485.6 mg) were added and stirred for 3 hours. After the reaction, aqueous saturated sodium hydrogencarbonate solution was added to the reaction liquid, extracted with ethyl acetate, and the organic layer was washed successively with water and saturated saline water. The organic layer was dried with magnesium sulfate, the solvent was evaporated off under reduced pressure, and the obtained residue was purified by silica gel column chromatography (chloroform:methanol = 10:0 to 19:1) to obtain the entitled compound (578.1 mg) as a yellow solid.

### Production Example 42-3:

### Methyl 2-(pyrrolidin-1-ylmethyl)quinoline-6-carboxylate:

The compound (578.1 mg) obtained in Production Example 42-2 was dissolved in THF (6 mL), and pyrrolidine (198 µL) and potassium carbonate (323.8 mg) were added, and stirred at 55°C for 2 hours. After the reaction, water was added to the reaction liquid, extracted with ethyl acetate, the organic layer was washed with saturated saline water. The organic layer was dried with anhydrous magnesium sulfate, then the solvent was evaporated off under reduced pressure, and the obtained residue was purified by silica gel column chromatography (chloroform:methanol = 10:0 to 19:1) to obtain the entitled compound (286.2 mg) as a yellow solid.

### Production Example 42-4:

### [2-(Pyrrolidin-1-ylmethyl)quinolin-6-yl]methanol:

In a nitrogen atmosphere, lithiumaluminium hydride (48.4 mg) was suspended and stirred in THF (5 mL), and with cooling at -78°C, a THF solution (5 mL) of the compound (286.2 mg) obtained in Production Example 42-3 was dropwise added over 20 minutes, and stirred for 3 hours. After the reaction, sodium sulfate 10-hydrate was added to the reaction liquid, stirred for 6 hours, and filtered through Celite. The solvent was evaporated off from the filtrate under reduced pressure, and the obtained residue was purified by silica gel column chromatography (chloroform:methanol = 10:0 to 9:1) to obtain the entitled compound (227.0 mg) as a yellow solid.

### Production Example 43-1:

### 2-Bromo-6-methyl-1,5-naphthyridine:

A mixture of 6-methyl-1,5-naphthyridin-2(1H)-one (309 mg), diphosphorus pentoxide (410 mg), tetrabutylammonium bromide (930 mg) and toluene (6 mL) was stirred overnight under reflux. The reaction liquid was cooled to room temperature, then water and ethyl acetate were added. The organic layer was washed with water and saturated saline water, dried with anhydrous magnesium sulfate. The solvent was concentrated under reduced pressure, and the obtained residue was purified by silica gel column chromatography (hexane:ethyl acetate = 4:1 to 1:1) to obtain the entitled compound (225 mg).

### Production Example 43-2:

### 4-[(4-Fluorobenzyl)oxy]-1-(6-methyl-1,5-naphthyridin-2-yl)pyridin-2(1H)-one:

In the same manner as in Production Example 1-4 but using the compound (225 mg) obtained in Production Example 43-1 and 4-(fluorobenzyl)oxy-2(1H)-pyridone (221 mg), the entitled compound (116 mg) was obtained as a pale yellow solid.

### Production Example 43-3:

### 6-[4-[(4-Fluorobenzyl)oxy]-2-oxopyridin-1(2H)-yl]-1,5-naphthyridine-2-carbaldehyde:

In the same manner as in Production Example 13-1 but using the compound (116 mg) obtained in Production Example 43-2, the entitled compound (80 mg) was obtained as a white solid.

### Production Example 44-1:

### 1- {2-[(6-Bromo-2-naphthyl)oxy]ethyl}pyrrolidine:

1-(2-Chloroethyl)pyrrolidine hydrochloride (1.25 g) and potassium carbonate (3.4 g) were added to a DMF solution (20 mL) of 6-bromo-2-naphthol (1.1 g), and stirred overnight at 75°C. Water was added to the reaction mixture, and extracted with chloroform. The chloroform layer was dried with anhydrous magnesium sulfate, and the solvent was evaporated off under reduced pressure. The obtained residue was purified by silica gel column chromatography (chloroform:methanol = 40:1) to obtain the entitled compound (1.4 g) as an orange oily substance.

### Production Example 45-1:

### 6-Iodo-2-{[(3R)-1-isopropylpyrrolidin-3-yl]oxy}quinoline:

In the same manner as in Production Example 4-1 but using the compound (150 mg) obtained in Production Example 1-2 and (3R)-1-isopropylpyrrolidin-3-ol (67.1 mg), the entitled compound (33 mg) was obtained.

### Production Example 46-1:

### 4-Bromo-N-formylphenylalanine ethyl ester:

At room temperature, ammonium formate (5.5 g) was added to an acetonitrile solution (150 mL) of (±)-(4-bromophenyl)alanine ethyl ester hydrochloride (12.5 g). The reaction liquid was stirred with heating under reflux for 12 hours, then the solvent was evaporated off under reduced pressure. Water was added to the residue, and extracted with ethyl acetate. The organic layer was washed with saturated saline water, and dried with anhydrous sodium sulfate. The solvent was evaporated off under reduced pressure, and the residue was purified by silica gel column chromatography (ethyl acetate:hexane = 2:1) to obtain the entitled compound (11.9 g).

### Production Example 46-2:

### Ethyl 7-bromoisoquinoline-3-carboxylate:

At room temperature, oxalyl chloride (1.9 mL) was added to a dichloromethane solution (80 mL) of the compound (6.0 g) obtained in Production Example 46-1. After stirred for 30 minutes, the reaction liquid was cooled to 0°C, and ferric chloride (3.9 g) was added. After stirred at room temperature for 14 hours, the reaction liquid was poured into 1 N hydrochloric acid, and extracted with chloroform. The organic layer was dried with anhydrous sodium sulfate, then the solvent was evaporated off under reduced pressure. The residue was dissolved in ethanol (60 mL), then concentrated sulfuric acid (3 mL) was added and stirred with heating under reflux for 7 hours. The reaction liquid was poured into aqueous saturated sodium hydrogencarbonate solution, and extracted with ethyl acetate. The organic layer was washed with saturated saline water, and dried with anhydrous sodium sulfate. The solvent was evaporated off under reduced pressure, then the obtained residue was purified by silica gel column chromatography (methanol:chloroform = 1:500 to 1:250) to obtain the entitled compound (431 mg).

### Production Example 46-3:

### Ethyl 7-[4-[(4-fluorobenzyl)oxy]-2-oxopyridin-1 (2H)-yl]isoquinoline-3-carboxylate:

At room temperature, 4-(fluorobenzyl)oxy-2(1H)-pyridone (337 mg), potassium carbonate (639 mg) and copper(I) iodide (322 mg) were successively added to a DMF solution (15 mL) of the compound (431 mg) obtained in Production Example 46-2. The reaction liquid was stirred at 100°C for 12 hours, then poured into diluted ammonia water. The formed precipitate was collected by filtration, the solid was washed with water, then dissolved in chloroform. The organic layer was washed with saturated saline water, then dried with anhydrous sodium sulfate. The solvent was evaporated off under reduced pressure, then the residue was dissolved in ethanol (60 mL), and concentrated sulfuric acid (3 mL) was added and stirred with heating under reflux for 12 hours. The reaction liquid was poured into aqueous saturated sodium hydrogencarbonate solution, and extracted with ethyl acetate. The organic layer was washed with saturated saline water, then dried with anhydrous sodium sulfate. The solvent was evaporated off under reduced pressure, and the residue was purified by silica gel column chromatography (methanol:chloroform = 1:200) to obtain the entitled compound (109 mg).

### Production Example 46-4:

### 4-[(4-Fluorobenzyl)oxy]-1-[3-(hydroxymethyl)isoquinolin-7-yl]pyridin-2(1H)-one:

Calcium chloride (128 mg) was added to an ethanol/THF mixed solvent (1;1, 20 mL) of the compound (109 mg) obtained in Production Example 46-3, and then sodium borohydride (26.8 mg) was added at 0°C. The reaction liquid was stirred at room temperature for 2 hours, then poured into water. The reaction liquid was extracted with chloroform, the organic layer was washed with saturated saline water, and dried with anhydrous sodium sulfate. The solvent was evaporated off under reduced pressure to obtain the entitled compound (103 mg) as a crude product.

### Production Example 47-1:

### 7-Bromo-2-chloro-3-[(tetrahydro-2H-pyran-2-yloxy)methyl]quinoline:

With cooling with ice, sodium borohydride (210 mg) was added to a THF/methanol mixed solution (1:1, 35 mL) of 7-bromo-2-chloroquinoline-3-carbaldehyde (2.5 g) produced according to SYNTHESIS, 2004, 9, p. 1419, and stirred for 1 hour. Water was added to the reaction liquid, and extracted with ethyl acetate, then the organic layer was washed with water and saturated saline water, then dried with anhydrous sodium sulfate. The solvent was evaporated off from the organic layer under reduced pressure to obtain a crude product of an alcohol compound. P-toluenesulfonic acid pyridine salt (200 mg) was added to a chloroform solution (30 mL) of the obtained alcohol compound and 3,4-dihydro-2H-pyran (0.5 mL), and stirred at room temperature for 15 hours. Aqueous saturated sodium hydrogencarbonate solution was added to the reaction liquid, then extracted with chloroform. The organic layer was dried with anhydrous sodium sulfate, then the solvent was evaporated off under reduced pressure, and the residue was purified by silica gel column chromatography (hexane/ethyl acetate = 30/1) to obtain the entitled compound (1.5 g) as a yellow oily substance.

### Production Example 47-2:

### 1- {2-Chloro-3-[(tetrahydro-2H-pyran-2-yloxy)methyl]quinolin-7-yl}-4-[(4-fluorobenzyl)oxy]pyridin-2(11H)-one:

In the same manner as in Production Example 1-4 but using the compound (1.5 g) obtained in Production Example 47-1 and 4-(fluorobenzyl)oxy-2(1H)-pyridone (700 mg), the entitled compound (120 mg) was obtained as a yellow solid.

### Production Example 47-3:

### 4-[(4-Fluorobenzyl)oxy]-1-{3-[(tetrahydro-2H-pyran-2-yloxy)methyl]quinolin-7-yl}pyridin-2(1H)-one:

Tetrakis(triphenylphosphino)palladium(0) (50 mg), formic acid (100 µL) and triethylamine (200 µL) were added to a DMF solution (5 mL) of the compound (120 mg) obtained in Production Example 47-2, and stirred at 110°C for 1 hour. Water was added to the reaction liquid, extracted with ethyl acetate, and the organic layer was washed with water and saturated saline water, then dried with anhydrous sodium sulfate. The solvent was evaporated off from the organic layer under reduced pressure, and the residue was purified by silica gel column chromatography (chloroform:ethyl acetate = 2:1) to obtain the entitled compound (30 mg) as a yellow solid.

### Example 1:

### 4-[(5-Chloropyridin-2-yl)methoxy]-1-(2-pyrrolidin-1-ylquinolin-6-yl)pyridin-2(1H)-one:

A DMF solution (3 mL) of the compound (20 mg) obtained in Production Example 1-5 and potassium carbonate (8 mg) was heated at 80°C, and at the same temperature, a DMF solution (1 mL) of (5-chloropyridin-2-yl)methyl methanesulfonate (10.6 mg) was dropwise added over 1 hour. After the addition, this was further stirred at the same temperature for 10 minutes. Water was added to the reaction liquid, and extracted with a mixed solvent of chloroform/methanol (10:1). The organic layer was dried with anhydrous magnesium sulfate, then the solvent was evaporated off under reduced pressure. The obtained residue was purified by silica gel column chromatography (chloroform:methanol = 10:1) to obtain the entitled compound (15.8 mg) as a pale yellow powder.
¹H-NMR (400 MHz, CDCl₃, δ ppm): 2.04-2.06 (4H, brm), 3.60-3.62 (4H, brm), 5.17 (2H, s), 6.05 (1H, d, J=2.3 Hz), 6.10 (1H, dd, J=7.8, 2.7 Hz), 6.75 (1H, d, J=9.0 Hz), 7.34 (1H, d, J=7.8 Hz), 7.45-7.46 (2H, brm), 7.56 (1H, d, J=2.7 Hz), 7.74-7.76 (2H, brm), 7.82 (1H, d, J=9.0 Hz), 8.59 (1H, m).
ESI-MS Found: m/z 433[M+H]⁺

### Example 2:

### 4-[(4-chlorobenzyl)oxy]-1-{2-[isopropyl(methyl)amino]quinolin-6-yl]pyridin-2(1H)-one:

In the same manner as in Example 1 but using the compound (23 mg) obtained in Production Example 2-1 and 1-chloro-4-(chloromethyl)benzene (14.4 mg), the entitled compound (11.6 mg) was obtained as a yellow powder.
¹H-NMR (400 MHz, CDCl₃, δ ppm): 1.24 (6H, d, J=6.7 Hz), 3.01 (3H, s), 5.01-5.03 (3H, m), 6.04-6.05 (2H, m), 6.92 (1H, d, J=9.2 Hz), 7.31-7.32 (1H, m), 7.35-7.41 (4H, m), 7.46 (1H, dd, J=8.9, 2.4 Hz), 7.54 (1H, d, J=2.3 Hz), 7.72 (1H, d, J=8.6 Hz), 7.83 (1H, d, J=9.0 Hz).
ESI-MS Found: m/z 434[M+H]⁺

### Example 3:

### 4-[(4-Chlorobenzyl)oxy]-1-(2- {[isopropyl(methyl)amino]methyl}quinolin-6-yl)pyridin-2(1H)-one:

Triethylamine (518 µL) and methanesulfonyl chloride (131 mg) were added to a THF solution (40 mL) of the compound (150 mg) obtained in Production Example 3-3-3), and stirred at room temperature for 5 hours. The reaction liquid was dropwise added to a THF solution (15 mL) of N-methylisopropylamine (10 mL), and after the addition, this was stirred overnight at room temperature. The solvent was evaporated off from the reaction liquid under reduced pressure, and water was added to the obtained residue, and extracted with a mixed solvent of chloroform/methanol (10:1). The organic layer was dried with anhydrous magnesium sulfate, then the solvent was evaporated off under reduced pressure. The obtained residue was purified by reversed-phase HPLC (YMC-Pack ODS-AQ, acetonitrile (containing 0.1 % TFA):water (containing 0.1 % TFA) (10:90 to 90:10) and then desalted to obtain the entitled compound (93.9 mg) as a white powder.
¹H-NMR (400 MHz, CDCl₃, δ ppm): 1.14 (6H, d, J=5.9 Hz), 2.25 (3H, s), 2.98-3.00 (1H, m), 3.90 (2H, s), 5.03 (2H, s), 6.06 (1H, d, J=2.7 Hz), 6.09 (1H, dd, J=7.6, 2.5 Hz), 7.32-7.40 (5H, m), 7.68 (1H, dd, J=9.0, 2.0 Hz), 7.74 (1H, d, J=8.2 Hz), 7.78 (1H, d, J=2.0 Hz), 8.13 (2H, d, J=9.0 Hz).
ESI-MS Found: m/z 448[M+H]⁺

### Example 4:

### 4-[(4-Chlorobenzyl)oxy]-1-{2-[(dimethylamino)methyl]quinolin-6-yl}pyridin-2(1H)-one:

In the same manner as in Example 3 but using the compound (30 mg) obtained in Production Example 3-3-3) and a THF solution (8 mL) of 2 M methylamine, the entitled compound (20.3 mg) was obtained as a pale yellow powder.
¹H-NMR (400 MHz, CDCl₃, δ ppm): 2.36 (6H, s), 3.81 (2H, s), 5.04 (2H, s), 6.08-6.10 (2H, m), 7.34-7.41 (5H, m), 7.67-7.70 (2H, m), 7.80 (1H, d, J=2.0 Hz), 8.15 (1H, d, J=9.4 Hz), 8.18 (1H, d, J=9.8 Hz).
ESI-MS Found: m/z 420[M+H]⁺

### Example 5:

### 4-[(4-Chlorobenzyl)oxy]-1-(2-{[(3R)-3-fluoropyrrolidin-1-yl]methyl}quinolin-6-yl)pyridin-2(1H)-one:

In the same manner as in Example 3 but using the compound (10 mg) obtained in Production Example 3-3-3) and 3-(R)-fluoropyrrolidine (600 mg) followed by purification by silica gel column chromatography (chloroform:methanol = 10:1), the entitled compound (4.4 mg) was obtained as an orange powder.
¹H-NMR (400 MHz, CDCl₃, δ ppm): 2.06-2.26 (2H, m), 2.57-2.59 (1H, m), 2.86-2.97 (3H, m), 4.03 (2H, dd, J=18.6, 13.9 Hz), 5.04 (2H, brs), 5.15-5.28 (1H, m), 6.07 (1H, d, J=2.7 Hz), 6.11 (1H, dd, J=7.4, 2.7 Hz), 7.35-7.40 (5H, m), 7.67-7.71 (2H, m), 7.80 (1H, d, J=2.3 Hz), 8.15 (1H, d, J=8.6 Hz), 8.16 (1H, d, J=9.0 Hz).
ESI-MS Found: m/z 464[M+H]⁺

### Example 6:

### 4-(Benzyloxy)-1-[2-(2-pyrrolidin-1-ylethoxy)quinolin-6-yl]pyridin-2(1H)-one:

In the same manner as in Production Example 1-4 but using the compound (27.4 g) obtained in Production Example 4-1 and 4-benzyloxy-2(1H)-pyridone (30 g), the entitled compound (16.1 g) was obtained as a pale yellow powder.
¹H-NMR (400 MHz, CDCl₃, δ ppm): 1.81-1.84 (4H, m), 2.64-2.67 (4H, m), 2.95 (2H, t, J=5.9 Hz), 4.64 (2H, t, J=5.9 Hz), 5.07 (2H, s), 6.08-6.10 (2H, m), 6.99 (1H, d, J=8.8 Hz), 7.30-7.32 (1H, m), 7.38-7.42 (5H, m), 7.60 (1H, dd, J=8.7, 2.4 Hz), 7.70 (1H, d, J=2.3 Hz), 7.90 (1H, d, J=8.8 Hz), 7.97 (1H, d, J=9.0 Hz).
ESI-MS Found: m/z 442[M+H]⁺

### Example 7:

### 4-[(4-Fluorobenzyl)oxy]-1-[2-(2-pyrrolidin-1-ylethoxy)quinolin-6-yl]pyridin-2(1H)-one:

In the same manner as in Production Example 1-4 but using the compound (360 mg) obtained in Production Example 4-1 and 4-(fluorobenzyl)oxy-2(1H)-pyridone (329 mg), the entitled compound (193 mg) was obtained as an orange powder.
¹H-NMR (400 MHz, CDCl₃, δ ppm): 1.84-1.86 (4H, m), 2.70-2.72 (4H, m), 2.98-3.01 (2H, m), 4.67 (2H, t, J=5.8 Hz), 5.02 (2H, s), 6.07-6.08 (2H, m), 7.00 (1H, d, J=8.8 Hz), 7.10-7.12 (2H, m), 7.31-7.33 (1H, m), 7.40-7.42 (2H, m), 7.60 (1H, dd, J=8.8, 2.3 Hz), 7.70 (1H, d, J=2.5 Hz), 7.90 (1H, d, J=9.0 Hz), 7.98 (1H, d, J=8.6 Hz).
ESI-MS Found: m/z 460[M+H]⁺

### Example 8:

### 4-[(5-Chloropyridin-2-yl)methoxy]-1-[2-(2-pyrrolidin-1-ylethoxy)quinolin-6-yl]pyridin-2(1H)-one:

In the same manner as in Example 1 but using the compound (57.4 mg) obtained in Production Example 5-1 and (5-chloropyridin-2-yl)methyl methanesulfonate (36.2 mg), the entitled compound (18.6 mg) was obtained as a yellow powder.
¹H-NMR (400 MHz, CDCl₃, δ ppm): 1.84-1.87 (4H, m), 2.71-2.73 (4H, m), 3.01 (2H, t, J=5.9 Hz), 4.67 (2H, t, J=5.7 Hz), 5.18 (2H, s), 6.06 (1H, d, J=2.3 Hz), 6.14 (1H, dd, J=7.6, 2.5 Hz), 7.00 (1H, d, J=9.0 Hz), 7.34 (1H, d, J=7.4 Hz), 7.45 (1H, d, J=8.6 Hz), 7.59 (1H, dd, J=9.0, 2.3 Hz), 7.70 (1H, d, J=2.3 Hz), 7.74 (1H, dd, J=8.4, 2.5 Hz), 7.90 (1H, d, J=8.6 Hz), 7.98 (1H, d, J=9.0 Hz), 8.59 (1H, d, J=2.0 Hz).
ESI-MS Found: m/z 477[M+H]⁺

### Example 9:

### 4-(Pyridin-2-ylmethoxy)-1-[2-(2-pyrrolidin-1-ylethoxy)quinolin-6-yl]pyridin-2(1H)-one:

In the same manner as in Example 1 but using the compound (100 mg) obtained in Production Example 5-1 and 2-(chloromethyl)pyridine hydrochloride (46.7 mg) followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (42.6 mg) was obtained as a yellow white powder.
¹H-NMR (400 MHz, CDCl₃, δ ppm): 1.82-1.83 (4H, m), 2.65-2.67 (4H, m), 2.96 (2H, t, J=5.9 Hz), 4.64 (2H, t, J=5.9 Hz), 5.20 (2H, s), 6.08 (1H, d, J=2.7 Hz), 6.15 (1H, dd, J=7.4, 2.7 Hz), 6.99 (1H, d, J=8.6 Hz), 7.28-7.29 (1H, m), 7.33 (1H, d, J=7.8 Hz), 7.48 (1H, d, J=7.8 Hz), 7.59 (1H, dd, J=8.8, 2.5 Hz), 7.70 (1H, d, J=2.3 Hz), 7.76 (1H, m), 7.90 (1H, d, J=9.0 Hz), 7.97 (1H, d, J=9.0 Hz), 8.63-8.64 (1H, m).
ESI-MS Found: m/z 443[M+H]⁺

### Example 10:

### 4-[(5-Fluoropyridin-2-yl)methoxy]-1-[2-(2-pyrrolidin-1-ylethoxy)quinolin-6-yl]pyridin-2(1H)-one:

In the same manner as in Example 1 but using the compound (100 mg) obtained in Production Example 5-1 and (5-fluoropyridin-2-yl)methyl methanesulfonate (58.4 mg) followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (60.0 mg) was obtained as a white powder.
¹H-NMR (400 MHz, CDCl₃, δ ppm): 1.81-1.85 (4H, m), 2.65-2.67 (4H, m), 2.96 (2H, t, J=5.9 Hz), 4.64 (2H, t, J=5.9 Hz), 5.18 (2H, s), 6.08 (1H, d, J=2.7 Hz), 6.13 (1H, dd, J=7.6, 2.5 Hz), 6.99 (1H, d, J=9.0 Hz), 7.34 (1H, d, J=7.8 Hz), 7.47-7.50 (2H, m), 7.59 (1H, dd, J=9.0, 2.3 Hz), 7.70 (1H, d, J=2.3 Hz), 7.90 (1H, d, J=9.0 Hz), 7.97 (1H, d, J=9.0 Hz), 8.49 (1H, d, J=2.3 Hz). ESI-MS Found: m/z 461[M+H]⁺

### Example 11:

### 4-[(5-Chloropyridin-2-yl)methoxy]-1-{2-[2-(dimethylamino)ethoxy]quinolin-6-yl}pyridin-2(1H)-one:

In the same manner as in Example 1 but using the compound (50 mg) obtained in Production Example 6-3 and (5-chloropyridin-2-yl)methyl methanesulfonate (34.9 mg) followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (14.4 mg) was obtained as a pale yellow powder.
¹H-NMR (400 MHz, CDCl₃, δ ppm): 2.43 (6H, s), 2.86 (2H, t, J=5.5 Hz), 4.63 (2H, t, J=5.5 Hz), 5.17 (2H, s), 6.06 (1H, d, J=2.3 Hz), 6.14 (1H, dd, J=7.4, 2.7 Hz), 7.00 (1H, d, J=8.6 Hz), 7.34 (1H, d, J=7.8 Hz), 7.45 (1H, d, J=8.6 Hz), 7.60 (1H, dd, J=9.0, 2.3 Hz), 7.70 (1H, d, J=2.3 Hz), 7.74 (1H, dd, J=8.6, 2.3 Hz), 7.90 (1H, d, J=9.0 Hz), 7.98 (1H, d, J=9.0 Hz), 8.59 (1H, d, J=2.0 Hz).
ESI-MS Found: m/z 451[M+H]⁺

### Example 12:

### 4-[(4-Chlorobenzyl)oxy]-1-{2-[2-(dimethylamino)ethoxy]quinolin-6-yl}pyridin-2(1H)-one:

In the same manner as in Example 1 but using the compound (37 mg) obtained in Production Example 7-3 and 1-chloro-4-(chloromethyl)benzene (14.7 mg), the entitled compound (1.6 mg) was obtained as a yellow powder.
¹H-NMR (400 MHz, CDCl₃, δ ppm): 2.38 (6H, s), 2.80 (2H, t, J=5.5 Hz), 4.62 (2H, t, J=5.7 Hz), 5.05 (2H, s), 6.07-6.11 (2H, m), 7.01-7.02 (1H, m), 7.35-7.40 (5H, m), 7.61 (1H, dd, J=8.6, 2.3 Hz), 7.71 (1H, d, J=2.3 Hz), 7.92 (1H, d, J=9.0 Hz), 7.99 (1H, d, J=8.6 Hz).
ESI-MS Found: m/z 450[M+H]⁺

### Example 13:

### 4-(Benzyloxy)-1-{2-[2-(methylamino)ethoxy]quinolin-6-yl}pyridin-2(1H)-one:

In the same manner as in Example 3 but using the compound (25 mg) obtained in Production Example 8-2 and a THF solution (5 mL) of 2 M methylamine, the entitled compound (13.5 mg) was obtained as a yellow powder.
¹H-NMR (400 MHz, CDCl₃, δ ppm): 3.12 (3H, s), 4.06 (2H, t, J=4.6 Hz), 4.46 (2H, t, J=4.6 Hz), 5.06 (2H, s), 6.07-6.10 (2H, m), 6.84 (1H, d, J=9.8 Hz), 7.21-7.24 (2H, m), 7.40-7.45 (8H, m). ESI-MS Found: m/z 402[M+H]⁺

### Example 14:

### 4-(Benzyloxy)-1-{2-[2-(ethylamino)ethoxy]quinolin-6-yl}pyridin-2(1H)-one:

In the same manner as in Example 3 but using the compound (25 mg) obtained in Production Example 8-2 and a THF solution (5 mL) of 2 M ethylamine, followed by purification by silica gel column chromatography (NH silica gel, ethyl acetate), the entitled compound (15.2 mg) was obtained.
¹H-NMR (400 MHz, CDCl₃, δ ppm): 1.29 (3H, t, J=7.2 Hz), 3.35 (2H, q, J=7.3 Hz), 4.06 (2H, t, J=4.3 Hz), 4.42 (2H, t, J=4.4 Hz), 5.05 (2H, s), 6.07-6.09 (2H, m), 6.78 (1H, d, J=9.8 Hz), 7.11 (1H, d, J=8.6 Hz), 7.23-7.25 (1H, m), 7.31 (1H, d, J=9.8 Hz), 7.38-7.42 (7H, m).
ESI-MS Found: m/z 416[M+H]⁺

### Example 15:

### 4-(Benzyloxy)-1-{2-[2-(propylamino)ethoxy]quinolin-6-yl}pyridin-2(1H)-one:

In the same manner as in Example 3 but using the compound (25 mg) obtained in Production Example 8-2 and n-propylamine (1 mL) followed by purification by silica gel column chromatography (NH silica gel, ethyl acetate), the entitled compound (17.2 mg) was obtained. ¹H-NMR (400 MHz, CDCl₃, δ ppm): 1.01 (3H, t, J=7.3 Hz), 1.68-1.72 (2H, m), 3.27 (2H, t, J=7.0 Hz), 4.07 (2H, t, J=4.4 Hz), 4.42 (2H, d, J=4.5 Hz), 5.05 (2H, s), 6.07-6.09 (2H, m), 6.79 (1H, d, J=10.0 Hz), 7.11 (1H, d, J=8.6 Hz), 7.23-7.25 (1H, m), 7.31 (1H, d, J=10.0 Hz), 7.37-7.43 (7H, m).
ESI-MS Found: m/z 430[M+H]⁺

### Example 16:

### 4-(Benzyloxy)-1-{2-[2-(isopropylamino)ethoxy]quinolin-6-yl}pyridin-2(1H)-one:

In the same manner as in Example 3 but using the compound (25 mg) obtained in Production Example 8-2 and isopropylamine (1 mL) followed by purification by silica gel column chromatography (NH silica gel, ethyl acetate), the entitled compound (20.1 mg) was obtained. ¹H-NMR (400 MHz, CDCl₃, δ ppm): 1.21 (6H, d, J=6.3 Hz), 3.74-3.76 (1H, m), 4.06 (2H, t, J=4.3 Hz), 4.39 (2H, t, J=4.3 Hz), 5.05 (2H, s), 6.07-6.08 (2H, m), 6.79 (1H, d, J=10.0 Hz), 7.08 (1H, d, J=8.4 Hz), 7.23-7.24 (1H, m), 7.27-7.29 (1H, m), 7.39-7.42 (7H, m).
ESI-MS Found: m/z 430[M+H]⁺

### Example 17:

### 4-[(5-Chloropyridin-2-yl)methoxy]-1-{2-[2-(diethylamino)ethoxy]quinolin-6-yl}pyridin-2(1H)-one:

In the same manner as in Example 1 but using the compound (37.9 mg) obtained in Production Example 9-3 and (5-chloropyridin-2-yl)methyl methanesulfonate (19.0 mg), the entitled compound (12.9 mg) was obtained as a pale yellow powder.
¹H-NMR (400 MHz, CDCl₃, δ ppm): 1.11 (6H, t, J=7.2 Hz), 2.72-2.77 (6H, m), 4.43-4.45 (2H, m), 5.17 (2H, s), 6.05 (1H, d, J=2.7 Hz), 6.14 (1H, dd, J=7.4, 2.7 Hz), 6.74 (1H, d, J=9.4 Hz), 7.29 (1H, d, J=7.4 Hz), 7.44 (1H, d, J=8.6 Hz), 7.57 (3H, s), 7.66 (1H, d, J=9.4 Hz), 7.74 (1H, dd, J=8.2, 2.3 Hz), 8.59 (1H, d, J=2.3 Hz).
ESI-MS Found: m/z 479[M+H]⁺

### Example 18:

### 4-[(E)-2-(5-chloropyridin-2-yl)vinyl]-1-[2-(2-pyrrolidin-2-ylethoxy)quinolin-6-yl]pyridin-2(1H)-one:

In the same manner as in Production Example 1-4 but using the compound (33 mg) obtained in Production Example 4-1 and 4-[(E)-2-(5-chloropyridin-2-yl)vinyl]pyridin-2(1H)-one (31 mg) followed by purification by silica gel column chromatography (chloroform:methanol = 40:1 to 10:1)), the entitled compound (4.2 mg) was obtained.
¹H-NMR (400 MHz, CDCl₃, δ ppm): 1.73-2.00 (4H, m), 2.73-3.19 (6H, m), 4.68-4.76 (2H, m), 6.50-6.52 (1H, m), 6.73-6.73 (1H, m), 6.96-6.98 (1H, m), 7.16-7.28 (2H, m), 7.35-7.45 (2H, m), 7.58-7.73 (3H, m), 7.86-7.99 (2H, m), 8.53-8.55 (1H, m).
ESI-MS Found: m/z 473[M+H]⁺

### Example 19:

### 4-(Benzyloxy)-1-[2-(3-pyrrolidin-1-ylpropoxy)quinolin-6-yl]pyridin-2(1H)-one:

In the same manner as in Production Example 1-4 but using the compound (400 mg) obtained in Production Example 10-2 and 4-benzyloxy-2(1H)-pyridone (421 mg), the entitled compound (230 mg) was obtained as a yellow powder.
¹H-NMR (400 MHz, CDCl₃, δ ppm): 1.79-1.85 (4H, m), 2.09-2.13 (2H, m), 2.59-2.65 (4H, m), 2.70-2.75 (2H, m), 4.55 (2H, t, J=6.6 Hz), 5.07 (2H, s), 6.06-6.10 (2H, m), 6.93-6.94 (2H, m), 7.31-7.44 (5H, m), 7.60 (1H, dd, J=8.9, 2.4 Hz), 7.70 (1H, d, J=2.3 Hz), 7.90 (1H, d, J=8.8 Hz), 7.98 (1H, d, J=8.6 Hz).
ESI-MS Found: m/z 456[M+H]⁺

### Example 20:

### 4-[(5-Chloropyridin-2-yl)methoxy]-1-[2-(3-pyrrolidin-1-ylpropoxy)quinolin-6-yl]pyridin-2(1H)-one:

In the same manner as in Example 1 but using the compound (165 mg) obtained in Production Example 11-1 and (5-chloropyridin-2-yl)methyl methanesulfonate (100 mg) followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (60.4 mg) was obtained as a white powder.
¹H-NMR (400 MHz, CDCl₃, δ ppm): 1.81-1.84 (4H, m), 2.09-2.10 (2H, m), 2.59-2.62 (4H, m), 2.69-2.71 (2H, m), 4.54 (2H, t, J=6.5 Hz), 5.17 (2H, s), 6.06 (1H, d, J=2.7 Hz), 6.13 (1H, dd, J=7.6, 2.7 Hz), 6.93 (1H, d, J=8.8 Hz), 7.34 (1H, d, J=7.6 Hz), 7.45 (1H, d, J=8.4 Hz), 7.59 (1H, dd, J=8.9, 2.4 Hz), 7.69-7.70 (1H, m), 7.74 (1H, dd, J=8.4, 2.5 Hz), 7.89 (1H, d, J=9.0 Hz), 7.97 (1H, d, J=9.2 Hz), 8.59 (1H, d, J=2.3 Hz).
ESI-MS Found: m/z 491[M+H]⁺

### Example 21:

### 4-[(4-Fluorobenzyl)oxy]-1-(2- {[isopropyl(methyl)amino]methyl}quinolin-6-yl)pyridin-2(1H)-one:

A THF solution (1 mL) of the compound (15 mg) obtained in Production Example 12-2-2) was dropwise added to a THF solution (3 mL) ofN-methylisopropylamine (1.5 mL), and after the addition, this was stirred overnight at room temperature. The solvent was evaporated off from the reaction liquid under reduced pressure, and water was added to the obtained residue, and extracted with a mixed solvent of chloroform/methanol (10:1). The organic layer was dried with anhydrous magnesium sulfate, then the solvent was evaporated off under reduced pressure. The obtained residue was purified by silica gel column chromatography (chloroform:methanol = 10:1) to obtain the entitled compound (5.8 mg) as a yellow solid.
¹H-NMR (400 MHz, CDCl₃, δ ppm): 1.13-1.17 (6H, m), 2.22-2.24 (3H, m), 2.90-3.11 (1H, m), 3.87-3.93 (2H, m), 5.03 (2H, s), 6.10-6.12 (2H, m), 7.10 (1H, d, J=8.6 Hz), 7.13 (1H, d, J=8.6 Hz), 7.33-7.35 (1H, m), 7.41 (1H, d, J=8.6 Hz), 7.43 (1H, d, J=8.6 Hz), 7.68-7.80 (3H, m), 8.14-8.16 (2H, m).
ESI-MS Found: m/z 432[M+H]⁺

### Example 22:

### 1- {2-[(Dimethylamino)methyl]quinolin-6-yl}-4-[(4-fluorobenzyl)oxy]pyridin-2(1H)-one:

In the same manner as in Example 21 but using the compound (15 mg) obtained in Production Example 12-2-2) and a THF solution (4 mL) of 2 M dimethylamine, the entitled compound (5.5 mg) was obtained as a yellow powder.
¹H-NMR (400 MHz, CDCl₃, δ ppm): 2.38 (6H, s), 3.83 (2H, brs), 5.03 (2H, s), 6.09-6.11 (2H, m), 7.11 (1H, d, J=8.6 Hz), 7.13 (1H, d, J=8.6 Hz), 7.35 (1H, d, J=8.2 Hz), 7.41-7.43 (2H, m), 7.70-7.72 (2H, m), 7.81 (1H, d, J=2.3 Hz), 8.16 (1H, d, J=7.0 Hz), 8.18 (1H, d, J=8.2 Hz).
ESI-MS Found: m/z 404[M+H]⁺

### Example 23:

### 1- {2-[(Ethylamino)methyl]quinolin-6-yl}-4-[(4-fluorobenzyl)oxy]pyridin-2(1H)-one:

A THF solution (1.4 mL) of 2 M ethylamine, zinc chloride (50 mg) and sodium cyanotrihydroborate (235.8 mg) were added to a methanol/THF mixed solution (10:1, 11 mL) of the compound (300 mg) obtained in Production Example 13-1, and stirred overnight at room temperature. The solvent was evaporated off from the reaction liquid under reduced pressure, and aqueous saturated sodium hydrogencarbonate solution was added to the obtained residue, and extracted with a mixed solvent of chloroform/methanol (10:1). The organic layer was dried with anhydrous magnesium sulfate, the solvent was evaporated off under reduced pressure. The obtained residue was purified by silica gel column chromatography (NH silica gel, ethyl acetate:methanol = 50:1) to obtain the entitled compound (215 mg) as a yellow powder.
¹H-NMR (400 MHz, CDCl₃, δ ppm): 1.20 (3H, t, J=7.0 Hz), 2.78 (2H, q, J=7.1 Hz), 4.13 (2H, s), 5.03 (2H, s), 6.09-6.11 (2H, m), 7.10-7.13 (2H, m), 7.33-7.36 (1H, m), 7.41-7.43 (2H, m), 7.51 (1H, d, J=8.4 Hz), 7.70 (1H, dd, J=8.9, 2.4 Hz), 7.80 (1H, d, J=2.3 Hz), 8.13 (1H, d, J=8.4 Hz), 8.15 (1H, d, J=9.0 Hz).
ESI-MS Found: m/z 404[M+H]⁺

### Example 24:

### 4-[(4-Fluorobenzyl)oxy]-1- (2-[(3-hydroxypyrrolidin-1-yl)methyl]quinolin-6-yl)pyridin-2(1H)-one:

In the same manner as in Example 23 but using the compound (98 mg) obtained in Production Example 12-1, 3-pyrrolidinol (68 mg), and methanol as a solvent, followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (62.2 mg) was obtained as a yellow brown powder.
¹H-NMR (400 MHz, CDCl₃, δ ppm): 1.82-1.84 (1H, m), 2.20-2.26 (1H, m), 2.50-2.60 (1H, m), 2.76-2.82 (2H, m), 3.01-3.04 (1H, m), 4.05 (2H, s), 4.38-4.41 (1H, m), 5.03 (2H, s), 6.10-6.11 (2H, m), 7.10-7.13 (2H, m), 7.34 (1H, d, J=7.0 Hz), 7.41-7.43 (2H, m), 7.63 (1H, d, J=8.6 Hz), 7.70 (1H, dd, J=8.9, 2.4 Hz), 7.81 (1H, d, J=2.2 Hz), 8.16 (2H, dd, J=8.6, 3.7 Hz).
ESI-MS Found: m/z 446[M+H]⁺

### Example 25:

### 4-[(5-Chloropyridin-2-yl)methoxy]-1-(2-{[(2R)-2-methylpyrrolidin-1-yl]methyl}quinolin-6-yl}pyridin-2(1H)-one:

Potassium carbonate (352 mg) was added to a methanol solution (2 mL) of (2R)-2-methylpyrrolidine hydrobromide (84 mg), and stirred at room temperature for 30 minutes. The reaction liquid was filtered, then the compound obtained in Production Example 18-2 was added to the filtrate and processed in the same manner as in Example 23 to obtain the entitled compound (15.1 mg).
¹H-NMR (400 MHz, CDCl₃, δ ppm): 1.18 (3H, d, J=6.1 Hz), 1.49-1.52 (1H, m), 1.67-2.04 (3H, m), 2.29-2.33 (1H, m), 2.59-2.61 (1H, m), 2.94-2.96 (1H, m), 3.61 (1H, d, J=13.9 Hz), 4.28 (1H, d, J=13.7 Hz), 5.18 (2H, s), 6.07 (1H, d, J=2.7 Hz), 6.16 (1H, dd, J=7.6, 2.7 Hz), 7.36 (1H, d, J=7.6 Hz), 7.45 (1H, d, J=8.4 Hz), 7.66-7.79 (4H, m), 8.12-8.16 (2H, m), 8.59 (1H, d, J=2.3 Hz).
ESI-MS Found: m/z 461[M+H]⁺

### Example 26:

### 4-(Benzyloxy)-1-[1-(2-pyrrolidin-1-ylethyl)-1H-indazol-6-yl]pyridin-2(1H)-one:

In the same manner as in Production Example 1-4 but using 6-iodo-1-(2-pyrrolidin-1-ylethyl)-1H-indazole (838 mg) obtained in Production Example 15-2 and 4-benzyloxy-2(1H)-pyridone (989 mg), the entitled compound (380 mg) was obtained as a yellow powder.
¹H-NMR (400 MHz, CDCl₃, δ ppm): 1.75-1.79 (4H, m), 2.57 (4H, s), 3.00 (2H, t, J=7.4 Hz), 4.52 (2H, t, J=7.3 Hz), 5.07 (2H, s), 6.08-6.10 (2H, m), 7.12 (1H, dd, J=8.5, 1.7 Hz), 7.29-7.31 (1H, m), 7.40-7.44 (6H, m), 7.79-7.81 (1H, m), 8.03-8.04 (1H, m).
ESI-MS Found: m/z 415[M+H]⁺

### Example 27:

### 4-(Benzyloxy)-1-[2-(2-pyrrolidin-1-ylethyl)-2H-indazol-6-yl]pyridin-2(1H)-one:

In the same manner as in Production Example 1-4 but using 6-iodo-2-(2-pyrrolidin-1-ylethyl)-2H-indazole (338 mg) obtained in Production Example 15-2 and 4-benzyloxy-2(1H)-pyridone (398 mg), the entitled compound (180 mg) was obtained as a yellow powder.
¹H-NMR (400 MHz, CDCl₃, δ ppm): 1.76-1.79 (4H, m), 2.53-2.55 (4H, m), 3.08 (2H, t, J=6.7 Hz), 4.57 (2H, t, J=6.7 Hz), 5.05 (2H, s), 6.06-6.09 (2H, m), 7.10 (1H, dd, J=8.8, 1.8 Hz), 7.31 (1H, d, J=7.4 Hz), 7.37-7.43 (5H, m), 7.60 (1H, m), 7.70-7.73 (1H, m), 8.06 (1H, brs).
ESI-MS Found: m/z 415[M+H]⁺

### Example 28:

### 1-[6-(Azetidin-1-ylmethyl)quinolin-2-yl]-4-(benzyloxy)pyridin-2(1H)-one:

In the same manner as in Production Example 1-4 but using the compound (190 mg) obtained in Production Example 16-4 and 4-benzyloxy-2(1H)-pyridone (330 mg) followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (41.9 mg) was obtained as a white powder.
¹H-NMR (400 MHz, CDCl₃, δ ppm): 2.10-2.17 (2H, m), 3.28 (4H, t, J=7.0 Hz), 3.76 (2H, s), 5.07 (2H, s), 6.05 (1H, d, J=2.7 Hz), 6.16 (1H, dd, J=7.8, 2.7 Hz), 7.37-7.43 (5H, m), 7.66 (1H, dd, J=8.7, 1.9 Hz), 7.75 (1H, s), 7.95-7.99 (3H, m), 8.20 (1H, d, J=8.8 Hz).
ESI-MS Found: m/z 398[M+H]⁺

### Example 29:

### 7-[4-(Benzyloxy)-2-oxopyridin-1 (2H)-yl]-3-(3-pyrrolidin-1-ylpropyl)quinazolin-4(3H)-one:

In the same manner as in Production Example 1-4 but using the compound (137 mg) obtained in Production Example 17-3 and 4-benzyloxy-2(1H)-pyridone (165 mg) and changing the reaction time to 6 hours, followed by purification by reversed-phase HPLC (YMC-Pack ODS-AQ, acetonitrile (containing 0.1 % TFA):water (containing 0.1 % TFA) (10:90 to 90:10) and further followed by desalting treatment, the entitled compound (44 mg) was obtained. ¹H-NMR (400 MHz, CDCl₃, δ ppm): 1.27-1.31 (2H, m), 1.78-1.80 (4H, m), 2.01-2.03 (2H, m), 2.51-2.52 (4H, m), 4.13 (2H, t, J=6.7 Hz), 5.06 (2H, s), 6.08 (1H, d, J=2.3 Hz), 6.12 (1H, dd, J=7.6, 2.5 Hz), 7.29 (1H, d, J=7.8 Hz), 7.39-7.42 (5H, m), 7.56 (1H, dd, J=8.4, 2.2 Hz), 7.67 (1H, d, J=2.0 Hz), 8.18 (1H, s), 8.39 (1H, d, J=8.6 Hz).
ESI-MS Found: m/z 457[M+H]⁺

### Example 30:

### 1-[2-(Azetidin-1-ylmethyl)quinolin-6-yl]-4-[(5-chloropyridin-2-yl)methoxy]pyridin-2(1H)-one:

1) At room temperature, triethylamine (531 µL) and methanesulfonyl chloride (196 µL) were added to a chloroform suspension (15 mL) of the compound (500 mg) obtained in Production Example 19-4. After stirred for 30 minutes, the reaction liquid was poured into aqueous saturated sodium hydrogencarbonate solution, and extracted with chloroform. The organic layer was washed with saturated saline water, and dried with anhydrous sodium sulfate. The solvent was evaporated off under reduced pressure to obtain {6-[4-[(5-chloropyridin-2-yl)methoxy]-2-oxopyridin-1(2H)-yl]quinolin-2-yl}methyl methanesulfonate (590 mg) as a crude product.
2) At room temperature, potassium carbonate (790 mg) was added to a methanol solution (15 mL) of azetidine hydrochloride (356 mg), and stirred for 30 minutes. A chloroform solution (10 mL) of the compound (590 mg) obtained in Example 30-1) was dropwise added to the obtained suspension at room temperature, and stirred for 12 hours. The reaction liquid was poured into water, and extracted with chloroform. The organic layer was washed with saturated saline water, then dried with anhydrous sodium sulfate. The solvent was evaporated off under reduced pressure, and the residue was purified by silica gel column chromatography (NH silica gel, ethyl acetate:hexane = 5:1) to obtain the entitled compound (330 mg).
¹H-NMR (400 MHz, CDCl₃, δ ppm): 2.08-2.16 (2H, m), 3.32 (4H, t, J=7.0 Hz), 3.90 (2H, s), 5.14 (2H, s), 6.03 (1H, d, J=2.7 Hz), 6.12 (1H, dd, J=7.8, 2.7 Hz), 7.32 (1H, d, J=7.8 Hz), 7.42 (1H, d, J=8.6 Hz), 7.53 (1H, d, J=8.2 Hz), 7.64 (1H, dd, J=9.0, 2.3 Hz), 7.71 (1H, dd, J=8.2, 2.3 Hz), 7.75 (1H, d, J=2.3 Hz), 8.09 (1H, d, J=9.0 Hz), 8.12 (1H, d, J=9.0 Hz), 8.56 (1H, d, J=2.3 Hz).
ESI-MS Found: m/z 433[M+H]⁺

### Example 31:

### 4-[(4-Fluorobenzyl)oxy]-1-{2-[(isopropylamino)methyl]quinolin-6-yl}pyridin-2(1H)-one:

In the same manner as in Example 21 but using the compound (63.5 mg) obtained in Production Example 20-2 and isopropylamine (1 mL), the entitled compound (42.1 mg) was obtained.
¹H-NMR (400 MHz, CDCl₃, δ ppm): 1.13 (6H, d, J=6.3 Hz), 1.74 (1H, s), 2.87-2.93 (1H, m), 4.09 (2H, s), 4.99 (2H, s), 6.05-6.07 (2H, m), 7.07 (1H, d, J=8.5 Hz), 7.09 (1H, d, J=8.6 Hz), 7.31 (1H, d, J=8.2 Hz), 7.36-7.40 (2H, m), 7.46 (1H, d, J=8.4 Hz), 7.66 (1H, dd, J=9.0, 2.3 Hz), 7.76 (1H, d, J=2.3 Hz), 8.08 (1H, d, J=8.4 Hz), 8.11 (1H, d, J=9.0 Hz).
ESI-MS Found: m/z 418[M+H]⁺

### Example 32:

### 4-[(4-Fluorobenzyl)oxy]-1-{2-[(propylamino)methyl]quinolin-6-yl}pyridin-2(1H)-one:

In the same manner as in Example 21 but using the compound (95.1 mg) obtained in Production Example 20-2 and propylamine (2 mL), the entitled compound (61.5 mg) was obtained.
¹H-NMR (400 MHz, CDCl₃, δ ppm): 0.92 (3H, t, J=7.3 Hz), 1.57 (2H, dt, J=7.3, 7.2 Hz), 1.87 (1H, s), 2.66 (2H, t, J=7.2 Hz), 4.09 (2H, s), 4.99 (2H, s), 6.05-6.07 (2H, m), 7.07 (1H, d, J=8.6 Hz), 7.09 (1H, d, J=8.6 Hz), 7.31 (1H, d, J=7.8 Hz), 7.37 (1H, d, J=8.6 Hz), 7.39 (1H, d, J=8.6 Hz), 7.48 (1H, d, J=8.6 Hz), 7.66 (1H, dd, J=8.6, 2.3 Hz), 7.76 (1H, d, J=2.3 Hz), 8.09 (1H, d, J=7.0 Hz), 8.11 (1H, d, J=8.6 Hz).
ESI-MS Found: m/z 418[M+H]⁺

### Example 33:

### 1-{2-[(Cyclopropylamino)methyl]quinolin-6-yl}-4-[(4-fluorobenzyl)oxy]pyridin-2(1H)-one:

In the same manner as in Example 21 but using the compound (59.0 mg) obtained in Production Example 20-2 and cyclopropylamine (180 µL), the entitled compound (35.1 mg) was obtained.
¹H-NMR (400 MHz, CDCl₃, δ ppm): 0.43-0.44 (4H, m), 1.89 (1H, s), 2.19-2.24 (1H, m), 4.16 (2H, s), 4.99 (2H, s), 6.05-6.08 (2H, m), 7.07 (1H, d, J=8.6 Hz), 7.09 (1H, d, J=8.6 Hz), 7.31 (1H, d, J=8.2 Hz), 7.37 (1H, d, J=8.6 Hz), 7.39 (1H, d, J=8.6 Hz), 7.43 (1H, d, J=8.2 Hz), 7.66 (1H, dd, J=8.8, 2.4 Hz), 7.76 (1H, d, J=2.4 Hz), 8.08 (1H, d, J=8.2 Hz), 8.12 (1H, d, J=8.6 Hz). ESI-MS Found: m/z 416[M+H]⁺

### Example 34:

### 1-[2-(Aminomethyl)quinolin-6-yl]-4-[(4-fluorobenzyl)oxy]pyridin-2(1H)-one:

At room temperature, sodium azide (42.3 mg) was added to a DMF solution (6 mL) of the compound (59.0 mg) obtained in Production Example 20-2. After stirred for 3.5 hours, the reaction liquid was poured into saturated saline water, and extracted with ethyl acetate. The organic layer was washed with saturated saline water, and dried with anhydrous sodium sulfate. The solvent was evaporated off under reduced pressure, and the residue was dissolved in THF (10 mL). Water (23.4 µL) and triphenyl phosphine (68.2 mg) were added to the obtained solution in a nitrogen atmosphere at room temperature. The reaction liquid was heated at 45°C, stirred for 16 hours, and the solvent was evaporated off under reduced pressure. The obtained residue was purified by silica gel column chromatography (chloroform:methanol = 100:1) to obtain the entitled compound (24.1 mg).
¹H-NMR (400 MHz, CDCl₃, δ ppm): 1.76 (2H, s), 4.16 (2H, s), 5.00 (2H, s), 6.05-6.08 (2H, m), 7.07 (1H, d, J=8.6 Hz), 7.09 (1H, d, J=8.6 Hz), 7.31 (1H, d, J=8.2 Hz), 7.37 (1H, d, J=8.6 Hz), 7.39 (1H, d, J=8.6 Hz), 7.42 (1H, d, J=8.4 Hz), 7.67 (1H, dd, J=8.9, 2.3 Hz), 7.77 (1H, d, J=2.3 Hz), 8.09 (1H, d, J=8.0 Hz), 8.11 (1H, d, J=8.6 Hz).
ESI-MS Found: m/z 376[M+H]⁺

### Example 35:

### 4-[(4-Chlorobenzyl)oxy]-1-{2-[(methylamino)methyl]quinolin-6-yl}pyridin-2(1H)-one:

1) In the same manner as in Example 30-1) but using the compound (80.0 mg) obtained in Production Example 3-3-3), {6-[4-[(4-chlorobenzyl)oxy]-2-oxopyridin-(2H)-yl]quinolin-2-yl}methyl methanesulfonate (94.0 mg) was obtained.
2) In the same manner as in Example 21 but using the compound (94.0 mg) obtained in Production Example 35-1) and a THF solution (15 mL) of 2 M methylamine, the entitled compound (57.5 mg) was obtained.
¹H-NMR (400 MHz, CDCl₃, δ ppm): 1.80 (1H, s), 2.61 (3H, s), 4.16 (2H, s), 5.01 (2H, s), 6.04-6.09 (2H, m), 7.30-7.38 (5H, m), 7.46 (1H, d, J=9.0 Hz), 7.68 (1H, dd, J=8.8, 2.2 Hz), 7.78 (1H, d, J=2.2 Hz), 8.11 (1H, d, J=8.2 Hz), 8.14 (1H, d, J=8.6 Hz).
ESI-MS Found: m/z 406[M+H]⁺

### Example 36:

### 4-[(4-Chlorobenzyl)oxy]-1-{2-[(propylamino)methyl]quinolin-6-yl}pyridin-2(1H)-one:

In the same manner as in Example 21 but using the compound (23.5 mg) obtained in Production Example 35-1) and propylamine (82.2 µL), the entitled compound (20.2 mg) was obtained.
¹H-NMR (400 MHz, CDCl₃, δ ppm): 0.92 (3H, t, J=7.4 Hz), 1.56 (2H, dt, J=7.4, 7.2 Hz), 1.88 (1H, s), 2.65 (2H, t, J=7.2 Hz), 4.09 (2H, s), 5.00 (2H, s), 6.03-6.08 (2H, m), 7.30-7.38 (5H, m), 7.48 (1H, d, J=8.6 Hz), 7.66 (1H, dd, J=9.0, 2.3 Hz), 7.76 (1H, d, J=2.3 Hz), 8.09 (1H, d, J=7.8 Hz), 8.11 (1H, d, J=8.6 Hz).
ESI-MS Found: m/z 434[M+H]⁺

### Example 37:

### 4-[(4-Chlorobenzyl)oxy]-1-{2-[(cyclopropylamino)methyl]quinolin-6-yl}pyridin-2(1H)-one:

In the same manner as in Example 21 but using the compound (23.5 mg) obtained in Production Example 35-1) and cyclopropylamine (100 µL), the entitled compound (7.1 mg) was obtained.
¹H-NMR (400 MHz, CDCl₃, δ ppm): 0.43-0.44 (4H, m), 2.07 (1H, s), 2.19-2.24 (1H, m), 4.16 (2H, s), 5.00 (2H, s), 6.04 (1H, d, J=2.5 Hz), 6.07 (1H, dd, J=7.4, 2.5 Hz), 7.30-7.38 (5H, m), 7.43 (1H, d, J=8.6 Hz), 7.66 (1H, dd, J=8.8, 2.4 Hz), 7.76 (1H, d, J=2.4 Hz), 8.08 (1H, d, J=8.6 Hz), 8.11 (1H, d, J=9.0 Hz).
ESI-MS Found: m/z 432[M+H]⁺

### Example 38:

### 4-(Benzyloxy)-1-[6-(2-pyrrolidin-1-ylethoxy)-2-naphthyl]pyridin-2(1H)-one:

In the same manner as in Production Example 1-4 but using the compound (600 mg) obtained in Production Example 44-1 and 4-benzyloxy-2(1H)-pyridone (567 mg), the entitled compound (117.3 mg) was obtained as a pale yellow powder.
¹H-NMR (400 MHz, CDCl₃, δ ppm): 1.82-1.85 (4H, m), 2.66-2.68 (4H, m), 2.98 (2H, t, J=6.0 Hz), 4.25 (2H, t, J=5.9 Hz), 5.06 (2H, s), 6.07-6.10 (2H, m), 7.18-7.18 (1H, m), 7.22 (1H, dd, J=8.9, 2.4 Hz), 7.32 (1H, d, J=7.4 Hz), 7.40-7.43 (6H, m), 7.70 (1H, d, J=2.0 Hz), 7.74 (1H, d, J=9.0 Hz), 7.79 (1H, d, J=8.8 Hz).
ESI-MS Found: m/z 442[M+H]⁺

### Example 39:

### 4-[(4-Chlorobenzyl)oxy]-1-[2-(pyrrolidin-1-ylmethyl)quinolin-6-yl]pyridin-2(1H)-one:

In the same manner as in Example 21 but using the compound (17.5 mg) obtained in Example 35-1) and pyrrolidine (0.5 mL), the entitled compound (15.2 mg) was obtained.
¹H-NMR (400 MHz, CDCl₃, δ ppm): 1.80-1.82 (4H, brm), 2.60-2.63 (4H, brm), 3.97 (2H, s), 5.00 (2H, s), 6.03-6.08 (2H, m), 7.30-7.38 (5H, m), 7.65 (2H, m), 7.76 (1H, d, J=2.2 Hz), 8.11 (1H, d, J=8.6 Hz), 8.13 (1H, d, J=9.0 Hz).
ESI-MS Found: m/z 446[M+H]⁺

### Example 40:

### 4-[(5-Chloropyridin-2-yl)methoxy]-1-{2-[(propylamino)methyl]quinolin-6-yl}pyridin-2(1H)-one:

In the same manner as in Example 23 but using the compound (30.0 mg) obtained in Production Example 18-2 and propylamine (7.6 µL), the entitled compound (11.2 mg) was obtained. ¹H-NMR (400 MHz, CDCl₃, δ ppm): 0.92 (3H, t, J=7.4 Hz), 1.57 (2H, dt, J=7.4, 7.2 Hz), 1.74 (1H, s), 2.66 (2H, t, J=7.2 Hz), 4.09 (2H, s), 5.15 (2H, s), 6.0.3 (1H, d, J=2.7, Hz), 6.12 (1H, dd, J=7.8, 2.7 Hz), 7.33 (1H, d, J=7.8 Hz), 7.42 (1H, d, J=8.2 Hz), 7.48 (1H, d, J=8.2 Hz), 7.66 (1H, dd, J=9.0, 2.3 Hz), 7.71 (1H, dd, J=8.2, 2.3 Hz), 7.76 (1H, d, J=2.3 Hz), 8.09 (1H, d, J=7.8 Hz), 8.11 (1H, d, J=7.8 Hz), 8.56 (1H, d, J=2.3 Hz).
ESI-MS Found: m/z 435[M+H]⁺

### Example 41:

### 4-[(5-Chloropyridin-2-yl)methoxy]-1-{2-[(isopropylamino)methyl]quinolin-6-yl}pyridin-2(1H)-one:

In the same manner as in Example 23 but using the compound (30.0 mg) obtained in Production Example 18-2 and isopropylamine (9.8 µL), the entitled compound (11.4 mg) was obtained.
¹H-NMR (400 MHz, CDCl₃, δ ppm): 1.14 (6H, d, J=6.3 Hz), 1.86 (1H, s), 2.87-2.94 (1H, m), 4.10 (2H, s), 5.15 (2H, s), 6.04 (1H, d, J=2.5 Hz), 6.12 (1H, dd, J=7.7, 2.5 Hz), 7.33 (1H, d, J=7.7 Hz), 7.42 (1H, d, J=8.4 Hz), 7.46 (1H, d, J=8.4 Hz), 7.65 (1H, dd, J=9.0, 2.4 Hz), 7.71 (1H, dd, J=8.4, 2.4 Hz), 7.76 (1H, d, J=2.3 Hz), 8.08 (1H, d, J=8.4 Hz), 8.11 (1H, d, J=9.0 Hz), 8.56 (1H, d, J=2.3 Hz).
ESI-MS Found: m/z 435[M+H]⁺

### Example 42:

### 4-[(5-Chloropyridin-2-yl)methoxy]-1-{2-[(cyclopropylamino)methyl]quinolin-6-yl}pyridin-2(1H)-one:

In the same manner as in Example 23 but using the compound (30.0 mg) obtained in Production Example 18-2 and cyclopropylamine (6.4 µL), the entitled compound (17.6 mg) was obtained.
¹H-NMR (400 MHz, CDCl₃, δ ppm): 0.43 (4H, t, J=3.3 Hz), 1.89 (1H, s), 2.19-2.24 (1H, m), 4.15 (2H, s), 5.15 (2H, s), 6.03 (1H, d, J=2.7 Hz), 6.12 (1H, dd, J=7.8, 2.7 Hz), 7.33 (1H, d, J=7.4 Hz), 7.41-7.44 (2H, m), 7.65 (1H, dd, J=8.6, 2.3 Hz), 7.71 (1H, dd, J=8.2, 2.3 Hz), 7.76 (1H, d, J=2.3 Hz), 8.08 (1H, d, J=8.6 Hz), 8.11 (1H, d, J=9.0 Hz), 8.56 (1H, d, J=2.3 Hz).
ESI-MS Found: m/z 433[M+H]⁺

### Example 43:

### 4-[(5-Chloropyridin-2-yl)methoxy]-1-[2-(morpholin-4-ylmethyl)quinolin-6-yl]pyridin-2(1H)-one:

In the same manner as in Example 23 but using the compound (30.0 mg) obtained in Production Example 18-2 and morpholine (8.1 µL), the entitled compound (11.7 mg) was obtained.
¹H-NMR (400 MHz, CDCl₃, δ ppm): 2.51-2.54 (4H, brm), 3.71-3.73 (4H, brm), 3.82 (2H, s), 5.15 (2H, s), 6.03 (1H, d, J=2.7 Hz), 6.12 (1H, dd, J=7.4, 2.7 Hz), 7.32 (1H, d, J=7.8 Hz), 7.42 (1H, d, J=8.2 Hz), 7.64-7.67 (2H, m), 7.71 (1H, dd, J=8.2, 2.3 Hz), 7.77 (1H, d, J=2.3 Hz), 8.10-8.14 (2H, m), 8.56 (1H, d, J=2.3 Hz).
ESI-MS Found: m/z 431[M+H]⁺

### Example 44:

### 4-[(5-Chloropyridin-2-yl)methoxy]-1-[2-(pyrrolidin-1-ylmethyl)quinolin-6-yl]pyridin-2(1H)-one:

In the same manner as in Example 21 but using the compound (2.40 g) obtained in Example 30-1) and pyrrolidine (6 mL), the entitled compound (1.21 g) was obtained.
¹H-NMR (400 MHz, CDCl₃, δ ppm): 1.84-1.86 (4H, brm), 2.63-2.66 (4H, brm), 4.00 (2H, s), 5.20 (2H, s), 6.09 (1H, d, J=2.7 Hz), 6.17 (1H, dd, J=7.4, 2.7 Hz), 7.38 (1H, d, J=7.4 Hz), 7.47 (1H, d, J=8.6 Hz), 7.67-7.71 (2H, m), 7.76 (1H, dd, J=8.4, 2.5 Hz), 7.81 (1H, d, J=2.3 Hz), 8.15 (1H, d, J=8.6 Hz), 8.19 (1H, d, J=9.0 Hz), 8.61 (1H, d, J=2.3 Hz).
ESI-MS Found: m/z 447[M+H]⁺

### Example 45:

### 1-[2-(Azetidin-1-ylmethyl)quinolin-6-yl]-4-[(4-fluorobenzyl)oxy]pyridin-2(1H)-one:

In the same manner as in Example 23 but using the compound (80.0 mg) obtained in Production Example 22-1 and azetidine (17.0 µL), the entitled compound (43.7 mg) was obtained.
¹H-NMR (400 MHz, CDCl₃, δ ppm): 2.08-2.15 (2H, m), 3.32 (4H, t, J=7.0 Hz), 3.90 (2H, s), 4.99 (2H, s), 6.05-6.07 (2H, m), 7.05-7.10 (2H, m), 7.30 (1H, dd, J=6.3, 2.0 Hz), 7.36-7.40 (2H, m), 7.52 (1H, d, J=8.4 Hz), 7.65 (1H, dd, J=9.0, 2.4 Hz), 7.75 (1H, d, J=2.3 Hz), 8.09 (1H, d, J=8.6 Hz), 8.12 (1H, d, J=9.0 Hz).
ESI-MS Found: m/z 416[M+H]⁺

### Example 46:

### 1-[2-(Azetidin-1-ylmethyl)quinolin-6-yl]-4-(benzyloxy)pyridin-2(1H)-one:

In the same manner as in Example 23 but using the compound (88.0 mg) obtained in Production Example 21-1 and azetidine (17.0 µL), the entitled compound (47.6 mg) was obtained.
¹H-NMR (400 MHz, CDCl₃, δ ppm): 2.09-2.16 (2H, m), 3.32 (4H, t, J=7.0 Hz), 3.90 (2H, s), 5.04 (2H, s), 6.07-6.09 (2H, m), 7.30 (1H, dd, J=6.3, 2.0 Hz), 7.34-7.40 (5H, m), 7.53 (1H, d, J=8.4 Hz), 7.66 (1H, dd, J=9.0, 2.3 Hz), 7.75 (1H, d, J=2.3 Hz), 8.10 (1H, d, J=9.4 Hz), 8.12 (1H, d, J=9.6 Hz).
ESI-MS Found: m/z 398[M+H]⁺

### Example 47:

### 4-(Benzyloxy)-1-[2-(pyrrolidin-1-ylmethyl)quinolin-6-yl]pyridin-2(1H)-one:

1) In the same manner as in Example 30-1) but using the compound (1.25 g) obtained in Production Example 19-1, {6-[4-(benzyloxy)-2-oxopyridin-1(2H)-yl]quinolin-2-yl}methyl methanesulfonate (1.50 g) was obtained.
2) In the same manner as in Example 21 but using the compound (1.50 g) obtained in Example 47-1) and pyrrolidine (2.91 mL), the entitled compound (918 mg) was obtained.
   ¹H-NMR (400 MHz, CDCl₃, δ ppm): 1.78-1.82 (4H, brm), 2.57-2.61 (4H, brm), 3.95 (2H, s), 5.04 (2H, s), 6.07-6.08 (2H, m), 7.30 (1H, d, J=8.2 Hz), 7.34-7.40 (6H, m), 7.66 (1H, dd, J=9.0, 2.3 Hz), 7.76 (1H, d, J=2.3 Hz), 8.10 (1H, d, J=8.6 Hz), 8.13 (1H, d, J=9.0 Hz).
   ESI-MS Found: m/z 412[M+H]⁺

### Example 48:

### 4-[(5-Chloropyridin-2-yl)methoxy]-1-[2-(1-pyrrolidin-2-ylethyl)quinolin-6-yl]pyridin-2(1H)-one:

In the same manner as in Example 23 but using the compound (73.0 mg) obtained in Production Example 23-2 and pyrrolidine (19.5 µL), a racemate of the entitled compound (63.2 mg) was obtained. This was processed for optical resolution with a chiral column (Daicel CHIRALCEL OJ-H, hexane:ethanol:diethylamine = 70:30:0.03, retention time 9.56 min, 11.2 min) to obtain their optically-active form.
¹H-NMR (400 MHz, CDCl₃, δ ppm): 1.46 (3H, d, J=6.5 Hz), 1.75-1.77 (4H, brm), 2.36-2.40 (2H, brm), 2.62-2.66 (2H, brm), 3.54-3.58 (1H, brm), 5.14 (2H, s), 6.03 (1H, d, J=2.7 Hz), 6.12 (1H, dd, J=7.6, 2.7 Hz), 7.32 (1H, d, J=7.6 Hz), 7.42 (1H, d, J=8.2 Hz), 7.62-7.65 (2H, m), 7.71 (1H, dd, J=8.2, 2.3 Hz), 7.75 (1H, d, J=2.3 Hz), 8.10 (1H, d, J=8.6 Hz), 8.14 (1H, d, J=9.0 Hz), 8.56 (1H, d, J=2.3 Hz).
ESI-MS Found: m/z 461 [M+H]⁺

### Example 49:

### 1-[2-(1-Azetidin-1-ylethyl)quinolin-6-yl]-4-[(5-chloropyridin-2-yl)methoxy]pyridin-2(1H)-one:

In the same manner as in Example 23 but using the compound (73.0 g) obtained in Production Example 23-2 and azetidine (15.8 µL), a racemate of the entitled compound (71.0 mg) was obtained. This was processed for optical resolution with a chiral column (Daicel CHIRALCEL OJ-H, hexane:ethanol:diethylamine = 70:30:0.03, retention time 9.12 min, 11.5 min) to obtain their optically-active form.
¹H-NMR (400 MHz, CDCl₃, δ ppm): 1.28 (3H, d, J=6.8 Hz), 2.02-2.09 (2H, m), 3.13 (2H, q, J=6.8 Hz), 3.26 (2H, q, J=6.8 Hz), 3.64 (1H, q, J=6.6 Hz), 5.14 (2H, s), 6.03 (1H, d, J=2.7 Hz), 6.12 (1H, dd, J=7.6, 2.7 Hz), 7.32 (1H, d, J=7.6 Hz), 7.42 (1H, d, J=8.4 Hz), 7.57 (1H, d, J=8.6 Hz), 7.64 (1H, dd, J=9.0, 2.3 Hz), 7.71 (1H, dd, J=8.4, 2.3 Hz), 7.75 (1H, d, J=2.3 Hz), 8.10 (1H, d, J=8.6 Hz), 8.14 (1H, d, J=9.0 Hz), 8.56 (1H, d, J=2.3 Hz).
ESI-MS Found: m/z 447[M+H]⁺

### Example 50:

### 4-[(5-Fluoropyridin-2-yl)methoxy]-1-[2-(pyrrolidin-1-ylmethyl)quinolin-6-yl]pyridin-2(1H)-one:

1) In a nitrogen atmosphere at room temperature, 10 % palladium-carbon (20.0 mg) was added to a methanol solution (5 mL) of the compound (45.9 mg) obtained in Example 47. The reaction system was purged with hydrogen (1 atmospheric pressure), stirred at room temperature for 2 hours, then the catalyst was separated by filtration. The solvent was evaporated off from the filtrate under reduced pressure, and dried under reduced pressure. Using this and (5-fluoropyridin-2-yl)methyl methanesulfonate (24.8 mg) and in the same manner as in Example 1 followed by purification by silica gel column chromatography (NH silica gel, ethyl acetate:hexane = 4:1 to 5:1) to obtain the entitled compound (10.7 mg).
   ¹H-NMR (400 MHz, CDCl₃, δ ppm): 1.78-1.81 (4H, brm), 2.58-2.60 (4H, brm), 3.95 (2H, s), 5.15 (2H, s), 6.05 (1H, d, J=2.3 Hz), 6.11 (1H, dd, J=7.4, 2.7 Hz), 7.32 (1H, d, J=7.4 Hz), 7.42-7.49 (2H, m), 7.62-7.66 (2H, m), 7.76 (1H, d, J=2.3 Hz), 8.10 (1H, d, J=8.2 Hz), 8.13 (1H, d, J=9.0 Hz), 8.46 (1H, d, J=2.7 Hz).
   ESI-MS Found: m/z 431[M+H]⁺

### Example 51:

### 1-[2-(Azetidin-1-ylmethyl)quinoxalin-6-yl]-4-[(4-fluorobenzyl)oxy]pyridin-2(1H)-one:

In the same manner as in Example 23 but using the compound (60.0 mg) obtained in Production Example 24-4 and azetidine (16.2 µL), the entitled compound (24.3 mg) was obtained.
¹H-NMR (400 MHz, CDCl₃, δ ppm): 2.11-2.18 (2H, m), 3.35 (4H, t, J=7.0 Hz), 3.95 (2H, s), 5.00 (2H, s), 6.06 (1H, d, J=2.2 Hz), 6.09 (1H, dd, J=7.5, 2.2 Hz), 7.07 (1H, d, J=8.4 Hz), 7.09 (1H, d, J=8.6 Hz), 7.33 (1H, d, J=7.4 Hz), 7.37 (1H, d, J=8.4 Hz), 7.39 (1H, d, J=8.6 Hz), 7.81 (1H, dd, J=8.8, 2.3 Hz), 7.99 (1H, d, J=2.3 Hz), 8.12 (1H, d, J=9.0 Hz), 8.91 (1H, s).
ESI-MS Found: m/z 417[M+H]⁺

### Example 52:

### 4-[(4-Fluorobenzyl)oxy]-1-[2-(pyrrolidin-1-ylmethyl)quinoxalin-6-yl]pyridin-2(1H)-one:

In the same manner as in Example 23 but using the compound (60.0 mg) obtained in Production Example 24-4 and pyrrolidine (20.0 µL), the entitled compound (26.1 mg) was obtained.
¹H-NMR (400 MHz, CDCl₃, δ ppm): 1.80-1.83 (4H, brm), 2.60-2.62 (4H, brm), 4.00 (2H, s), 5.00 (2H, s), 6.06 (1H, d, J=2.5 Hz), 6.09 (1H, dd, J=7.6, 2.5 Hz), 7.07 (1H, d, J=8.6 Hz), 7.09 (1H, d, J=8.6 Hz), 7.34 (1H, d, J=7.6 Hz), 7.38 (1H, d, J=8.6 Hz), 7.39 (1H, d, J=8.6 Hz), 7.81 (1H, dd, J=9.0, 2.3 Hz), 8.00 (1H, d, J=2.3 Hz), 8.13 (1H, d, J=8.8 Hz), 8.99 (1H, s).
ESI-MS Found: m/z 431[M+H]⁺

### Example 53:

### 1- {2-[(Ethylamino)methyl]quinoxalin-6-yl}-4-[(4-fluorobenzyl)oxy]pyridin-2(1H)-one:

1) In the same manner as in Example 30-1) but using the compound (219 mg) obtained in Production Example 25-1, {6-[4-[(4-fluorobenzyl)oxy]-2-oxopyridin-1(2H)-yl]quinoxalin-2-yl}methyl methanesulfonate (260 mg) was obtained.
2) In the same manner as in Example 21 but using the compound (80.0 mg) obtained in Example 53-1) and a THF solution (4 mL) of 2 M ethylamine, the entitled compound (29.5 mg) was obtained.
   ¹H-NMR (400 MHz, CDCl₃, δ ppm): 1.17 (3H, t, J=7.1 Hz), 2.77 (2H, q, J=7.1 Hz), 4.16 (2H, s), 5.00 (2H, s), 6.06 (1H, d, J=2.7 Hz), 6.09 (1H, dd, J=7.6, 2.7 Hz), 7.07 (1H, d, J=8.8 Hz), 7.09 (1H, d, J=8.6 Hz), 7.34 (1H, d, J=7.4 Hz), 7.37 (1H, d, J=8.6 Hz), 7.39 (1H, d, J=8.8 Hz), 7.82 (1H, dd, J=9.0, 2.3 Hz), 8.00 (1H, d, J=2.3 Hz), 8.11 (1H, d, J=9.0 Hz), 8.89 (1H, s).
   ESI-MS Found: m/z 405[M+H]⁺

### Example 54:

### 4-[(4-Fluorobenzyl)oxy]-1-{2-[(propylamino)methyl]quinoxalin-6-yl}pyridin-2(1H)-one:

In the same manner as in Example 21 but using the compound (80.0 mg) obtained in Example 53-1) and propylamine (2 mL), the entitled compound (32.2 mg) was obtained.
¹H-NMR (400 MHz, CDCl₃, δ ppm): 0.93 (3H, t, J=7.3 Hz), 1.56 (2H, dt, J=7.3, 7.1 Hz), 2.67 (2H, t, J=7.1 Hz), 4.16 (2H, s), 5.00 (2H, s), 6.06 (1H, d, J=2.7 Hz), 6.10 (1H, dd, J=7.6, 2.7 Hz), 7.07 (1H, d, J=8.6 Hz), 7.09 (1H, d, J=8.6 Hz), 7.34 (1H, d, J=7.6 Hz), 7.38 (1H, d, J=8.6 Hz), 7.39 (1H, d, J=8.6 Hz), 7.82 (1H, dd, J=9.0, 2.3 Hz), 8.00 (1H, d, J=2.3 Hz), 8.11 (1H, d, J=9.0 Hz), 8.90 (1H, s).
ESI-MS Found: m/z 419[M+H]⁺

### Example 55:

### 4-[(4-Fluorobenzyl)oxy]-1-{2-[(isopropylamino)methyl]quinoxalin-6-yl}pyridin-2(1H)-one:

In the same manner as in Example 21 but using the compound (80.0 mg) obtained in Example 53-1) and isopropylamine (2 mL), the entitled compound (35.1 mg) was obtained. ¹H-NMR (400 MHz, CDCl₃, δ ppm): 1.14 (6H, d, J=6.3 Hz), 2.88-2.94 (1H, m), 4.16 (2H, s), 5.00 (2H, s), 6.06 (1H, d, J=2.7 Hz), 6.09 (1H, dd, J=7.6, 2.7 Hz), 7.07 (1H, d, J=8.6 Hz), 7.09 (1H, d, J=8.6 Hz), 7.34 (1H, d, J=7.6 Hz), 7.38 (1H, d, J=8.6 Hz), 7.39 (1H, d, J=8.6 Hz), 7.82 (1H, dd, J=8.8, 2.3 Hz), 7.99 (1H, d, J=2.3 Hz), 8.11 (1H, d, J = 8.8 Hz), 8.89 (1H, s).
ESI-MS Found: m/z 419[M+H]⁺

### Example 56:

### 4-[(4-Fluorobenzyl)oxy]-1-[2-(2-pyrrolidin-1-ylethoxy)quinoxalin-6-yl]pyridin-2(1H)-one:

In the same manner as in Production Example 1-4 but using the compound (200 mg) obtained in Production Example 26-5 and 4-(fluorobenzyl)oxy-2(1H)-pyridone (118 mg), the entitled compound (92.4 mg) was obtained.
¹H-NMR (400 MHz, CDCl₃, δ ppm): 1.79-1.82 (4H, brm), 2.62-2.64 (4H, brm), 2.95 (2H, t, J=5.7 Hz), 4.61 (2H, t, J=5.7 Hz), 4.99 (2H, s), 6.05-6.08 (2H, m), 7.07 (1H, d, J=8.6 Hz), 7.09 (1H, d, J=8.6 Hz), 7.32 (1H, d, J=7.4 Hz), 7.37 (1H, d, J=8.6 Hz), 7.39 (1H, d, J=8.6 Hz), 7.71 (1H, dd, J=8.6, 2.3 Hz), 7.86 (1H, d, J=9.0 Hz), 7.91 (1H, d, J=2.3 Hz), 8.51 (1H, s).
ESI-MS Found: m/z 461[M+H]⁺

### Example 57:

### 4-[(E)-2-(5-chloropyridin-2-yl)vinyl]-1-[2-(2-pyrrolidin-1-ylethoxy)quinoxalin-6-yl]pyridin-2(1H)-one:

At room temperature, 4-[(E)-2-(5-chloropyridin-2-yl)vinyl]pyridin-2(1H)-one (25.6 mg), copper acetate (25.4 mg) and a THF solution (220 µL) of 1.0 M tetrabutylammonium fluoride were successively added to a dichloromethane solution (4 mL) of the crude product obtained in Production Example 27-1. After stirred for 20 hours, 4-[(E)-2-(5-chloropyridin-2-yl)vinyl]pyridin-2(1H)-one (25.6 mg) was added, and further stirred for 16 hours. The reaction liquid was poured into aqueous diluted ammonia solution, and extracted with chloroform. The organic layer was washed with saturated saline water, and dried with anhydrous sodium sulfate. The solvent was evaporated off under reduced pressure, and the residue was purified by silica gel column chromatography (NH silica gel, hexane:ethyl acetate = 1:2 to 1:4) to obtain the entitled compound (4.0 mg).
¹H-NMR (400 MHz, CDCl₃, δ ppm): 1.80-1.83 (4H, brm), 2.63-2.66 (4H, brm), 2.95-2.98 (2H, brm), 4.61-4.64 (2H, brm), 6.54 (1H, brd, J=7.0 Hz), 6.73 (1H, brs), 7.19 (1H, d, J=16.0 Hz), 7.36 (1H, d, J=8.2 Hz), 7.43 (1H and 1H, d and d, J=16.0 and 7.4 Hz), 7.66 (1H, dd, J=8.2, 2.3 Hz), 7.76 (1H, dd, J=8.7, 2.2 Hz), 7.89 (1H, d, J=8.7 Hz), 7.96 (1H, d, J=2.2 Hz), 8.52 (1H, s), 8.56 (1H, d, J=2.3 Hz).
ESI-MS Found: m/z 474[M+H]⁺

### Example 58:

### 1-[2-(Azetidin-1-ylmethyl)quinazolin-6-yl]-4-[(4-fluorobenzyl)oxy]pyridin-2(1H)-one:

1) In the same manner as in Example 30-1) but using the compound (171 mg) obtained in Production Example 28-6, {6-[4-[(4-fluorobenzyl)oxy]-2-oxopyridin-1(2H)-yl]quinazolin-2-yl}methyl methanesulfonate (200 mg) was obtained.
2) In the same manner as in Example 21 but using the compound (40.0 mg) obtained in Example 58-1) and azetidine (60.0 µL), the entitled compound (22.5 mg) was obtained.
   ¹H-NMR (400 MHz, CDCl₃, δ ppm): 2.12-2.19 (2H, m), 3.41 (4H, t, J=7.0 Hz), 4.03 (2H, s), 5.00 (2H, s), 6.05 (1H, d, J=2.7 Hz), 6.09 (1H, dd, J=7.6, 2.7 Hz), 7.07 (1H, d, J=8.6 Hz), 7.09 (1H, d, J=8.6 Hz), 7.30 (1H, d, J=7.6 Hz), 7.37 (1H, d, J=8.6 Hz), 7.38 (1H, d, J=8.6 Hz), 7.87-7.89 (2H, m), 8.11 (1H, brd, J=9.6 Hz), 9.36 (1H, s).
   ESI-MS Found: m/z 417[M+H]⁺

### Example 59:

### 4-[(4-Fluorobenzyl)oxy]-1-[2-(pyrrolidin-1-ylmethyl)quinazolin-6-yl]pyridin-2(1H)-one:

In the same manner as in Example 21 but using the compound (40.0 mg) obtained in Example 58-1) and pyrrolidine (500 µL), the entitled compound (26.0 mg) was obtained.
¹H-NMR (400 MHz, CDCl₃, δ ppm): 1.82-1.85 (4H, brm), 2.67-2.70 (4H, brm), 4.10 (2H, s), 5.00 (2H, s), 6.05 (1H, d, J=2.7 Hz), 6.10 (1H, dd, J=7.6, 2.7 Hz), 7.07 (1H, d, J=8.8 Hz), 7.09 (1H, d, J=8.8 Hz), 7.30 (1H, d, J=7.6 Hz), 7.38 (1H, d, J=8.8 Hz), 7.39 (1H, d, J=8.8 Hz), 7.87-7.90 (2H, m), 8.13 (1H, brd, J=9.6 Hz), 9.39 (1H, s).
ESI-MS Found: m/z 431[M+H]⁺

### Example 60:

### 1- {2-[(Ethylamino)methyl]quinazolin-6-yl}-4-[(4-fluorobenzyl)oxy]pyridin-2(1H)-one:

In the same manner as in Example 21 but using the compound (40.0 mg) obtained in Example 58-1) and a THF solution (2 mL) of 2 M ethylamine, the entitled compound (17.6 mg) was obtained.
¹H-NMR (400 MHz, CDCl₃, δ ppm): 1.19 (3H, t, J=7.2 Hz), 2.77 (2H, q, J=7.2 Hz), 4.23 (2H, s), 5.00 (2H, s), 6.05 (1H, d, J=2.7 Hz), 6.10 (1H, dd, J=7.6, 2.7 Hz), 7.07 (1H, d, J=8.6 Hz), 7.09 (1H, d, J=8.6 Hz), 7.31 (1H, d, J=7.6 Hz), 7.38 (1H, d, J=8.6 Hz), 7.39 (1H, d, J=8.6 Hz), 7.89-7.91 (2H, m), 8.08 (1H, brd, J=9.8 Hz), 9.36 (1H, s).
ESI-MS Found: m/z 405[M+H]⁺

### Example 61:

### 4-[(4-Fluorobenzyl)oxy]-1-{2-[(propylamino)methyl]quinazolin-6-yl}pyridin-2(1H)-one:

In the same manner as in Example 21 but using the compound (40.0 mg) obtained in Example 58-1) and propylamine (500 µL), the entitled compound (14.9 mg) was obtained.
¹H-NMR (400 MHz, CDCl₃, δ ppm): 0.93 (3H, t, J=7.4 Hz), 1.59 (2H, dt, J=7.4, 7.3 Hz), 2.67 (2H, t, J=7.3 Hz), 4.23 (2H, s), 5.00 (2H, s), 6.05 (1H, d, J=2.7 Hz), 6.10 (1H, dd, J=7.6, 2.7 Hz), 7.07 (1H, d, J=8.6 Hz), 7.09 (1H, d, J=8.6 Hz), 7.31 (1H, d, J=7.6 Hz), 7.37 (1H, d, J=8.6 Hz), 7.39 (1H, d, J=8.6 Hz), 7.88-7.91 (2H, m), 8.08 (1H, brd, J=9.6 Hz), 9.36 (1H, s).
ESI-MS Found: m/z 419[M+H]⁺

### Example 62:

### 4-[(4-Fluorobenzyl)oxy]-1-{2-[(isopropylamino)methyl]quinazolin-6-yl}pyridin-2(1H)-one:

In the same manner as in Example 21 but using the compound (40.0 mg) obtained in Example 58-1) and isopropylamine (500 µL), the entitled compound (20.2 mg) was obtained. ¹H-NMR (400 MHz, CDCl₃, δ ppm): 1.15 (6H, d, J=6.3 Hz), 2.87-2.93 (1H, m), 4.24 (2H, s), 5.00 (2H, s), 6.05 (1H, d, J=2.7 Hz), 6.10 (1H, dd, J=7.6, 2.7 Hz), 7.07 (1H, d, J=8.6 Hz), 7.09 (1H, d, J=8.6 Hz), 7.31 (1H, d, J=7.6 Hz), 7.37 (1H, d, J=8.6 Hz), 7.39 (1H, d, J=8.6 Hz), 7.89-7.91 (2H, m), 8.07 (1H, brd, J=9.8 Hz), 9.35 (1H, s).
ESI-MS Found: m/z 419[M+H]⁺

### Example 63:

### 4-[(5-Chloropyridin-2-yl)methoxy]-1-[3-methyl-2-(3-pyrrolidin-1-ylpropyl)imidazo[1,2-a]pyridin-6-yl]pyridin-2(1H)-one:

1) In the same manner as in Example 30-1) but using the compound (16.0 mg) obtained in Production Example 29-9, 3- {6-[4-[(5-chloropyridin-2-yl)methoxy]-2-oxopyridin-1(2H)-yl]-3-methylimidazo[1,2-a]pyridin-2-yl}propyl methanesulfonate was obtained.
2) In the same manner as in Example 21 but using the compound obtained in Example 63-1) and pyrrolidine (2 mL), the entitled compound (2.8 mg) was obtained.
   ¹H-NMR (400 MHz, CDCl₃, δ ppm): 1.82-1.85 (4H, brm), 2.03-2.05 (2H, brm), 2.40 (3H, s), 2.61-2.67 (6H, m), 2.82 (2H, t, J=7.4 Hz), 5.16 (2H, s), 6.04 (1H, d, J=2.7 Hz), 6.14 (1H, dd, J=7.8, 2.7 Hz), 7.07 (1H, dd, J=9.4, 1.8 Hz), 7.28 (1H, d, J=7.8 Hz), 7.43 (1H, d, J=8.4 Hz), 7.58 (1H, d, J=9.4 Hz), 7.73 (1H, dd, J=8.4, 2.5 Hz), 7.93 (1H, d, J=1.8 Hz), 8.58 (1H, d, J=2.5 Hz).
   ESI-MS Found: m/z 478[M+H]⁺

### Example 64:

### 4-[(5-Chloropyridin-2-yl)methoxy]-1-[3-methyl-2-(pyrrolidin-1-ylmethyl)imidazo [1,2-a]pyridin-6-yl]pyridin-2(1H)-one:

1) In the same manner as in Example 29-9 but using the compound (90.0 mg) obtained in Production Example 30-1-3), 4-[(5-chloropyridin-2-yl)methoxy]-1-[2-(hydroxymethyl)-3-methylimidazo[1,2-a]pyridin-6-yl]pyridin-2(1H)-one (2.6 mg) was obtained.
2) In the same manner as in Example 30-1) but using the compound (2.6 mg) obtained in Example 64-1), {6-[4-[(5-chloropyridin-2-yl)methoxy]-2-oxopyridin-1(2H)-yl]-3-methylimidazo[1,2-a]pyridin-2-yl}methylmethanesulfonate was obtained.
3) In the same manner as in Example 21 but using the compound obtained in Example 64-2) and pyrrolidine (1 mL), the entitled compound (3.0 mg) was obtained.
   ¹H-NMR (400 MHz, CDCl₃, δ ppm): 1.78-1.81 (4H, brm), 2.50 (3H, s), 2.60-2.62 (4H, brm), 3.84 (2H, s), 5.19 (2H, s), 6.07 (1H, d, J=2.7 Hz), 6.17 (1H, dd, J=7.8, 2.7 Hz), 7.10 (1H, dd, J=9.4, 1.8 Hz), 7.30 (1H, d, J=7.8 Hz), 7.46 (1H, d, J=8.4 Hz), 7.65 (1H, d, J=9.4 Hz), 7.76 (1H, dd, J=8.4, 2.5 Hz), 7.99 (1H, d, J=1.8 Hz), 8.61 (1H, d, J=2.5 Hz).
   ESI-MS Found: m/z 450[M+H]⁺

### Example 65:

### 4-[(4-Chlorobenzyl)oxy]-1-[1-methyl-2-(3-pyrrolidin-1-ylpropyl)-1H-benzimidazol-6-yl]pyridin-2(1H)-one:

1) In a nitrogen atmosphere at 0°C, lithiumaluminium hydride (1.4 mg) was added to a THF solution (4 mL) of the compound (8.9 mg) obtained in Production Example 31-3. After stirred for 1 hour, sodium sulfate 10-hydrate was added to the reaction liquid, and further stirred at room temperature for 1 hour. The insoluble matter was separated by filtration, and the filtrate was concentrated under reduced pressure. The obtained residue was purified by silica gel column chromatography (chloroform:methanol = 10:1) to obtain 4-[(4-chlorobenzyl)oxy]-1-[2-(3-hydroxypropyl)-1-methyl-1H-benzimidazol-6-yl]pyridin-2(1H)-one(5.7 mg).
2) In the same manner as in Example 30-1) but using the compound (5.7 mg) obtained in Example 65-1), 3-{6-[4-[(4-chlorobenzyl)oxy]-2-oxopyridin-1(2H)-yl]-1-methyl-1H-benzimidazol-2-yl}propyl methanesulfonate was obtained.
3) In the same manner as in Example 21 but using the compound obtained in Example 65-2) and pyrrolidine (1 mL), the entitled compound (4.5 mg) was obtained.
   ¹H-NMR (400 MHz, CDCl₃, δ ppm): 1.73-1.76 (4H, brm), 2.02-2.09 (2H, m), 2.48-2.50 (4H, brm), 2.54 (2H, t, J=7.2 Hz), 2.94 (2H, t, J=7.6 Hz), 3.71 (3H, s), 4.99 (2H, s), 6.00-6.03 (2H, m), 7.09 (1H, dd, J=8.2, 2.0 Hz), 7.27-7.37 (6H, m), 7.72 (1H, d, J=8.2 Hz).
   ESI-MS Found: m/z 478[M+H]⁺

### Example 66:

### 4-[(5-Chloropyridin-2-yl)methoxy]-1-[1-methyl-2-(3-pyrrolidin-1-ylpropyl)-1H-benzimidazol-6-yl]pyridin-2(1H)-one:

1) In the same manner as in Example 65-1 but using the compound (48.0 mg) obtained in Production Example 32-2, 4-[(5-chloropyridin-2-yl)methoxy]-1-[2-(3-hydroxypropyl)-1-methyl-1H-benzimidazol-6-yl]pyridin-2(1H)-one (14.8 mg) was obtained.
2) In the same manner as in Example 30-1) but using the compound (14.8 mg) obtained in Example 66-1), 3-{6-[4-[(5-chloropyridin-2-yl)methoxy]-2-oxopyridin-1(2H)-yl]-1-methyl-1H-benzimidazol-2-yl}propyl methanesulfonate was obtained.
3) In the same manner as in Example 21 but using the compound obtained in Example 66-2) and pyrrolidine (1 mL), the entitled compound (13.7 mg) was obtained.
   ¹H-NMR (400 MHz, CDCl₃, δ ppm): 1.73-1.76 (4H, brm), 2.01-2.09 (2H, m), 2.47-2.49 (4H, brm), 2.54 (2H, t, J=7.2 Hz), 2.94 (2H, t, J=7.6 Hz), 3.71 (3H, s), 5.13 (2H, s), 6.02 (1H, d, J=2.7 Hz), 6.07 (1H, dd, J=7.8, 2.7 Hz), 7.08 (1H, dd, J=8.6, 2.0 Hz), 7.29-7.32 (2H, m), 7.41 (1H, d, J=8.2 Hz), 7.69-7.73 (2H, m), 8.55 (1H, d, J=2.0 Hz).
   ESI-MS Found: m/z 478[M+H]⁺

### Example 67:

### 4-[(4-Chlorobenzyl)oxy]-1-[1-methyl-2-(2-pyrrolidin-1-ylethoxy)-1H-benzimidazol-6-yl]pyridin-2(1H)-one:

In the same manner as in Example 57 but using the crude product obtained in Production Example 33-3 and 4-[(4-chlorobenzyl)oxy]pyridin-2(1H)-one (35.4 mg), the entitled compound (2.5 mg) was obtained.
¹H-NMR (400 MHz, CDCl₃, δ ppm): 1.77-1.80 (4H, brm), 2.61-2.64 (4H, brm), 2.94-2.97 (2H, brm), 3.53 (3H, s), 4.67 (2H, t, J=5.5 Hz), 4.98 (2H, s), 5.99-6.01 (2H, m), 7.02 (1H, d, J=8.2 Hz), 7.17 (1H, d, J=2.0 Hz), 7.26 (1H, d, J=7.0 Hz), 7.31-7.37 (4H, m), 7.53 (1H, d, J=8.2 Hz). ESI-MS Found: m/z 480[M+H]⁺

### Example 68:

### 4-[(E)-2-(5-chloropyridin-2-yl)vinyl]-1-[1-methyl-2-(2-pyrrolidin-2-ylethoxy)-1H-benzimidazol-6-yl]pyridin-2(1H)-one:

In the same manner as in Example 57 but using the crude product obtained in Production Example 33-3 and 4-[(E)-2-(5-chloropyridin-2-yl)vinyl]pyridin-2(1H)-one (34.9 mg), the entitled compound (15.5 mg) was obtained.
¹H-NMR (400 MHz, CDCl₃, δ ppm): 1.77-1.80 (4H, brm), 2.61-2.63 (4H, brm), 2.94-2.97 (2H, brm), 3.54 (3H, s), 4.67 (2H, t, J=5.7 Hz), 6.47 (1H, d, J=7.4 Hz), 6.71 (1H, s), 7.06 (1H, dd, J=8.2, 2.0 Hz), 7.16 (1H, d, J=16.0 Hz), 7.34-7.43 (4H, m), 7.56 (1H, d, J=8.2 Hz), 7.66 (1H, dd, J=8.4, 2.5 Hz), 8.56 (1H, d, J=2.5 Hz).
ESI-MS Found: m/z 476[M+H]⁺

### Example 69:

### 4-[(5-Chloropyridin-2-yl)methoxy]-1-{2-[(diethylamino)methyl]-1-methyl-1H-indol-6-yl}pyridin-2(1H)-one:

In the same manner as in Example 23 but using the compound (20.0 mg) obtained in Production Example 34-7 and diethylamine (27.6 µL), the entitled compound (15.0 mg) was obtained.
¹H-NMR (400 MHz, CDCl₃, δ ppm): 0.99 (6H, t, J=7.1 Hz), 2.49 (4H, q, J=7.1 Hz), 3.65 (2H, s), 3.76 (3H, s), 5.13 (2H, s), 6.02-6.06 (2H, m), 6.35 (1H, s), 6.94 (1H, dd, J=8.2, 2.0 Hz), 7.26 (1H, s), 7.31 (1H, d, J=7.4 Hz), 7.41 (1H, d, J=8.2 Hz), 7.55 (1H, d, J=8.6 Hz), 7.70 (1H, dd, J=8.6, 2.0 Hz), 8.55 (1H, d, J=2.0 Hz).
ESI-MS Found: m/z 451[M+H]⁺

### Example 70:

### 4-[(5-Chloropyridin-2-yl)methoxy]-1-[1-methyl-2-(pyrrolidin-1-ylmethyl)-1H-indol-6-yl]pyridin-2(1H)-one:

In the same manner as in Example 23 but using the compound (80 mg) obtained in Production Example 34-7 and pyrrolidine (50 µL), the entitled compound (56.0 mg) was obtained.
¹H-NMR (400 MHz, CDCl₃, δ ppm): 1.72-1.74 (4H, brm), 2.47-2.49 (4H, brm), 3.72 (2H, s), 3.75 (3H, s), 5.13 (2H, s), 6.02-6.06 (2H, m), 6.35 (1H, s), 6.94 (1H, dd, J=8.4, 1.8 Hz), 7.26 (1H, s), 7.31 (1H, d, J=7.4 Hz), 7.41 (1H, d, J=8.6 Hz), 7.56 (1H, d, J=8.2 Hz), 7.70 (1H, dd, J=8.4, 2.5 Hz), 8.55 (1H, d, J=2.5 Hz).
ESI-MS Found: m/z 449[M+H]⁺

### Example 71:

### 4-[(5-Chloropyridin-2-yl)methoxy]-1-[1-methyl-2-(3-pyrrolidin-1-ylpropyl)-1H-indol-6-yl]pyridin-2(1H)-one:

1) In the same manner as in Example 30-1) but using the compound (11.3 mg) obtained in Production Example 35-3, 3- {6-[4-[(5-chloropyridin-2-yl)methoxy]-2-oxopyridin-1(2H)-yl]-1-methyl-1H-indol-2-yl}propyl methanesulfonate was obtained.
2) In the same manner as in Example 21 but using the compound obtained in Example 71-1) and pyrrolidine (1 mL), the entitled compound (9.8 mg) was obtained.
   ¹H-NMR (400 MHz, CDCl₃, δ ppm): 1.75-1.77 (4H, brm), 1.87-1.95 (2H, m), 2.49-2.54 (6H, brm), 2.77 (2H, t, J=7.6 Hz), 3.63 (3H, s), 5.13 (2H, s), 6.01-6.05 (2H, m), 6.26 (1H, s), 6.93 (1H, dd, J=8.2, 2.0 Hz), 7.23 (1H, s), 7.30 (1H, d, J=7.4 Hz), 7.41 (1H, d, J=8.6 Hz), 7.53 (1H, d, J=8.2 Hz), 7.70 (1H, dd, J=8.4, 2.5 Hz), 8.55 (1H, d, J=2.5 Hz).
   ESI-MS Found: m/z 478[M+H]⁺

### Example 72:

### 4-(Benzyloxy)-1-[2-(2-pyrrolidin-1-ylethyl)quinolin-6-yl]pyridin-2(1H)-one:

Pyrrolidine (0.5 mL) was added to a DMF solution (1 mL) of the compound (60 mg) obtained in Production Example 36-3, and stirred at 60°C for 2 hours. Water was added to the reaction liquid, then extracted with chloroform, and the organic layer was dried with anhydrous sodium sulfate. The organic layer was concentrated under reduced pressure, and the obtained residue was purified by silica gel column chromatography (chloroform:methanol = 80:1 to 8:1) to obtain the entitled compound (49 mg) as a colorless solid.
¹H-NMR (400 MHz, CDCl₃, δ ppm): 1.80-1.84 (4H, m), 2.61-2.67 (4H, m), 2.99 (2H, t, J=8.0 Hz), 3.25 (2H, t, J=8.0 Hz), 5.07 (2H, s), 6.10 (1H, s), 6.12 (1H, dd, J=6.8, 2.4 Hz), 7.32-7.44 (7H, m), 7.68 (1H, dd, J=8.8, 2.4 Hz), 7.77 (1H, d, J=2.4 Hz), 8.08 (1H, d, J=8.0 Hz), 8.12 (1H, d, J=8.8 Hz)
ESI-MS Found: m/z 426[M+H]⁺

### Example 73:

### 4-(Benzyloxy)-1-{2-[2-(isopropylamino)ethyl]quinolin-6-yl}pyridin-2(1H)-one:

In the same manner as in Example 72 but using the compound (60 mg) obtained in Production Example 36-3 and isopropylamine (0.5 mL), the entitled compound (34 mg) was obtained as a colorless solid.
¹H-NMR (400 MHz, CDCl₃, δ ppm): 1.09 (6H, d, J=6.4 Hz), 2.85-2.91 (1H, m), 3.10-3.15 (2H, m), 3.18-3.22 (2H, m), 5.07 (2H, s), 6.10 (1H, s), 6.12 (1H, dd, J=8.0, 3.2 Hz), 7.32-7.44 (7H, m), 7.69 (1H, dd, J=8.8, 2.4 Hz), 7.78 (1H, d, J=2.0 Hz), 8.09 (1H, d, J=8.8 Hz), 8.11 (1H, d, J=8.8 Hz)
ESI-MS Found: m/z 414[M+H]⁺

### Example 74:

### 4-(Benzyloxy)-1-{2-[2-(methylamino)ethyl]quinolin-6-yl}pyridin-2(1H)-one:

In the same manner as in Example 72 but using the compound (50 mg) obtained in Production Example 36-3 and a methanol solution (1 mL) of 40 % methylamine, the entitled compound (23 mg) was obtained as a colorless solid.
¹H-NMR (400 MHz, CDCl₃, δ ppm): 2.49 (3H, s), 3.09 (2H, t, J=6.8 Hz), 3.20 (2H, t, J=6.8 Hz), 5.07 (2H, s), 6.10 (1H, s), 6.12 (1H, dd, J=8.0, 3.2 Hz), 7.32-7.44 (7H, m), 7.69 (1H, dd, J=8.8, 2.4 Hz), 7.78 (1H, d, J=2.4 Hz), 8.08 (1H, d, J=8.0 Hz), 8.12 (1H, d, J=9.2 Hz)
ESI-MS Found: m/z 386[M+H]⁺

### Example 75:

### 4-(Benzyloxy)-1-{2-[2-(ethylamino)ethyl]quinolin-6-yl}pyridin-2(1H)-one:

In the same manner as in Example 72 but using the compound (50 mg) obtained in Production Example 36-3 and an aqueous solution (0.5 mL) of 70 % ethylamine, the entitled compound (19 mg) was obtained as a colorless solid.
¹H-NMR (400 MHz, CDCl₃, δ ppm): 1.12 (3H, t, J=7.2 Hz), 2.71 (2H, q, J=7.2 Hz), 3.11-3.15 (2H, m), 3.18-3.22 (2H, m), 5.07 (2H, s), 6.10 (1H, s), 6.12 (1H, dd, J=8.0, 3.2 Hz), 7.32-7.44 (7H, m), 7.69 (1H, dd, J=8.8, 2.4 Hz), 7.78 (1H, d, J=2.4 Hz), 8.09 (1H, d, J=8.0 Hz), 8.12 (1H, d, J=8.8 Hz)
ESI-MS Found: m/z 400[M+H]⁺

### Example 76:

### 4-(Benzyloxy)-1-{2-[2-(propylamino)ethyl]quinolin-6-yl}pyridin-2(1H)-one:

In the same manner as in Example 72 but using the compound (50 mg) obtained in Production Example 36-3 and n-propylamine (0.5 mL), the entitled compound (21 mg) was obtained as a colorless solid.
¹H-NMR (400 MHz, CDCl₃, δ ppm): 0.92 (3H, t, J=7.2 Hz), 1.47-1.56 (2H, m), 2.64 (2H, t, J=7.2 Hz), 3.09-3.13 (2H, m), 3.18-3.22 (2H, m), 5.07 (2H, s), 6.10 (1H, s), 6.11 (1H, dd, J=7.2, 2.8 Hz), 7.32-7.44 (7H, m), 7.69 (1H, dd, J=8.8, 2.4 Hz), 7.78 (1H, d, J=2.4 Hz), 8.08 (1H, d, J=8.8 Hz), 8.12 (1H, d, J=8.8 Hz)
ESI-MS Found: m/z 414[M+H]⁺

### Example 77:

### 1-[2-(2-Azetidin-1-ylethyl)quinolin-6-yl]-4-(benzyloxy)pyridin-2(1H)-one:

In the same manner as in Example 72 but using the compound (50 mg) obtained in Production Example 36-3, azetidine hydrochloride (100 mg) and triethylamine (150 µL), the entitled compound (21 mg) was obtained as a colorless solid.
¹H-NMR (400 MHz, CDCl₃, δ ppm): 2.06-2.14 (2H, m), 2.94-2.98 (2H, m), 3.05-3.08 (2H, m)3.28 (4H, t, J=6.8 Hz), 5.07 (2H, s), 6.10 (1H, s), 6.12 (1H, dd, J=6.8, 2.4 Hz), 7.32-7.44 (7H, m), 7.68 (1H, dd, J=9.2, 2.4 Hz), 7.77 (1H, d, J=2.0 Hz), 8.07 (1H, d, J=8.8 Hz), 8.11 (1H, d, J=8.8 Hz)
ESI-MS Found: m/z 412[M+H]⁺

### Example 78:

### 1- {2-[2-(ethylamino)ethyl]quinolin-6-yl}-4-[(4-fluorobenzyl)oxy]pyridin-2(1H)-one:

In the same manner as in Example 72 but using the compound (15 mg) obtained in Production Example 37-5 and an aqueous solution (0.5 mL) of 70 % ethylamine, the entitled compound (5 mg) was obtained as a colorless solid.
¹H-NMR (400 MHz, CDCl₃, δ ppm): 1.28 (3H, t, J=7.2 Hz), 2.93 (2H, q, J=7.2 Hz), 3.30-3.35 (4H, m), 5.04 (2H, s), 6.09-6.13 (2H, m), 7.10 (1H, d, J=8.3 Hz), 7.13 (1H, d, J=8.3 Hz), 7.33 (1H, d, J=8.3 Hz), 7.34 (1H, d, J=7.3 Hz), 7.41 (1H, d, J=8.3 Hz), 7.42 (1H, d, J=8.3 Hz), 7.67 (1H, dd, J=8.8, 2.4 Hz), 7.76 (1H, d, J=2.4 Hz), 8.02 (1H, d, J=8.4 Hz), 8.05 (1H, d, J=8.4 Hz) ESI-MS Found: m/z 418[M+H]⁺

### Example 79:

### 4-[(4-Fluorobenzyl)oxy]-1-{2-[2-(propylamino)ethyl]quinolin-6-yl}pyridin-2(1H)-one:

In the same manner as in Example 72 but using the compound (15 mg) obtained in Production Example 37-5 and n-propylamine (0.5 mL), the entitled compound (5 mg) was obtained as a colorless solid.
¹H-NMR (400 MHz, CDCl₃, δ ppm): 1.05 (3H, t, J=7.2 Hz), 1.72-1.79 (2H, m), 2.91 (2H, t, J=7.2 Hz), 3.37 (4H, s), 5.04 (2H, s), 6.09-6.15 (2H, m), 7.11 (1H, d, J=8.8 Hz), 7.13 (1H, d, J=8.8 Hz), 7.31 (1H, d, J=8.8 Hz), 7.34 (1H, d, J=7.3 Hz), 7.41 (1H, d, J=8.8 Hz), 7.43 (1H, d, J=8.8 Hz), 7.66 (1H, dd, J=8.8, 2.4 Hz), 7.74 (1H, d, J=2.4 Hz), 7.96 (1H, d, J=8.8 Hz), 8.03 (1H, d, J=8.0 Hz)
ESI-MS Found: m/z 432[M+H]⁺

### Example 80:

### 4-[(4-Fluorobenzyl)oxy]-1- {2-[2-(isopropylamino)ethyl]quinolin-6-yl}pyridin-2(1H)-one:

In the same manner as in Example 72 but using the compound (15 mg) obtained in Production Example 37-5 and isopropylamine (0.5 mL), the entitled compound (6 mg) was obtained as a colorless solid.
¹H-NMR (400 MHz, CDCl₃, δ ppm): 1.30 (6H, d, J=6.4 Hz), 3.16-3.24 (1H, m), 3.37 (4H, s), 5.03 (2H, s), 6.08-6.13 (2H, m), 7.10 (1H, d, J=8.8 Hz), 7.12 (1H, d, J=8.8 Hz), 7.33-7.44 (4H, m), 7.70 (1H, dd, J=8.8, 2.4 Hz), 7.78 (1H, s), 8.05 (1H, d, J=8.8 Hz), 8.09 (1H, d, J=8.4 Hz) ESI-MS Found: m/z 432[M+H]⁺

### Example 81:

### 4-[(4-Fluorobenzyl)oxy]-1-[2-(2-{[(1R)-1-methylpropyl]amino}ethyl)quinolin-6-yl]pyridin-2(1H)-one:

In the same manner as in Example 72 but using the compound (15 mg) obtained in Production Example 37-5 and (2R)-butan-2-amine (0.07 mL), the entitled compound (5 mg) was obtained as a colorless solid.
¹H-NMR (400 MHz, CDCl₃, δ ppm): 0.98 (3H, t, J=7.2 Hz), 1.28 (3H, d, J=6.4 Hz), 1.54-1.62 (1H, m), 1.75-1.83 (1H, m), 2.96-3.03 (1H, m), 3.32-3.45 (4H, m), 4.99 (2H, s), 6.05-6.09 (2H, m), 7.07 (1H, d, J=8.6 Hz), 7.09 (1H, d, J=8.6 Hz), 7.30 (1H, d, J=7.4 Hz), 7.32 (1H, d, J=8.4 Hz), 7.37 (1H, d, J=8.6 Hz), 7.38 (1H, d, J=8.6 Hz), 7.67 (1H, dd, J=8.8, 2.4 Hz), 7.74 (1H, d, J=2.4 Hz), 7.99 (1H, d, J=8.8 Hz), 8.04 (1H, d, J=8.8 Hz)
ESI-MS Found: m/z 446[M+H]⁺

### Example 82:

### 4-[(4-Fluorobenzyl)oxy]-1-[2-(2-{[(1S)-1-methylpropyl]amino}ethyl)quinolin-6-yl]pyridin-2(1H)-one:

In the same manner as in Example 72 but using the compound (15 mg) obtained in Production Example 37-5 and (2S)-butan-2-amine, the entitled compound (5 mg) was obtained as a colorless solid.
¹H-NMR (400 MHz, CDCl₃, δ ppm): 1.02 (3H, t, J=7.2 Hz), 1.34 (3H, d, J=6.4 Hz), 1.60-1.68 (1H, m), 1.82-1.90 (1H, m), 3.04-3.10 (1H, m), 3.35-3.50 (4H, m), 5.03 (2H, s), 6.08-6.12 (2H, m), 7.10 (1H, d, J=8.8 Hz), 7.12 (1H, d, J=8.8 Hz), 7.33-7.37 (2H, m), 7.41 (1H, d, J=8.8 Hz), 7.42 (1H, d, J=8.8 Hz), 7.71 (1H, dd, J=8.8, 2.4 Hz), 7.77 (1H, d, J=2.4 Hz), 8.02 (1H, d, J=8.8 Hz), 8.08 (1H, d, J=8.8 Hz)
ESI-MS Found: m/z 446[M+H]⁺

### Example 83:

### 4-[(5-Chloropyridin-2-yl)methoxy]-1-{2-[2-(propylamino)ethyl]quinolin-6-yl}pyridin-2(1H)-one:

In the same manner as in Example 72 but using the compound (30 mg) obtained in Production Example 38-3 and n-propylamine (0.5 mL), the entitled compound (22 mg) was obtained as a colorless solid.
¹H-NMR (400 MHz, CDCl₃, δ ppm): 0.95 (3H, t, J=7.6 Hz), 1.55-1.63 (2H, m), 2.72 (2H, t, J=7.6 Hz), 3.17-3.22 (2H, m), 3.24-3.28 (2H, m), 5.18 (2H, s), 6.07 (1H, d, J=2.8 Hz), 6.16 (1H, dd, J=7.6, 2.8 Hz), 7.36 (1H, d, J=7.3 Hz), 7.37 (1H, d, J=8.3 Hz), 7.45 (1H, d, J=8.4 Hz), 7.69 (1H, dd, J=8.4, 2.4 Hz), 7.74 (1H, dd, J=8.4, 2.4 Hz), 7.79 (1H, d, J=2.4 Hz), 8.09 (2H, d, J=8.4 Hz), 8.59 (1H, d, J=2.0 Hz)
ESI-MS Found: m/z 449, 451[M+H]⁺

### Example 84:

### 4-[(5-Chloropyridin-2-yl)methoxy]-1- {2-[2-(isopropylamino)ethyl]quinolin-6-yl}pyridin-2(1H)-one:

In the same manner as in Example 72 but using the compound (30 mg) obtained in Production Example 38-3 and isopropylamine (0.5 mL), the entitled compound (15 mg) was obtained as a colorless solid.
¹H-NMR (400 MHz, CDCl₃, δ ppm): 1.16 (6H, d, J=6.0 Hz), 2.96-3.02 (1H, m), 3.17-3.22 (2H, m), 3.24-3.28 (2H, m), 5.18 (2H, s), 6.07 (1H, d, J=2.4 Hz), 6.16 (1H, dd, J=7.6, 2.4 Hz), 7.36 (1H, d, J=7.8 Hz), 7.37 (1H, d, J=8.3 Hz), 7.45 (1H, d, J=8.4 Hz), 7.69 (1H, dd, J=8.4, 2.4 Hz), 7.74 (1H, dd, J=8.4, 2.4 Hz), 7.78 (1H, d, J=2.4 Hz), 8.09 (1H, d, J=9.3 Hz), 8.10 (1H, d, J=8.3 Hz), 8.59 (1H, d, J=2.4 Hz)
ESI-MS Found: m/z 449, 451[M+H]⁺

### Example 85:

### 1-[2-(2-Azetidin-1-ylethyl)quinolin-6-yl]-4-[(5-chloropyridin-2-yl)methoxy]pyridin-2(1H)-one:

In the same manner as in Example 72 but using the compound (30 mg) obtained in Production Example 38-3 and azetidine hydrochloride (100 mg) and triethylamine (150 µL), the entitled compound (18 mg) was obtained as a colorless solid.
¹H-NMR (400 MHz, CDCl₃, δ ppm): 2.13 (2H, t, J=7.2 Hz), 2.98-3.03 (2H, m), 3.06-3.12 (2H, m), 3.33 (4H, t, J=7.2 Hz), 5.18 (2H, s), 6.07 (1H, d, J=2.4 Hz), 6.15 (1H, dd, J=7.6, 2.4 Hz), 7.35 (1H, d, J=8.0 Hz), 7.38 (1H, d, J=8.8 Hz), 7.45 (1H, d, J=8.8 Hz), 7.68 (1H, dd, J=8.8, 2.4 Hz), 7.74 (1H, dd, J=8.8, 2.4 Hz), 7.77 (1H, d, J=2.4 Hz), 8.07 (1H, d, J=8.8 Hz), 8.11 (1H, d, J=8.8 Hz), 8.59 (1H, d, J=2.0 Hz)
ESI-MS Found: m/z 447, 449[M+H]⁺

### Example 86:

### 4-[(5-Chloropyridin-2-yl)methoxy]-1-[2-(2-pyrrolidin-1-ylethyl)quinolin-6-yl]pyridin-2(1H)-one:

In the same manner as in Example 72 but using the compound (30 mg) obtained in Production Example 38-3 and pyrrolidine (0.5 mL), the entitled compound (24 mg) was obtained as a colorless solid.
¹H-NMR (400 MHz, CDCl₃, δ ppm): 1.80-1.86 (4H, m), 2.63-2.69 (4H, m), 3.00-3.06 (2H, m), 3.23-3.29 (2H, m), 5.18 (2H, s), 6.07 (1H, d, J=2.8 Hz), 6.15 (1H, dd, J=7.6, 2.8 Hz), 7.35 (1H, d, J=8.0 Hz), 7.40 (1H, d, J=8.4 Hz), 7.45 (1H, d, J=8.0 Hz), 7.67 (1H, dd, J=8.8, 2.4 Hz), 7.74 (1H, dd, J=8.8, 2.4 Hz), 7.78 (1H, d, J=2.4 Hz), 8.08 (1H, d, J=8.8 Hz), 8.12 (1H, d, J=8.8 Hz), 8.59 (1H, d, J=2.4 Hz)
ESI-MS Found: m/z 461, 463[M+H]⁺

### Example 87:

### 4-[(4-Fluorobenzyl)oxy]-1-[2-(pyrrolidin-1-ylmethyl)-1-benzofuran-5-yl]pyridin-2(1H)-one:

Potassium carbonate (532 mg) and copper(I) iodide (210 mg) were added to a DMF solution (10 mL) of the compound (600 mg) obtained in Production Example 39-4 and 4-(fluorobenzyl)oxy-2(1H)-pyridone (402 mg), and stirred at 150°C for 17 hours in a nitrogen atmosphere. Ethyl acetate and water were added to the reaction liquid, then stirred, and filtered through Celite. The filtrate was subjected to liquid-liquid separation, and the organic layer was washed with saturated saline water. The organic layer was dried with anhydrous sodium sulfate, then the solvent was evaporated off under reduced pressure. The obtained residue was purified by reversed-phase HPLC (YMC-Pack ODS-AQ, acetonitrile (containing 0.1 % TFA):water (containing 0.1 % TFA) (5:95 to 75:25) and then desalted to obtain the entitled compound (235 mg, 31 %) as a colorless solid.
¹H-NMR (400 MHz, DMSO-d₆, δ ppm): 1.66-1.74 (4H, m), 2.48-2.56 (4H, m), 3.77 (2H, s), 5.11 (2H, s), 5.98 (1H, d, J=2.5 Hz), 6.07 (1H, dd, J=7.6, 2.7 Hz), 6.79 (1H, s), 7.19 (1H, dd, J=8.6, 2.2 Hz), 7.21-7.29 (2H, m), 7.48-7.56 (3H, m), 7.57-7.64 (2H, m).
ESI-MS Found: m/z 419[M+H]⁺

### Example 88:

### 4-[(4-Fluorobenzyl)oxy]-1-[2-(piperidin-1-ylmethyl)-1-benzofuran-5-yl]pyridin-2(1H)-one:

Piperidine (0.10 mL) was added to a dimethyl sulfoxide solution (0.90 mL) of the compound (30 mg) obtained in Production Example 40-5, and stirred in a nitrogen atmosphere at room temperature for 16 hours. The reaction liquid was purified by reversed-phase HPLC (YMC-Pack ODS-AQ, acetonitrile (containing 0.1 % TFA):water (containing 0.1 % TFA) (5:95 to 75:25) and then desalted to obtain the entitled compound (13 mg) as a colorless solid.
¹H-NMR (400 MHz, CDCl₃, δ ppm): 1.38-1.70 (6H, m), 2.42-2.54 (4H, m), 3.68 (2H, s), 5.01 (2H, s), 6.04 (1H, dd, J=7.6, 2.7 Hz), 6.07 (1H, d, J=2.7 Hz), 6.61 (1H, s), 7.06-7.15 (2H, m), 7.19 (1H, dd, J=8.6, 2.2 Hz), 7.25-7.30 (1H, m), 7.36-7.45 (2H, m), 7.49 (1H, d, J=2.2 Hz), 7.54 (1H, d, J=8.6 Hz).
ESI-MS Found: m/z 433[M+H]⁺

### Example 89:

### 1-[2-(Azepan-1-ylmethyl)-1-benzofuran-5-yl]-4-[(4-fluorobenzyl)oxy]pyridin-2(1H)-one:

In the same manner as in Example 88 but using the compound (30 mg) obtained in Production Example 40-5 and hexamethyleneimine (0.10 mL), the entitled compound (15 mg) was obtained as a colorless solid.
¹H-NMR (400 MHz, CDCl₃, δ ppm): 1.55-1.75 (8H, m), 2.66-2.82 (4H, m), 3.68 (2H, s), 5.01 (2H, s), 6.04 (1H, dd, J=7.4, 2.5 Hz), 6.07 (1H, d, J=2.5 Hz), 6.61 (1H, s), 7.06-7.15 (2H, m), 7.18-7.22 (1H, m), 7.25-7.30 (1H, m), 7.37-7.45 (2H, m), 7.49 (1H, d, J=1.8 Hz), 7.53 (1H, d, J=8.6 Hz).ESI-MS Found: m/z 447[M+H]⁺

### Example 90:

### 4-[2-(4-fluorophenoxy)ethoxy]-1-{2-[isopropyl(methyl)amino]-1H-benzimidazol-6-yl}pyridin-2(1H)-one:

Potassium carbonate (60 mg) and 1-(2-bromoethoxy)-4-fluorobenzene (60 mg) were added to a DMF solution (5 mL) of the compound (60 mg) obtained in Production Example 41-2, and stirred overnight at room temperature. Ethyl acetate was added to the reaction liquid, washed successively with water and saturated saline water, and dried with anhydrous magnesium sulfate. The solvent was evaporated off under reduced pressure, and the obtained residue was purified by silica gel column chromatography (NH silica gel, chloroform) to obtain the entitled compound (14.6 mg).
¹H-NMR (400 MHz, DMSO-d₆, δ ppm): 1.17 (6H, d, J=6.7 Hz), 2.90 (3H, s) 4.29-4.35 (4H, m) 4.49 (1H, septet, J=6.7Hz), 5.92 (1H, d, J=2.7 Hz), 6.03 (1H, dd, J=7.6 Hz, 2.7 Hz), 6.79 (1H, brs), 6.99-7.05 (3H, m), 7.11-7.19 (3H, m), 7.52 (1H, d, J=7.4 Hz), 11.32 (1H, brs).
ESI-MS Found: m/z 437[M+H]⁺

### Example 91:

### 1-{2-[Isopropyl(methyl)amino]-1H-benzimidazol-6-yl}-4-(2-phenoxyethoxy)pyridin-2(1H)-one:

In the same manner as in Example 90 but using the compound (50 mg) obtained in Production Example 41-2 and (2-bromoethoxy)benzene (40 mg), the entitled compound (17.2 mg) was obtained.
¹H-NMR (400 MHz, CDCl₃, δ ppm): 1.17 (6H, d, J=6.7 Hz), 2.77 (3H, s), 4.39 (4H, s), 4.44 (1H, septet, J=6.7 Hz), 6.09-6.14 (2H, m), 6.75 (1H, dd, J=8.2, 2.0 Hz), 6.98-7.06 (1H, m), 7.14-7.17 (1H, m), 7.33-7.38 (6H, m).
ESI-MS Found: m/z 419[M+H]⁺

### Example 92:

### 1- {2-[Isopropyl(methyl)amino]-1H-benzimidazol-6-yl}-4-(2-phenylethoxy)pyridin-2(1H)-one:

In the same manner as in Example 90 but using the compound (50 mg) obtained in Production Example 41-2 and (2-bromoethyl)benzene (27 µL), the entitled compound (12.5 mg) was obtained.
¹H-NMR (400 MHz, CDCl₃, δ ppm): 1.12 (6H, d, J=6.7 Hz), 2.69 (3H, s), 3.13 (2H, dd, J=7.0, 6.7 Hz), 4.22 (2H, dd, J=7.0, 6.7 Hz), 4.38 (1H, septet, J=6.7 Hz), 6.00-6.03 (2H, m), 6.68 (1H, dd, J=8.2, 2.0 Hz), 6.99 (1H, s), 7.10 (1H, s), 7.24-7.36 (6H, m).
ESI-MS Found: m/z 403[M+H]⁺

### Example 93:

### 4-[(4-Chlorobenzyl)oxy]-1-{2-[isopropyl(methyl)amino]-1H-benzimidazol-6-yl}pyridin-2(1H)-one:

In the same manner as in Example 90 but using the compound (50 mg) obtained in Production Example 41-2 and 1-chloro-4-(chloromethyl)benzene (25 µL), the entitled compound (15 mg) was obtained.
¹H-NMR (400 MHz, CDCl₃, δ ppm): 1.12 (6H, d, J=6.7 Hz), 2.71 (3H, s), 4.40 (1H, septet, J=6.7 Hz), 5.05 (2H, s), 6.06-6.11 (2H, m), 6.71 (1H, dd, J=8.3, 2.0 Hz), 7.01 (1H, s), 7.02-7.10 (1H, brs), 7.32-7.41 (5H, m).
ESI-MS Found: m/z 423[M+H]⁺

### Example 94:

### 4-[(4-Fluorobenzyl)oxy]-1-{[2-(pyrrolidin-1-ylmethyl)quinolin-6-yl]methoxy}pyridin-2(1H)-one:

In a nitrogen atmosphere, 4-[(4-fluorobenzyl)oxy]-1-hydroxypyridin-2(1H)-one (50.2 mg) and the compound (61.9 mg) obtained in Production Example 42-4 were dissolved in THF (1 mL), and with cooling with ice, tributyl phosphine (160 µL) and 1,1'-(azodicarbonyl)dipiperidine (80.6 mg) were successively added, and stirred at room temperature for 20 hours. After the reaction, ethyl acetate and aqueous 10 % hydrochloric acid solution were added to the reaction liquid, then the aqueous layer was made alkaline with aqueous saturated sodium hydrogencarbonate solution, and extracted with ethyl acetate. The organic layer was washed with saturated saline water, then dried with anhydrous magnesium sulfate, and the solvent was evaporated off under reduced pressure. The obtained residue was purified by reversed-phase HPLC (YMC-Pack™ PRO C-18, acetonitrile (containing 0.1 % TFA):water (containing 0.1 % TFA) (10:90 to 95:5) and then desalted to obtain the entitled compound (44.3 mg) as a colorless solid.
¹H-NMR (400 MHz, CDCl₃, δ ppm): 1.81-1.85 (4H, m), 2.60-2.67 (4H, m), 3.97 (2H, s), 4.92 (2H, s), 5.42 (2H, s), 5.65 (1H, dd, J=7.8, 3.4 Hz), 6.07 (1H, d, J=3.4 Hz), 7.00 (1H, d, J=7.8 Hz), 7.04-7.11 (2H, m), 7.33-7.36 (2H, m), 7.64 (1H, d, J=8.3 Hz), 7.77-7.80 (2H, m), 8.10 (2H, d, J=8.3 Hz).
ESI-MS Found: m/z 460[M+H]⁺

### Example 95:

### 1-[6-(Azetidin-1-ylmethyl)-1,5-naphthyridin-2-yl]-4-[(4-fluorobenzyl)oxy]pyridin-2(1H)-one:

In the same manner as in Example 23 but using the compound (16 mg) obtained in Production Example 43-3 and azetidine (60 µL), the entitled compound (3.5 mg) was obtained as a white solid.
¹H-NMR (400 MHz, DMSO-d₆, δ ppm): 2.03 (2H, t, J=7.0 Hz), 3.24 (4H, t, J=7.0 Hz), 3.86 (2H, s), 5.16 (2H, s), 6.06 (1H, d, J=2.9 Hz), 6.23 (1H, dd, J=7.8, 2.9 Hz), 7.25 (1H, d, J=8.8 Hz), 7.26 (1H, d, J=8.8 Hz), 7.52 (1H, d, J=8.8 Hz), 7.54 (1H, d, J=8.8 Hz), 7.82 (1H, d, J=8.8 Hz), 7.97 (1H, d, J=7.8 Hz), 8.05 (1H, d, J=9.3 Hz), 8.36 (1H, d, J=8.8 Hz), 8.45 (1H, d, J=9.3 Hz).
ESI-MS Found: m/z 417[M+H]⁺

### Example 96:

### 1- {6-[(Ethylamino)methyl]-1,5-naphthyridin-2-yl}-4-[(4-fluorobenzyl)oxy]pyridin-2(1H)-one:

In the same manner as in Example 23 but using the compound (20 mg) obtained in Production Example 43-3 and a methanol solution (100 µL) of 30 to 40 % ethylamine, the entitled compound (11.1 mg) was obtained as a white solid.
¹H-NMR (400 MHz, DMSO-d₆, δ ppm): 1.05 (3H, t, J=7.1 Hz), 2.58 (2H, q, J=7.0 Hz), 4.02 (2H, s), 5.16 (2H, s), 6.07 (1H, d, J=2.9 Hz), 6.23 (1H, dd, J=7.8, 2.9 Hz), 7.24 (1H, d, J=8.8 Hz), 7.27 (1H, d, J=8.8 Hz), 7.53 (1H, d, J=8.8 Hz), 7.54 (1H, d, J=8.8 Hz), 7.92 (1H, d, J=8.8 Hz), 7.98 (1H, d, J=7.8 Hz), 8.05 (1H, d, J=9.3 Hz), 8.37 (1H, d, J=8.8 Hz), 8.45 (1H, d, J=9.3 Hz).
ESI-MS Found: m/z 405[M+H]⁺

### Example 97:

### 4-(Benzyloxy)-1-(2-{[(3R)-1-isopropylpyrrolidin-3-yl]oxy}quinolin-6-yl)pyridin-2(1H)-one:

In the same manner as in Production Example 1-4 but using the compound (33 mg) obtained in Production Example 45-1 and 4-benzyloxy-2(1H)-pyridone (34.8 mg), the entitled compound (7.7 mg) was obtained as an orange powder.
¹H-NMR (400 MHz, CDCl₃, δ ppm): 1.18-1.23 (6H, m), 2.05-2.17 (1H, m), 2.41-2.81 (2H, m), 2.83-3.27 (4H, m), 5.03 (2H, s), 5.68 (1H, m), 6.05-6.07 (2H, m), 6.91 (1H, d, J=8.8 Hz), 7.27 (1H, d, J=8.4 Hz), 7.35-7.38 (5H, m), 7.57 (1H, dd, J=8.7, 2.4 Hz), 7.66 (1H, d, J=2.3 Hz), 7.86 (1H, d, J=8.8 Hz), 7.94 (1H, d, J=8.8 Hz).
ESI-MS Found: m/z 456[M+H]⁺

### Example 98:

### 1-[3-(Azetidin-1-ylmethyl)quinolin-7-yl]-4-[(4-fluorobenzyl)oxy]pyridin-2(1H)-one:

P-toluenesulfonic acid monohydrate (20 mg) was added to a methanol (10 mL) solution of the compound (30 mg) obtained in Production Example 47-3, and stirred at 60°C for 2 hours. The solvent was evaporated off from the reaction liquid under reduced pressure, then the residue was diluted with chloroform and washed with aqueous saturated sodium hydrogencarbonate solution. The organic layer was dried with anhydrous sodium sulfate, and the solvent was evaporated off under reduced pressure to obtain an alcohol compound. Next, triethylamine (100 µL) and methanesulfonyl chloride (30 µL) were added to a chloroform solution of the alcohol compound (10 mL), and stirred at 0°C for 3 hours. Water was added to the reaction liquid, and extracted with chloroform. The organic layer was dried with anhydrous sodium sulfate, and the solvent was evaporated off under reduced pressure to obtain a methanesulfonate compound. Next, azetidine hydrochloride (240 mg) and potassium carbonate (360 mg) were added to a THF/methanol mixed solution (4:1, 10 mL) of the methanesulfonate compound, and stirred at 80°C for 20 hours. The reaction liquid was filtered, then the solvent was evaporated off under reduced pressure, and the obtained residue was purified by silica gel column chromatography (chloroform:methanol = 10:1) to obtain the entitled compound (9 mg) as a white solid.
¹H-NMR (400 MHz, CDCl₃, δ ppm): 2.16-2.23 (2H, m), 3.40 (4H, t, J=7.0 Hz), 3.88 (2H, s), 5.03 (2H, s), 6.08 (1H, d, J=2.9 Hz), 6.11 (1H, dd, J=7.8H, 2.9 Hz), 7.11 (2H, t, J=7.8 Hz), 7.36-7.43 (3H, m), 7.65 (1H, dd, J=8.8, 2.0 Hz), 7.89 (1H, d, J=8.8 Hz), 7.99 (1H, d, J=2.0 Hz), 8.15 (1H, s), 8.87 (1H, d, J=2.0 Hz)
ESI-MS Found: m/z 416[M+H]⁺

### Example 99:

### 1-[3-(Azetidin-1-ylmethyl)isoquinolin-7-yl]-4-[(4-fluorobenzyl)oxy]pyridin-2(1H)-one:

In the same manner as in Example 30 but using the compound (34.3 mg) obtained in Production Example 46-4 and azetidine hydrochloride (84.2 mg), the entitled compound (11.0 mg) was obtained. ¹H-nMr (400 MHz, CDCl₃ δ ppm): 2.10-2.17 (2H, m), 3.34 (4H, t, J=7.0 Hz), 3.88 (2H, s), 4.99 (2H, s), 6.05 (1H, d, J=2.6 Hz), 6.07 (1H, dd, J=7.5, 2.6 Hz), 7.07 (2H, m), 7.30 (1H, d,J=7.5 Hz), 7.37 (1H, d, J=8.4 Hz), 7.39 (1H, d,J=8.8 Hz), 7.65 (1H, s), 7.68 (1H, dd, J=8.8, 2.2 Hz), 7.85-7.87 (2H, m), 9.19 (1H, s).
ESI-MS Found: m/z 416[M+H]⁺

### Example 100:

### 1-{3-[(Ethylamino)methyl]isoquinolin-7-yl}-4-[(4-fluorobenzyl)oxy]pyridin-2(1H)-one:

In the same manner as in Example 3 but using the compound (34.3 mg) obtained in Production Example 46-4 and a THF solution (1 mL) of 2 M ethylamine, the entitled compound (13.5 mg) was obtained.
¹H-NMR (400 MHz, CDCl₃, δ ppm): 1.17 (3H, t, J=7.1 Hz), 2.75 (2H, q, J=7.1 Hz), 4.05 (2H, s), 5.00 (2H, s), 6.06 (1H, d, J=2.7 Hz), 6.09 (1H, dd, J=7.6, 2.7 Hz), 7.07 (1H, d, J=8.6 Hz), 7.09 (1H, d, J=8.6 Hz), 7.31 (1H, d, J=7.6 Hz), 7.37 (1H, d, J=8.8 Hz), 7.39 (1H, d, J=8.8 Hz), 7.63 (1H, s), 7.66 (1H, dd, J=8.8, 2.0 Hz), 7.81 (1H, d, J=8.6 Hz), 7.87 (1H, d, J=2.0 Hz), 9.19 (1H, s).
ESI-MS Found: m/z 404[M+H]⁺

### Example 101:

### 4-[(4-Fluorobenzyl)oxy]-1-{3-[(isopropylamino)methyl]isoquinolin-7-yl}pyridin-2(1H)-one:

In the same manner as in Example 3 but using the compound (34.3 mg) obtained in Production Example 46-4 and isopropylamine (1 mL), the entitled compound (12.1 mg) was obtained.
¹H-NMR (400 MHz, CDCl₃, δ ppm): 1.13 (6H, d, J=6.3 Hz), 2.85-2.91 (1H, m), 4.04 (2H, s), 5.00 (2H, s), 6.05 (1H, d, J=2.7 Hz), 6.08 (1H, dd, J=7.5, 2.7 Hz), 7.08 (2H, m), 7.31 (1H, d, J=7.5 Hz), 7.37 (1H, d, J=8.8 Hz), 7.39 (1H, d, J=8.8 Hz), 7.66 (1H, s), 7.68 (1H, dd, J=8.7, 2.1 Hz), 7.84 (1H, d, J=8.8 Hz), 7.88 (1H, d, J=2.1 Hz), 9.20 (1H, s).
ESI-MS Found: m/z 418[M+H]⁺

### INDUSTRIAL APPLICABILITY

The compounds of the invention have an MCH-1R antagonistic effect and are useful, for example, as a preventive, treating or remedial agent for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; and other digestive disorders, respiratory disorders, cancer or pigmentation et al.

## Claims

1. A pyridone derivative of a formula (I) or a pharmaceutically-acceptable salt thereof: 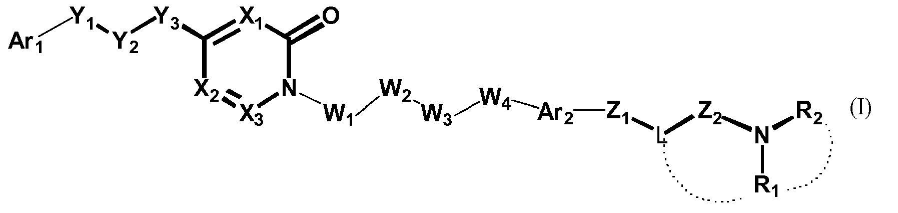 [wherein R₁ and R₂ are the same or different, each representing a hydrogen atom, a lower alkyl group optionally having substituent(s), or a lower cycloalkyl group optionally having substituent(s); or R₁ and R₂, taken together with the nitrogen atom to which they bond, may form an aliphatic nitrogen-containing hetero ring optionally having substituent(s);
X₁, X₂ and X₃ are the same or different, each representing a methine group optionally having substituent(s), or a nitrogen atom; provided that X₁, X₂ and X₃ are not all nitrogen atoms at the same time;
Y₁ represents a single bond, -O-, -NR-, -S-, -SO- or -SO₂-;
Y₂ represents a lower alkylene group optionally having substituent(s), a lower alkenylene group optionally having substituent(s), or a lower cycloalkylene group optionally having substituent(s);
Y₃ represents a single bond, -O-, -NR-, -S-, -SO- or -SO₂-;
R each independently represents a hydrogen atom, or a lower alkyl group optionally having substituent(s);
W₁, W₂, W₃ and W₄ are the same or different, each representing a single bond, a methylene group optionally having substituent(s), or -O-; provided that continuing two or more of W₁, W₂, W₃ and W₄ are not -O- at the same time;
L represents a single bond, a methylene group optionally having substituent(s), or an ethylene group optionally having substituent(s); and L may form, taken together with Z₂, R₁ and the nitrogen atom to which R₁ bonds, an aliphatic nitrogen-containing hetero ring optionally having substituent(s);
Z₁ and Z₂ are the same or different, each representing a single bond, or a C₁-₄ alkylene group optionally having substituent(s), or -O-;
Ar₁ represents an aromatic carbocyclic group optionally having substituent(s), or an aromatic heterocyclic group optionally having substituent(s);
Ar₂ is a divalent group, representing a bicyclic aromatic carbocyclic group optionally having substituent(s), or a bicyclic aromatic heterocyclic group optionally having substituent(s)].

2. The compound or the pharmaceutically-acceptable salt thereof as claimed in claim 1, wherein X₁, X₂ and X₃ are all methine groups optionally having substituent(s).

3. The compound or the pharmaceutically-acceptable salt thereof as claimed in claim 1 or 2, wherein Y₁ is a single bond or -O-.

4. The compound or the pharmaceutically-acceptable salt thereof as claimed in any of claims 1 to 3, wherein Y₂ is a methylene group optionally having substituent(s), an ethylene group optionally having substituent(s), or a vinylene group optionally having substituent(s).

5. The compound or the pharmaceutically-acceptable salt thereof as claimed in any of claims 1 to 4, wherein Y₃ is a single bond or -O-.

6. The compound or the pharmaceutically-acceptable salt thereof as claimed in any of claims 1 to 5, wherein Z₁ is a single bond, a methylene group optionally having substituent(s), or -O-.

7. The compound or the pharmaceutically-acceptable salt thereof as claimed in any of claims 1 to 6, wherein L is a single bond, a methylene group optionally having substituent(s), or an ethylene group optionally having substituent(s).

8. The compound or the pharmaceutically-acceptable salt thereof as claimed in any of claims 1 to 7, wherein Z₂ is a single bond, or a methylene group optionally having substituent(s).

9. The compound or the pharmaceutically-acceptable salt thereof as claimed in any of claims 1 to 6, wherein L, Z₂ and R₁, taken together with the nitrogen atom to which R₁ bonds, form an azetidine ring, a pyrrolidine ring or a piperidine ring.

10. The compound or the pharmaceutically-acceptable salt thereof as claimed in any of claims 1 to 8, wherein R₁ and R₂ are the same or different, and are selected from a group consisting of a hydrogen atom, a methyl group, an ethyl group, an n-propyl group, an isopropyl group, an isobutyl group and a cyclopropyl group.

11. The compound or the pharmaceutically-acceptable salt thereof as claimed in any of claims 1 to 8, wherein R₁ and R₂, taken together with the nitrogen atom to which they bond, form an azetidine ring, a pyrrolidine ring, a morpholine ring, a hexamethyleneimine ring or a piperidine ring.

12. The compound or the pharmaceutically-acceptable salt thereof as claimed in any of claims 1 to 11, wherein Ar₁ is a phenyl group optionally having substituent(s), a naphthyl group optionally having substituent(s), or a pyridinyl group optionally having substituent(s).

13. The compound or the pharmaceutically-acceptable salt thereof as claimed in claim 12, wherein the substituent is selected from a group consisting of a fluorine atom, a chlorine atom, a methyl group, an ethyl group, a methoxy group, a trifluoromethyl group, a difluoromethoxy group and a trifluoromethoxy group.

14. The compound or the pharmaceutically-acceptable salt thereof as claimed in any of claims 1 to 13, wherein the bicyclic aromatic carbon ring or the bicyclic aromatic hetero ring in Ar₂ is naphthalene, quinoline, isoquinoline, quinoxaline, quinazoline, 1,5-naphthyridine, imidazo[1,2-a]pyridine, indazole, benzimidazole, indole or benzofuran.

15. The compound or the pharmaceutically-acceptable salt thereof as claimed in any of claims 1 to 13, wherein Ar₂ is selected from a group of the following formulae:

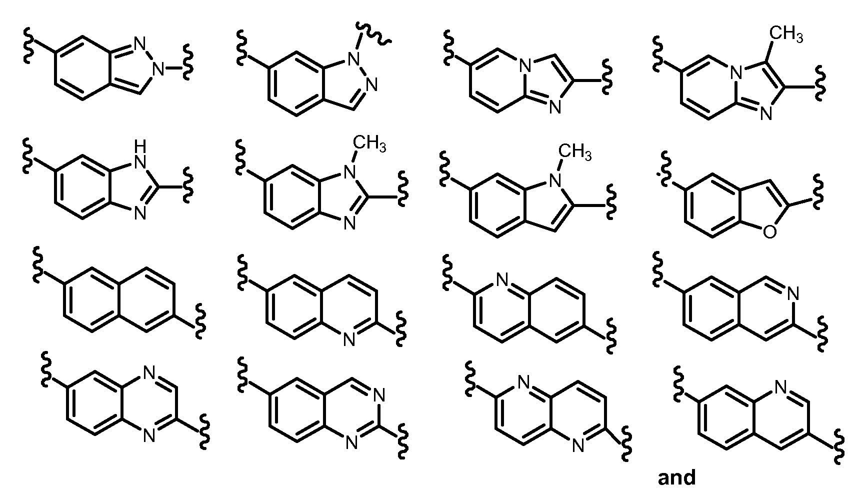

16. The compound or the pharmaceutically-acceptable salt thereof as claimed in any of claims 1 to 15, wherein -W₁-W₂-W₃-W₄- is -O-CH₂-.

17. The compound or the pharmaceutically-acceptable salt thereof as claimed in any of claims 1 to 15, wherein W₁, W₂, W₃ and W₄ are all single bonds.

18. The compound or the pharmaceutically-acceptable salt thereof as claimed in claim 1, wherein the compound of the formula (I) is selected from a group consisting of the following:
1-[6-(azetidin-1-ylmethyl)quinolin-2-yl]-4-(benzyloxy)pyridin-2(1H)-one,
4-(benzyloxy)-1-[6-(2-pyrrolidin-1-ylethoxy)-2-naphthyl]pyridin-2(1H)-one,
1-[3-(azetidin-1-ylmethyl)isoquinolin-7-yl]-4-[(4-fluorobenzyl)oxy]pyridin-2(1H)-one,
4-[(4-chlorobenzyl)oxy]-1-{2-[(methylamino)methyl]quinolin-6-yl}pyridin-2(1H)-one,
1-{2-[(ethylamino)methyl]quinolin-6-yl}-4-[(4-fluorobenzyl)oxy]pyridin-2(1H)-one,
4-[(4-fluorobenzyl)oxy]-1-{2-[(propylamino)methyl]quinolin-6-yl}pyridin-2(1H)-one,
4-[(5-chloropyridin-2-yl)methoxy]-1-{2-[(propylamino)methyl]quinolin-6-yl}pyridin-2(1H)-one,
4-[(4-fluorohenzyl)oxy]-1-{2-[(isopropylamino)methyl]quinolin-6-yl}pyridin-2(1H)-one.
1-[2-(azetidin-1-ylmethyl)quinolin-6-yl]-4-[(5-chloropyridin-2-yl)methoxy]pyridin-2(1H)-one,
4-[(5-chloropyridin-2-yl)methoxy]-1-[2-(pyrrolidin-1-ylmethyl)quinolin-6-yl]pyridin-2(1H)-one,
4-(benzyloxy)-1-{2-[2-(propylamino)ethyl]quinolin-6-yl}pyridin-2(1H)-one,
4-[(5-chloropyridin-2-yl)methoxy]-1-{2-[2-(dimethylamino)ethoxy]quinolin-6-yl}pyridin-2(1H)-one,
4-[(5-chloropyridin-2-yl)methoxy]-1-[2-(2-pyrrolidin-1-ylethoxy)quinolin-6-yl]pyridin-2(1H)-one,
4-[(5-chloropyridin-2-yl)methoxy]-1-[2-(3-pyrrolidin-1-ylpropoxy)quinolin-6-yl]pyridin-2(1H)-one,
4-[(E)-2-(5-chloropyridin-2-yl)vinyl]-1-[2-(2-pyrrolidin-1-ylethoxy)quinoxalin-6-yl]pyridin-2(1H)-one,
4-[(4-chlorobenzyl)oxy]-1-[1-methyl-2-(3-pyrrolidin-1-ylpropyl)-1H-benzimidazol-6-yl]pyridin-2(1H)-one,
4-[(5-chloropyridin-2-yl)methoxy]-1-[1-methyl-2-(pyrrolidin-1-ylmethyl)-1H-indol-6-yl]pyridin-2(1H)-one,
4-[2-(4-fluorophenoxy)ethoxy]-1-{2-[isopropyl(methyl)amino]-1H-benzimidazol-6-yl}pyridin-2(1H)-one,
1-[3-(azetidin-1-ylmethyl)quinolin-7-yl]-4-[(4-fluorobenzyl)oxy]pyridin-2(1H)-one,
4- [(4-fluorobenzyl)oxy]-1-[2-(pyrrolidin-1-ylmethyl)quinazolin-6-yl]pyridin-2(1H)-one,
1-[6-(azetidin-1-ylmethyl)-1,5-naphthyridin-2-yl]-4-[(4-fluorobenzyl)oxy]pyridin-2(1H)-one,
4-(benzyloxy)-1-[2-(2-pyrrolidin-1-ylethyl)-2H-indazol-6-yl]pyridin-2(1H)-one,
4-[(5-chloropyridin-2-yl)methoxy]-1-[3-methyl-2-(3-pyrrolidin-1-ylpropyl)imidazo[1,2-a]pyridin-6-yl]pyridin-2(1H)-one,
1-[2-(azepan-1-ylmethyl)-1-benzofuran-5-yl]-4-[(4-fluorobenzyl)oxy]pyridin-2(1H)-one, and
4-[(4-fluorobenzyl)oxy]-1-{[2-(pyrrolidin-1-ylmethyl)quinolin-6-yl]methoxy}pyridin-2(1H)one.

19. The compound or the pharmaceutically-acceptable salt thereof as claimed in claim 1, wherein the compound of formula (I) is 1-{2-[(ethylamino)methyl]quinolin-6-yl}4-[(4-fluorobenzyl)oxy]pyridin-2(1H)-one.

20. The compound or the pharmaceutically-acceptable salt thereof as claimed in claim 1, wherein the compound of formula (I) is 1-[2-(azetidin-1-ylmethyl)quinolin-6-yl]-4-[(5-chloropyridin-2-yl)methoxy]pyridin-2(1H)-one.

21. The compound or the pharmaceutically-acceptable salt thereof as claimed in claim 1, wherein the compound of formula (I) is 4-(benzyloxy)-1-{2-[2-(propylamino)ethyl]quinolin-6-yl}pyridin-2(1H)-one.

22. The compound or the pharmaceutically-acceptable salt thereof as claimed in claim 1, wherein the compound of formula (I) is 4-[(5-chloropyridin-2-yl)methoxy]-1-[2-(2-pyrrolidin-1-ylethoxy)quinolin-6-yl]pyridin-2(1H)-one.

23. The compound or the pharmaceutically-acceptable salt thereof as claimed in claim 1, wherein the compound of formula (I) is 4-[(4-fluorobenzyl)oxy]-1-{2-[(propylamino)methyl]quinolin-6-yl}pyridin-2(1H)-one.

24. The compound or the pharmaceutically-acceptable salt thereof as claimed in claim 1, wherein the compound of formula (I) is 4-[(5-chloropyridin-2-yl)methoxy]-l-{2-[(propylamino)methyl]quinolin-6-yl}pyridin-2(1H)-one.

25. The compound or the pharmaceutically-acceptable salt thereof as claimed in claim 1, wherein the compound of formula (I) is 4-[(4-fluorobenzyl)oxy]-1-{2-[(isopropylamino)methyl]quinolin-6-yl}pyridin-2(1H)-one.

26. A melanin concentrating hormone receptor antagonist comprising a compound or a pharmaceutically-acceptable salt thereof of claims 1 to 25 as the active ingredient.

27. A pharmaceutical composition comprising a pharmaceutically-acceptable additive and an effective amount of a compound or a pharmaceutically-acceptable salt thereof of claims 1 to 25.

28. A preventive, treating or remedial agent comprising a compound or a pharmaceutically-acceptable salt thereof of claims 1 to 25 as the active ingredient, for metabolic disorders such as obesity, diabetes, hormone disorder, hyperlipidemia, gout, fatty liver, hepatitis, cirrhosis; cardiovascular disorders such as stenocardia, acute or congestive heart failure, myocardial infarction, coronary atherosclerosis, hypertension, renal diseases, electrolyte abnormality; central and peripheral nervous system disorders such as bulimia, emotional disturbance, depression, anxiety, epilepsy, delirium, dementia, schizophrenia, attention-deficit hyperactivity disorder, memory impairment, sleep disorders, cognitive failure, dyskinesia, paresthesias, smell disorders, morphine tolerance, drug dependence, alcoholism; reproductive disorders such as infertility, preterm labor and sexual dysfunction; digestive disorders; respiratory disorders; cancer or pigmentation.
